# EUROPÄISCHE PATENTSCHRIFT

(11) **EP 1 483 576 B1**
(45) Veröffentlichungstag und Bekanntmachung des Hinweises auf die Patenterteilung: **06.10.2010**
(21) Anmeldenummer: 03743880.1
(22) Anmeldetag: 13.03.2003
(51) Int. Cl.: G01N 33/50, G01N 33/68, C12Q 1/37, C12N 15/00, C12N 15/62

(54) **NEUE VERFAHREN ZUR DETEKTION UND ANALYSE VON PROTEIN-INTERAKTIONEN IN VIVO**
NOVEL METHOD FOR DETECTING AND ANALYZING PROTEIN INTERACTIONS IN VIVO
PROCEDE DE DETECTION ET D'ANALYSE D'INTERACTIONS PROTEIQUES IN VIVO

(30) Priorität: 13.03.2002 DE 10211063
(43) Veröffentlichungstag der Anmeldung: 08.12.2004
(60) Teilanmeldung: 09165808.8
(73) Patentinhaber: Sygnis Bioscience GmbH & Co. KG, 69120 Heidelberg (DE)
(72) Erfinder: ROSSNER, Moritz, 37077 Göttingen (DE); LAAGE, Rico, 69198 Schriesheim (DE); NAVE, Klaus-Armin, 37075 Göttingen (DE); GRÜNEWALD, Sylvia, 78462 Konstanz (DE)
(74) Vertreter: Isenbruck, Günter
(86) Internationale Anmeldenummer: PCT/EP2003/002611
(87) Internationale Veröffentlichungsnummer: WO 2003/076932

(56) Entgegenhaltungen:
- MATTHEAKIS LARRY C ET AL: "Expression of Cre recombinase as a reporter of signal transduction in mammalian cells" CHEMISTRY AND BIOLOGY (LONDON), Bd. 6, Nr. 11, November 1999 (1999-11), Seiten 835-844, XP002265769 ISSN: 1074-5521
- DUNNWALD M ET AL: "DETECTION OF TRANSIENT IN VIVO INTERACTIONS BETWEEN SUBSTRATE AND TRANSPORTER DURING PROTEIN TRANSLOCATION INTO THE ENDOPLASMIC RETICULUM" MOLECULAR BIOLOGY OF THE CELL, BETHESDA, MD, US, Bd. 10, Nr. 2, Februar 1999 (1999-02), Seiten 329-344, XP008006622 ISSN: 1059-1524
- VIDAL M ET AL: "REVERSE TWO-HYBRID AND ONE-HYBRID SYSTEMS TO DETECT DISSOCIATION OF PROTEIN-PROTEIN AND DNA-PROTEIN INTERACTIONS" PROCEEDINGS OF THE NATIONAL ACADEMY OF SCIENCES OF USA, NATIONAL ACADEMY OF SCIENCE. WASHINGTON, US, Bd. 93, 1. September 1996 (1996-09-01), Seiten 10315-10320, XP000749719 ISSN: 0027-8424

## Beschreibung

Die Erfindung betrifft diverse Verfahren zur Detektion und zur Analyse von Protein-Interaktionen in einer Zelle, bei denen das Auftreten einer spezifischen Protein-Interaktion in ein permanentes Detektionssignal mittels der Protein-Interaktions-abhängigen Bereitstellung einer TEV-Protease-Aktivität, umgesetzt wird.

Ein weiterer Aspekt der Erfindung betrifft einen Kit zur Durchführung der oben genannten Verfahren sowie ein Screening Verfahren zur Identifizierung eines spezifischen Interaktionspartners eines definierten Proteins.

Das erfindungsgemäße Verfahren ermöglicht insbesondere den Nachweis der Dynamik von spezifischen Protein-Interaktionen. Sowohl das Zustandekommen wie auch die induzierte Dissoziation von Interaktionspartnern sind nachweisbar. Die Interaktionspartner können in direktem Kontakt stehen oder Teil eines Proteinkomplexes sein. Das Verfahren ist besonders geeignet für den Nachweis von transienten Interaktionen, sehr schwachen Interaktionen oder solchen, die durch einen zellulären Stimulus oder eine Substanz induziert werden.

Nahezu alle biologischen Prozesse in lebenden Organismen werden durch die Funktion von spezifisch interagierenden Proteinen gesteuert. Die gezielte Analyse von Protein-Interaktionen ermöglicht die Isolierung und Zuordnung unbekannter Proteine zu Funktionsgruppen und die Aufklärung des molekularen Wirkmechanismus bekannter Proteine. Die zelluläre Signaltransduktion, welche die Übertragung von extrazellulären Signalen in spezifische intrazelluläre Veränderungen beinhaltet, ist der Schlüsselmechanismus zur Steuerung einer Zelle während der Entwicklung und bei der Reaktion auf Umweltveränderungen. Die Übertragung dieser Signale wird durch streng regulierte Kaskaden von spezifisch interagierenden Proteinen gesteuert. Darüberhinaus werden nahezu alle wichtigen zellulären Funktionen, die meist an die Signaltransduktion gekoppelt sind, durch kontrollierte Protein-Interaktionen ausgeführt (Pawson and Scott 1997). Diese umfassen u.a. die Steuerung des Zell-Zyklus, Proteinsynthese und - degradation, Verhinderung bzw. Einleitung von Apoptose, Transportvorgänge, Reizerkennung und Weiterleitung, Gen-Expression, mRNA-Prozessierung, DNA-Synthese und -Reparatur sowie den gesamten Energiemetabolismus. All diese Prozesse sind sehr dynamisch, d.h. sie unterliegen Veränderungen, die eingebettet sind in den Gesamtzustand der Zelle. Auf Proteinebene wird dies wiedergespiegelt durch die regulierte Veränderung der Zusammensetzung der funktionsausführenden Komplexe (Ashman, Moran et al. 2001).

Proteine und deren Interaktionen unterliegen starken und dynamischen Veränderungen in der Zelle, die häufig durch Signaltransduktionskaskaden verursacht werden. Durch allosterische Effekte oder Änderung der intrazellulären Lokalisation kann sich auch ihre Funktion bzw. die Zusammensetzung von Proteinkomplexen wandeln. Die Regulation der Funktion von Proteinen ist in besonderem Maße abhängig von der Aktivierung durch Enzyme der Signaltransduktionskaskaden, die Protein-Modifikationen an spezifischen Zielproteinen katalysieren. Post-translationale Modifikationen haben oft dramatischen Einfluß auf die Aktivität einer Vielzahl von Proteinen. Regulierte Protein-Phosphorylierung, -Acetylierung, -Methylierung, -Sulfatidierung, -Acylierung - Prenylierung, -Ribosylierung, -Glykosylierung, -Ubiquitinierung oder proteolytische Aktivierung und Inaktivierung sind bekannte Modifkationen, deren Wirkungen und Regulationsmechanismen teilweise nur wenig verstanden sind. Modifikationsabhängige Proteininteraktionen sind für eine Vielzahl von Transduktionswegen bei verschiedenen zellulären Anpassungsereignissen beschrieben (Hunter 2000).

Die Aktivierung bzw. Deaktivierung bestimmter Signaltransduktionskaskaden muß in der Zelle sowohl zeitlich als auch in ihrer Ausprägung genau kontrolliert werden. Eine Vielzahl pathologischer Abläufe in Zellen wird durch eine Störung in der Kontrolle der Signaltransduktion verursacht und kann zu Stoffwechselerkrankungen, Kanzerogenese, immunologischen Erkrankungen oder zu neurologischen Defiziten fuhren. Solche pathologischen Abläufe können insbesondere durch spezifische Mutationen in den Genen kodierend für Proteine mit wichtiger Funktion in der neuronalen Signaltransduktion verursacht werden. So verändern beispielsweise bestimmte Mutationen in den Genen der NMDA-Rezeptor-Untereinheiten die Zusammensetzung und die Signaleigenschaften des NMDA-Rezeptor-Proteinkomplexes (Migaud, Charlesworth et al. 1998). Molekulare Mechanismen, die für die genaue zeitliche Kontrolle von zellulären Ereignissen verantwortlich sind und oftmals durch Rückkopplungsmechanismen gesteuert werden, sind erst in Anfängen verstanden (Marshall 1995). Zeitlich begrenzte, z.B. durch reversible post-translationale Modifikationen kontrollierte Protein-Protein Interaktionen üben hierbei eine Schlüsselfunktion aus (Yasukawa, Sasaki et al. 2000)(Hazzalin and Mahadevan 2002).

Zur Identifizierung und Analyse regulierter, zeitlich begrenzter oder modifikationsabhängiger Protein Interaktionen fehlen derzeit geeignete Methoden.

Zur Charakterisierung von Protein-Protein Interaktionen sind eine Vielzahl von Methoden beschrieben.

Biochemische Methoden der Proteinaufreinigung gekoppelt an massenspektrometrische Analyseverfahren ermöglichen die Charakterisierung von Proteinkomplexen (Ashman, Moran et al. 2001). So konnte z.B. mit Hilfe einer Tandem-Affinitäts Aufreinigung (TAP) (Rigaut, Shevchenko et al. 1999), denaturierender eindimensionaler Gelelektrophorese und tryptischem Verdau von Einzelbanden in Kombination mit massenspektrometrischer Analyse eine Vielzahl von Proteinkomplexen aus Hefe isoliert und deren Komponenten bestimmt werden (Gavin, Bosche et al. 2002). Unter Verwendung eines optimierten Immunpräzipitationsprotokolls, Massenspektrometrie und der "Western Blot"-Technik konnte eine Vielzahl der Komponenten des neuronalen NMDA Rezeptor Signalverarbeitungs-Komplexes charakterisiert werden (Husi, Ward et al. 2000). Für die Analyse von stabilen und statischen Proteinkomplexen scheinen diese biochemischbiophysikalischen Verfahren besonders gut geeignet zu sein; weisen allerdings einige experimentelle und prinzipielle Nachteile auf. So ist für alle biochemischen Verfahren der experimentelle Aufwand und die Menge an benötigtem biologischen Ausgangsmaterial sehr hoch. Zudem ist es für bereits optimierte Aufreinigungsverfahren (z.b. die TAP-Methode) notwendig, die entsprechenden Fusionsproteine transgen in den Organismen der Wahl stabil zu exprimieren. Bei der Analyse komplexer Gewebe können schwach exprimierte oder zelltyp-spezifisch exprimierte Proteine leicht unter die Detektionsgrenze fallen. Ein grundsätzlicher Nachteil aller biochemischer Methoden ergibt sich aus der Notwendigkeit des Zellaufschlusses bzw. der Solubilisierung großer Membrankomplexe. Schwache oder transiente Interaktionen können im Zuge der meist mehrere Schritte umfassenden biochemischen Aufarbeitung leicht verloren gehen (Ashman, Moran et al. 2001). Analysen der Interaktionen von Proteinen mit extremen physiko-chemischen Eigenschaften, wie z.B. von Membranproteinen oder von Proteinen mit hoher Gesamtladung, bedürfen jeweils eines optimierten Protokolls oder sind in Einzelfällen nicht möglich.

Bisherige massenspektrometrische Methoden sind zur Charakterisierung von posttranslational modifizierten Proteinen nur bedingt geeignet, da Modifikationen wie spezifische Phosphatreste während der Fragmentation leicht verloren gehen können (Ashman, Moran et al. 2001). Zusammensetzung und funktionelle Aktivität von Proteinkomplexen unterliegen einer steten Dynamik und werden durch eine Vielfalt von Regulationsmechanismen kontrolliert. Zudem wurde in jüngster Zeit deutlich, dass spezifische physiko-chemische Eigenschaften oder spezifische Umgebungen, wie z.B spezifische Membrantopologien und -zusammensetzungen Protein-Protein Interaktionen stark beeinflussen. So spielt z.B. die Lipidzusammensetzung der unterschiedlichen intrazellulären Membransysteme eine große Rolle bei der Zusammensetzung von Proteinkomplexen (Huttner and Schmidt 2000). Sogenannte "Lipid-rafts", Subdomänen in der Zellmembran, erlauben aufgrund ihrer besonderen molekularen Zusammensetzung gänzlich andere Interaktionen als benachbarte Bereiche in der Lipid-Doppelschicht (Simons and Ikonen 1997). Zur Detail-Analyse der spezifischen Interaktionen oder Interaktionsdomänen von Proteinen innerhalb eines Komplexes, oder zur Analyse möglicher transienter Interaktionen assoziierter Proteine sind biochemische Verfahren wegen des relativ hohen experimentellen Aufwands nur bedingt geeignet.

Mikroarrays sind ein weiteres Werkzeug zur systematischen Analyse von Protein-Protein-Interaktionen, Protein-Peptid-Interaktionen oder der Interaktionen von Proteinen mit niedermolekularen Substanzen. Bei dieser Methode werden in vitro translatierte oder rekombinant produzierte Proteine bzw. Peptide, Antikörper, spezifische Liganden oder niedermolekulare Substanzen in Analogie zu DNA-Mikroarrays auf ein Trägermaterial aufgebracht. Komplexe Proteinmischungen oder Substanzbibliotheken können somit simultan u.a. nach spezifischen Interaktionen durchmustert werden (Ashman, Moran et al. 2001)(Xu, Piston et al. 1999). Die vollständig in vitro durchgeführten Analysen mit Protein- oder Substanzarrays haben jedoch ebenfalls große Nachteile. So werden Proteine bei dieser Methode unter vollkommen artifiziellen Bedingungen hergestellt oder analysiert. Weiterhin werden die Anwendungsmöglichkeiten dieser Array-basierten in vitro Analysemethoden zur Detektion und zur Analyse von Protein-Interaktionen durch das Auftreten von hohen unspezifischen Hintergrundsignalen, durch die begrenzte Sensitivität und durch Schwierigkeiten bei der Detektion von Proteinen mit bestimmten physiko-chemischen Eigenschaften stark eingeschränkt (Ashman, Moran et al. 2001).

Es sind weiterhin diverse Verfahren bekannt, bei denen Protein-Interaktionen in der Zelle - und damit in vivo - durch die indirekte Aktivierung genetischer Reporter detektiert werden. Die meisten der gängigen Verfahren sind auf den Nachweis binärer Proteininteraktionen im Karyoplasma beschränkt und beruhen auf der funktionalen Modularität von Transkriptionsfaktoren, wie beispielsweise das 2-Hybrid-System in Hefen (Fields and Song 1989). Beim 2-Hybrid-System werden in Hefezellen ein oder mehrere Proteine oder Proteinabschnitte als Fusionsproteine mit einem DNA-Bindungsprotein ohne Transaktivierungsfähigkeit exprimiert und als sogenannter Köder (Bait) zur Detektion interagierender Komponenten eingesetzt. Ein zweites Protein oder Proteinfragment wird als Fusionsprotein mit einer transkriptionellen Transaktivierungsdomäne exprimiert und stellt die sogenannte Beute-Komponente (Prey) dar. Die sogenannte Beute- bzw. Prey-Komponente ist häufig ein Fusionsprotein, das neben der transkriptionellen Transaktivierungsdomäne ein Genprodukt einer komplexen cDNA-Bibliothek umfaßt. Bei Interaktion des bait- und prey-Fusionsproteins kommt es zu einer funktionalen Rekonstituierung des aktivierenden Transkriptionsfaktors. Als Reporter-Gene werden beim 2-Hybrid-System Enzyme verwendet, mit denen die Protein-Interaktion entweder durch eine Wachstumsselektion oder durch einen einfachen kolorimetrischen Test nachgewiesen werden können und die sehr sensitiv sind. Ein beim 2-Hybrid-System häufig verwendetes Reporter-Gen, das eine positive Wachstumsselektion von Zellen mit spezifschen Protein-Protein-Interaktionen ermöglicht, ist das Histidin 3-Gen, welches ein essentielles Enzym zur biosynthetischen Herstellung von Histidin darstellt und dessen Protein-Protein-Interaktions-abhängige Expression das Wachstum der Zellen auf Histidin-defizientem Medium ermöglicht. Das häufigst verwendete Reporter-Gen, dessen Protein-Protein-Interaktions-abhängige Expression durch einen einfachen kolorimetrischen Test nachgewiesen werden kann, ist das beta-Galaktosidase-Gen.

Ursprünglich wurde das 2-Hybrid System in Hefe entwickelt, in der Folge wurden jedoch auch Varianten des 2-Hybrid Systems für die Anwendung in E. coli und in höheren eukaryotischen Zellen beschrieben (Luban and Goff 1995) (Karimova, Pidoux et al. 1998) (Fearon, Finkel et al. 1992) (Shioda, Andriole et al. 2000).

Ein wesentlicher Nachteil der klassischen 2-Hybrid-basierten Systeme liegt u.a. in der relativ hohen Rate an sowohl falsch-negativ wie auch falsch-positiv detektierten Interaktionspartnern. Dies liegt zum einem an der hohen Sensitivität der Reporter, aber auch an der räumlichen Kopplung der Interaktion und der basalen Transkriptionsmaschinerie. Kürzlich wurden Interaktionssysteme für Hefezellen beschrieben, die den Ort der Interaktion räumlich von der Aktivierung der verwendeten Reportergene oder der zur Detektion verwendeten Selektionsmechanismen entkoppeln (Maroun and Aronheim 1999). Verwandte Systeme in Hefe erlauben auch die Analyse zumindest einen Interaktionspartners, der ein integrales oder membran-assoziiertes Protein sein kann (Hubsman, Yudkovsky et al. 2001) (Ehrhard, Jacoby et al. 2000).

Die funktionelle Komplementation von Proteinen oder Enzymen, die auch die Basis der klassischen 2-Hybrid-basierten Systeme darstellt, ist eine seit längerer Zeit bekannte und angewandte Methode zur Analyse von Interaktionen in lebenden Zellen und Bakterien (Ullmann, Perrin et al. 1965) (Fields and Song 1989) (Mohler and Blau 1996) (Rossi, Charlton et al. 1997) (Pelletier, Campbell-Valois et al. 1998). Unter Transkomplementation versteht man die Trennung eines intakten und funktionalen Proteins oder Proteinkomplexes in zwei künstliche Untereinheiten auf Gen-Ebene. Die beiden Untereinheiten sind hierbei für sich gesehen inaktiv bezüglich der Funktion des Gesamtproteins, jedoch aktiv bezüglich ihrer entsprechenden Untereinheiten-Funktion und sie besitzen keine Fähigkeit zur Selbstrekonstitution. Die Fusion solcher Untereinheiten an Proteine oder Proteindomänen, die miteinander interagieren, führt durch die Protein-Interaktions-abhängige Herstellung großer räumlicher Nähe zwischen den beiden getrennten Untereinheiten zur Komplementation des geteilten Proteins, wobei es wieder funktionell wird. Die Wiedererlangung der Funktion des Proteins (z.B. eines Enzyms) durch eine Protein-Interaktion wird dabei direkt oder indirekt als Nachweis für die Wechselwirkung benutzt (Mohler and Blau 1996) (Rossi, Charlton et al. 1997). Das bekannteste Beispiel ist die Transkomplementation des Transkriptionsfaktors Ga14, welche die Grundlage des klassischen Hefe-2-Hybrid Systems darstellt (Fields and Song 1989).

Darüberhinaus wurden Transkomplementationen und daran gekoppelte Nachweismethoden für Protein-Interaktionen für unterschiedliche Proteine mit enzymatischer Aktivität beschrieben, u.a. beta-Galaktosidase (bGal), Dihydrofolat-Reduktase (DHFR) und beta-Lactamase (bLac) beschrieben (Michnick and Remy 2001) (Rossi, Charlton et al. 1997) (Michnick and Galarneau 2001). Der indirekte Nachweis von Protein-Interaktionen kann bei diesen Systemen nach Transkomplementation der oben genannten Enzyme durch Wachstumsselektion oder durch fluoreszenz- oder kolorimetrische Enzym-Nachweise erfolgen. Je nach verwendetem Substrat kann der Nachweis meist erst nach Aufschluß der Zellen und Zugabe des Substrates in vitro durchgeführt werden.

Im Gegensatz dazu kann beim DHFR-basierten System die Protein-Interaktion bzw. die Transkomplementation auch in vivo nachgewiesen werden. Dazu wird ein zellpermeabler fluoreszenzmarkierter Antagonist (Methotrexat) zugegeben, der nur an das intakte Protein bindet. Der Nachteil hierbei ist allerdings, daß der Antagonist kein Substrat des Enzyms ist, sondern als kompetetiver Inhibitor das Enzym bindet. Es kommt daher zu keinerlei enzymatischen Verstärkung des Detektionssignals. Dies führt dazu, daß die Sensitivität dieser Detektionsmethode gegenüber der Detektion positiver Klone durch positive Wachstumsselektion unter den entsprechenden Kulturbedingungen stark herabgesetzt ist. Zudem ist das Detektionssignal beim DHFR-basierten System zur Analyse von Protein-Interaktionen nur direkt nach der Zugabe des fluoreszenz-markierten Inhibitors sichtbar, d.h. dynamische Abläufe in Zellen, die häufig mit dynamischen bzw. transienten Protein-Interaktionen einhergehen, können mit diesen nicht permanenten, nur kurzzeitig detektierbaren Signalen nicht nachgewiesen werden. Darüberhinaus ist der beschriebene Inhibitor (Methotrexat) eine stark zytotoxische Substanz, die nach Applikation Zellwachstum, Metabolismus und andere intrazelluläre Prozesse stark beeinträchtigt und verändert und damit die normalen in vivo-Bedingungen verfälscht.

Eine DHFR-basierte Methode zur Analyse von Protein-Interaktionen, die auf der positiven Wachstumsselektion von Zellen mit transkomplementiertem DHFR - und nicht auf der Applikation von Methotrexat - basiert, bietet zwar eine entsprechend höhere Sensitivität, beansprucht jedoch Zeiträume von mehreren Tagen und Wochen, ein Umstand der diese Methode zur Anwendung in Hochdurchsatzverfahren, so z.B. im high-throughput-Screening, wenig geeignet erscheinen läßt. Aufgrund dieser Nachteile eignen sich diese Systeme auch nur sehr begrenzt zur Analyse transienter oder Stimulus-induzierter Interaktionen, da eine kurzzeitige Transkomplementation unzureichend ist, um eine Wachstumsselektion positiver Zellen über einen längeren Zeitraum zu ermöglichen. Andererseits kann die Bindung fluoreszierender Antagonisten, wie Methotrexat, durch das transkomplementierte DHFR nur dann nachgewiesen werden, wenn man den exakten Zeitpunkt der transienten oder der Stimulus-induzierten Interaktion trifft.

Diese Probleme bei der Detektion transienter, d.h. zeitlich begrenzter Protein-Interaktionen betreffen auch das klassische 2-Hybrid-System und seine nach dem Stand der Technik bekannten Varianten, da auch hier Reporter-Systeme eingesetzt werden, die zu keinem permanenten Detektionssignal führen.

Transiente Protein-Interaktionen führen lediglich zum Zeitpunkt ihres Auftretens zur kurzzeitigen Expression der verwendeten Reporter-Gene, wobei die Genprodukte der bisher verwendeten Reporter-Gene lediglich im Zeitraum ihrer Lebensdauer ein Detektionssignal erzeugen können. Dies bedeutet, daß beim 2-Hybrid-System und seinen Varianten - und insbesondere bei der Analyse schwacher und transienter Protein-Interaktionen - lediglich ein relativ kurzer Zeitraum zur Detektion der Protein-Interaktions-abhängigen Signale zur Verfügung steht. Dies stellt insbesondere dann ein großes Problem dar, wenn eine große Anzahl unterschiedlicher potentieller Interaktionspartner eines Bait-Proteins simultan auf Interaktion getestet werden müssen, wie beispielsweise in Screening-Verfahren, insbesondere in high-throughput-Screening-Verfahren. In Screening-Verfahren muß eine Vielzahl von unterschiedlichen potentiellen Interaktionen, die eventuell auch zeitlich versetzt stattfinden können und eventuell unterschiedliche Interaktionsstärken und Lebensdauer besitzen, zu einem definierten Zeitpunkt simultan analysiert werden. Wenn bestimmte Detektionssignale jedoch lediglich in einem engen "Zeitfenster" detektierbar sind, die eventuell für verschiedene zu detektierende Interaktionen nicht einmal überlappen, so können gewisse Interaktionen, insbesondere schwache und transiente Protein-Interaktionen, vom 2-Hybrid-System, seinen Varianten und von den bekannten Transkomplementations-basierten Detektionssystemen zur Analyse von Protein-Interaktionen nicht erfaßt werden.

Ein weiteres Selektionssystem, das auf einer Protein-Transkomplementation besonderer Art beruht, ist das ursprünglich für Studien in Hefe entwickelte und kürzlich auch in Säugerzellen angewandte "Split Ubiquitin System". Dieses benutzt die Trennung von Ubiquitin, in zwei nicht-funktionelle Anteile, ein N- und ein C-terminales Fragment (NUb und Cub) (Johnsson and Varshavsky 1994) (Rojo-Niersbach, Morley et al. 2000). Ubiquitin ist ein kleines Protein, welches normalerweise an seinen C-Terminus fusionierte Proteine für den zellulären Abbau markiert. Dieser biologische Mechanismus wird im "Split Ubiquitin System" zur Detektion von Protein-Interaktionen eingesetzt. In einer Ausführungsform des "Split Ubiquitin Systems" wird in der Zelle ein erstes Fusionsprotein umfassend das C-terminale Fragment Cub, ein daran gekoppeltes Selektionsmarker-Protein oder fluoreszierendes Protein und einen ersten Interaktionspartner und ein zweites Fusionsprotein umfassend das N-terminale Fragment Nub und den zweiten Interaktionspartner heterolog exprimiert. Bei einer spezifischen Interaktion der entsprechenden Fusionsproteine wird ein korrekt gefaltetes Ubiquitin wiederhergestellt, welches vom Proteasom erkannt und prozessiert werden kann und wobei der gekoppelte zunächst aktive Reporter anschließend degradiert wird. Das System erlaubt demnach eine negative, auf Abwesenheit des Selektionsmarkers gerichtete Wachstumsselektion bzw. die Beobachtung des Verschwindens eines fluoreszierenden Reporters. Diese in wissenschaftlichen Publikationen dargestellten Ausführungen des "Split Ubiquitin Systems" offenbaren somit zwei große Schwachpunkte, erstens erschwert die negative Selektion die schnelle und eindeutige Detektion relevanter Interaktionen und zweitens findet nach erfolgter Interaktion keine Signalverstärkung in der Zelle statt. Letzteres macht es nahezu unmöglich, schwache bzw. transiente Interaktionen zu detektieren.

In einer im Patent WO 95/29195 offenbarten Ausführungsform des "Split Ubiquitin Systems" werden zwei unterschiedliche Fusionsproteine in einer Zelle exprimiert, die jeweils einen Interaktionspartner und einen Teil des Ubiquitins umfassen. Eines der beiden Fusionsproteine umfaßt hierbei weiterhin ein Reporter-Protein, welches durch eine Ubiquitin-spezifische Protease proteolytisch abspaltbar ist. Dieses Reporter-Protein wird hierbei erst nach dem Auftreten einer spezifischen Protein-Protein-Interaktion durch eine Ubiquitin-spezifische Protease abgespalten und danach erst aktiviert. Diese Ausführungsform des "Split Ubiquitin Systems" überkommt jedoch nicht die Tatsache, daß pro stattgefundener Interaktion nur einmalig ein Reportermolekül freigesetzt bzw. aktiviert werden kann. Letzteres macht es nahezu unmöglich, schwache bzw. transiente Interaktionen zu detektieren. Darüberhinaus ist der Reporter direkt an einen der Interaktionspartner gekoppelt, was zum einen bedeutet, daß die Reportermenge stark abhängig ist vom Expressionsniveau und der Stabilität des Interaktionspartners, an den er fusioniert wurde. Dies führt dazu, daß ein instabiles oder leicht degradierbares Protein bei der Analyse ein falsch positives Signal liefern kann.

Ein weiteres System, für Säugerzellen beschrieben, beruht auf der Aktivierung und Dimerisierung von modifizierten Typ I Cytokine-Rezeptoren (Eyckerman, Verhee et al. 2001). Die Aktivierung des STAT3-Signalweges kann in diesem System nur erfolgen, wenn eine Interaktion zwischen dem Bait-Rezeptorfusionsprotein und dem Prey-Fusionsprotein stattfindet. Das Prey-Protein ist an gp130 fusioniert, welches STAT-Bindungsstellen trägt. Die Rezeptor assoziierten Janus-Kinasen phosphorylieren gp130 nur nach Bait-Prey Interaktion, dies führt zu einer Bindung, Phosphorylierung und nachgeschalteter Kerntranslokation von STAT3-Transkriptionsfaktoren. STAT regulierte Reportergene werden abhängig von der Bait-Prey Interaktion exprimiert. Zur Identifizierung neuer intrazellulärer Interaktionspartner wurde eine Selektionsstrategie etabliert, die Puromycin-Resistenz vermittelt (Eyckerman, Verhee et al. 2001). Die Methode erlaubt zwar die Detektion einer Protein-Protein Interaktion an der Membran durch die Expression eines Reportergens, sie verlangt allerdings die Kopplung mindestens eines Interaktionspartneres an besagten Membranrezeptor. Zudem ist die komplexe Quartärstruktur des Rezeptor-Kinase-GP130 Multimers nicht dazu geeignet, schwierige Proteinklassen wie Membranproteine zu analysieren. Das System ist aufgrund mangelnder Amplifikation und Beständigkeit des Signals nicht in der Lage, transiente oder schwache Interaktionen zu analysieren.

Verfahren, die auf der Übertragung von Energiequanten eines Donor-Moleküls auf ein Akzeptor-Molekül beruhen, sofern diese in unmittelbare Nähe gebracht werden, sind theoretisch wenig eingeschränkt. Diese Verfahren können verschiedene Varianten des Green Fluorescent Protein (GFP) von Aequorea victoria verwenden, die aufgrund ihrer spezifischen Spektraleigenschaften zu Fluoreszenz-Resonanz-Energie-Transfer (FRET) fähig sind (Siegel, Chan et al. 2000). Ein ähnliches Verfahren beruht auf einer Energie-Kopplung der Bioluminiszenz der Luziferase-Luziferin Reaktion als Energie-Donor und GFP als Energie-Akzeptor. Die Energieübertragung wird als Bioluminizenz-Resonanz-Energie-Transfer (BRET) Effekt bezeichnet (Xu, Piston et al. 1999). Hierbei ist allerdings die Zugabe von entsprechenden Luziferase-Substraten notwendig. Die wesentlichen Nachteile dieser Methoden liegen in der Sensitivität und der Schwierigkeit des Nachweises begründet. Zur Detektion von FRET-Effekten in vivo ist sowohl eine sehr starke Expression als auch eine aufwendige Analyse notwendig. Starke Überexpression von Proteinen in heterologen Zellen führen oft zur Aggregatbildung, falscher Faltung oder fehlgeleiteter subzellulärer Lokalisation. Die Methode bietet keine Möglichkeit einer Signalverstärkung bzw. -amplifikation, ein entscheidender Nachteil, der die Detektion schwacher Interaktionen oder Interaktionen schwach exprimierter Proteine ausschließt. Um eine Proteininteraktion von spektral kompatiblen GFP-Fusionsproteinen durch FRET-Effekte in der Zelle nachweisen zu können, müssen Hintergrundsubtraktionen und Ausbleichungsanalysen durchgeführt werden (Haj, Verveer et al. 2002). Die Methode eignet sich aufgrund des hohen technischen Aufwandes nicht für Hochdurchsatzverfahren zur Analyse von Protein-Interaktionen, darüberhinaus verlangt sie aufwendige Analysen und große Erfahrung, was einer breiten Anwendung entgegen steht.

Alle bisher beschriebenen indirekten Methoden haben den prinzipiellen Nachteil, die Umsetzung der Proteininteraktion an ein Nachweisverfahren zu koppeln, das eine andauernde Aktivierung benötigt oder die Analyse insbesondere transienter Interaktionen nur in einem sehr engem Zeitfenster zuläßt. Die Analyse von Protein-Interaktionen in postmitotischen Zellen oder die Identifizierung transienter Interaktionen sind daher nicht oder nur sehr eingeschränkt möglich. Ein automatisierbarer Nachweis von Interaktionen die einer unbekannten Kinetik unterliegen ist somit nicht möglich.

Die bisher beschriebenen Methoden zur Analyse oder Detektion von Protein-Protein Interaktionen weisen somit mindestens einen oder mehrere der folgenden Nachteile auf:
- Die Interaktionen finden nicht in vivo, oder zumindest nicht in Säugetier-Zellen, statt.
- Es werden große Mengen biologischen Materials benötigt.
- Die Interaktionen müssen dauerhaft sein bzw. die Analyse muß genau zum richtigen Zeitpunkt erfolgen.
- Die Messung der Interaktion erfordert aufwendige Messungen bzw. Gerätschaften.
- Die Sensitivität des Nachweises ist sehr beschränkt.
- Die Analyse endogen sehr schwach exprimierter Gene ist eingeschränkt.
- Es werden nur binäre Interaktionen detektiert.
- Die Rate falsch-positiver oder falsch-negativer Interaktionen ist hoch.
- Die Analyse zelltyp-spezifisch exprimierter Gene ist im komplexen Gewebeverband oder in Zelllinien bei biochemischen Methoden nahezu unmöglich.
- Die Nachweisverfahren sind nur schwer zu automatisieren.

Es war Aufgabe der vorliegenden Erfindung, ein Verfahren zur Detektion und Analyse von Protein-Interaktionen bereitzustellen, welches die obigen Nachteile des Standes der Technik überwindet. Insbesondere war es Aufgabe der vorliegenden Erfindung, ein Verfahren bereitzustellen, welches auch schwache Protein-Interaktionen und/oder stimulus-induzierte Protein-Interaktionen transienter Natur durch die Protein-Interaktions-abhängige Erzeugung eines permanenten Detektionssignals, welches nicht mehr von der Analyse in einem engen Zeitfenster abhängig ist, der Analyse zugänglich macht. Insbesondere war es auch Aufgabe der Erfindung, ein Verfahren zur Analyse von Protein-Interaktionen bereitzustellen, bei dem ein Detektionssignal zum Nachweis spezifischer Protein-Interaktionen erzeugt wird, welches gegenüber der klassischen Aktivierung eines Reporter-Gens durch vorübergehende Transkriptionsaktivierung verstärkt, d.h. amplifiziert ist.

Die erfindungsgemäße Aufgabe wird gelöst durch die Gegenstände der Ansprüche 1 bis 14.

In den erfindungsgemäßenVerfahrenzur Detektion und Analyse von Protein-Interaktionen in einer Zelle kann
a) der Enzymaktivität, nämlicher einer einer TEV-Protease und ggf. zusätzlich einer Rekombinase, in der Zelle als Folge einer Protein-Interaktion bereitgestellt werden.

### Dadurch kann erfolgen

b) eine andauernde Generierung eines aktiven Reporterproteins in der betreffenden Zelle und gegebenenfalls in den Tochterzellen dieser Zelle als Folge der Enzymaktivität aus Schritt a) für einen Zeitraum, der über die Dauer der Protein-Interaktion aus Schritt a) hinausgeht.

### Dadurch kann

c) eine Erzeugung eines Detektionssignals durch die in b) generierten Reporterproteine erfolgen.

Die hier beschriebenen erfindungsgemäßeVerfahren sind geeignet, Protein-Interaktionen zu analysieren,
- die transienter Natur sind
- die sehr schwach sind
- die von spezifischen Stimuli abhängig sind und nur in vivo stattfinden
- die von den intrinsischen Eigenschaften bestimmter eukaryontischer Zellen abhängig sind
- die von einem bestimmten Zellzustand abhängig sind
- die von spezifischen Modifikationen abhängig sind
- die von einer komplexen Topologie und Umgebung abhängig sind
- die an die Funktion von Proteinkomplexen gekoppelt sind.

Mit den erfindungsgemäßen Verfahren kann eine Protein-Interaktion in allen lebenden Zellen oder Zellverbänden nachgewiesen werden und kann vollständig in vivo erfolgen. Ein biochemischer Eingriff ist hierbei nicht zwingend notwendig. Weiterhin erfolgt die Verstärkung des Detektionssignals nicht graduell, d.h. in Abhängigkeit der Interaktionsstärke, sondern ist durch das Anschalten eines permanent stark exprimierten Reportergens oder durch die andauernde Neugenerierung von aktiven Reporterproteinen auch über das Bestehen der Protein-Interaktion hinaus maximal.

Der Zeitpunkt der Analyse kann zeitlich entkoppelt von der möglicherweise Stimulus-abhängigen und/oder transienten Interaktion stattfinden. Die synchronisierte Analyse einer Vielzahl potentieller Interaktionspartner, wie z.B. im Screening-Verfahren, wird damit möglich. Weiterhin ist es möglich in einem Experiment zeitlich hintereinander geschaltete Stimuli unterschiedlicher Signaltransduktionswege und deren möglichen Einfluß auf regulierte Protein-Interaktionen auch unbekannter Partner zu untersuchen oder den Einfluß niedermolekularer Wirkstoffe auf Protein-Interaktionen zu analysieren. Der Nachweis der Reporter ist experimentell einfach durchzuführen und kompatibel mit HochdurchsatzVerfahren, wie z.B. "high throughput Screening"-Verfahren. Ein Protein-Interaktionsabhängiges kurzzeitiges Aktivieren des Reportersystems reicht bei dem erfindungsgemäßen Verfahren schon aus, um ein permanentes, stabiles und in vivo langfristig detektierbares Signal zu generieren. Darüberhinaus erfährt das Signal durch die andauernde Neu-Generierung des aktiven Reporterproteins für einen Zeitraum, der über das Bestehen der Protein-Interaktion hinausgeht, und durch die dadurch gegebenenfalls auftretende Akkumulation des aktiven Reporterproteins in der Zelle eine enorme Verstärkung. Die Verstärkung unterliegt hierbei einem "Alles oder Nichts-Prinzip", wobei ungeachtet der ursprünglichen Stärke der Interaktion das Detektionssignnal maximal aktiviert wird, was sich vor allem bei schwachen und/oder stimulus-induzierten Protein-Interaktionen transienter Natur als Vorteil erweist.

Weiterhin bietet die beim erfindungsgemäßen Verfahren räumliche Trennung der Protein-Interaktion vom Ort der Signal-Generierung gegenüber einigen Verfahren nach dem Stand der Technik den Vorteil, daß der Hintergrund falsch positiver Signale reduziert sein sollte. Diese Eigenschaften stellen eine deutliche Verbesserung gegenüber den bestehenden Systemen dar. Die Modularität und enorme Flexibilität des System erlaubt es, die Analysen und Experimente sehr fein auf die jeweilige Fragestellung bzw. auf die Stärke der gesuchten Interaktion einzustellen.

Bei dem erfindungsgemäßen Verfahren zur Detektion und Analyse von Protein-Interaktionen in einer Zelle wird eine Enzymaktivität, nämlich die einer TEV-Protease und ggf. zusätzlich die einer Rekombinase als Folge einer spezifischen Protein-Interaktion bereitgestellt. Die Aktivität des entsprechenden Enzyms in der betreffenden Zelle kann hierbei vorzugsweise entweder durch die Induzierung oder Steigerung der Expression des Enzyms als Folge der Protein-Interaktion, durch die Aktivierung - insbesondere durch die proteolytische Aktivierung - des Enzyms als Folge der Protein-Interaktion, oder durch die Transkomplementierung des Enzyms als Folge der Protein-Interaktion bereitgestellt werden. Bei der Transkomplementierung des Enzyms hat das Auftreten einer spezifischen Protein-Interaktion zur Folge, daß zwei Fusionsproteine, die beide jeweils einen Teil der Aminosäuresequenz des Enzyms (also der TEV-Protease) umfassen, in räumliche Nähe zueinander geraten und so ein transkomplementiertes, funktionales Enzym bilden können.

Die Enzymaktivität einer TEV-Protease und ggf. zusätzlich die einer Rekombinase hat zur Folge, daß ein aktives Reporterprotein in der betreffenden Zelle und gegebenenfalls in den Tochterzellen dieser Zelle für einen Zeitraum, der über das Bestehen der Protein-Interaktion hinausgeht, andauernd generiert wird. Hierbei ist der Zeitraum, der über das Bestehen der Protein-Interaktion hinausgeht, vorzugsweise der Zeitraum, der die gesamte Lebensdauer der betreffenden Zelle erfasst. Stärker bevorzugt wird das aktive Reporterprotein in der betreffenden Zelle und zusätzlich auch in den Tochterzellen der betreffenden Zelle über einen solchen Zeitraum hinweg andauernd generiert, der die gesamte Lebensdauer der Tochterzellen umfasst. Durch diesen in der Regel sehr langen Zeitraum, in dem das aktive Reporterprotein in der betreffenden Zelle oder zusätzlich sogar auch in deren Tochterzelle andauernd neu generiert wird, erfolgt vorzugsweise auch eine gewisse Akkumulierung des aktiven Reporterproteins in der betreffenden Zelle und/oder in deren Tochterzelle.

In einem weiteren Schritt wird durch das generierte aktive Reporterprotein ein Detektionssignal erzeugt und gemessen. Die Akkumulierung des aktiven Reporterproteins führt letztlich dazu, daß im erfindungsgemäßen Verfahren ein Detektionssignal erzeugt wird, welches gegenüber einem Detektionssignal verstärkt ist, das durch ein lediglich während der Dauer der Protein-Interaktion generiertes - und daher geringer akkumuliertes - Reporterprotein erzeugt wurde. Das Detektionssignal kann die Fluoreszenz eines fluoreszierenden Reporterproteins wie insbesondere GFP und seine Varianten oder Luciferase sein. Das Detektionssignal kann jedoch auch das von einem als Reporter fungierenden Enzym umgesetzte Substrat sein wie insbesondere bei einem β-Galaktosidase-Assay. Weiterhin können Resistenz-vermittelnde Gene oder auch Gene zur Wachstumsselektion als Reporter-Proteine und die aus der Expression dieser Gene resultierenden Resistenzen oder Wachstumskonditionen als Detektionssignal eingesetzt werden.

Das dem Verfahren zugrunde liegende Prinzip beruht darauf, daß eine spezifische Protein-Interaktion zunächst zur andauernden Neugenerierung einen aktiven Reporterproteins in der Zelle in ein permanentes Signal umsetzt. Die Aktivierung des Signals erfolgt durch einen oder mehrere miteinander gekoppelte molekulare Schalter-Systeme, an denen Proteasen und ggf. zusätzlich Rekombinasen beteiligt sind. Das System kann so ausgestaltet sein, daß die Dynamik von Protein-Interaktionen, ihr Zustandekommen sowie ihre Dissoziierung, analysiert werden kann.

Im erfindungsgemäßen Verfahren wird Protein-Interaktions-abhängig die Aktivität einer TEV-Protease und ggf. zusätzlich einer Rekombinase oder einer weiteren Protease in der Zelle bereitgestellt und in ein permanentes Detektionssignal der Zelle umgesetzt. Durch die Aktivität einer TEV-Protease wird in der Zelle ein schalterartiger Mechanismus in Gang gesetzt, der letzlich zur Ausbildung eines permanenten und eines gegenüber der Aktivierung eines Reporter-Gens durch klassische Transkriptionsaktivierung stark amplifizierten Detektionssignal der Zelle führt.

DNA-Doppelstrang-spezifische Rekombinasen der Integrase-Familie können den Austausch oder die Integration von verschiedenen nicht-homologen DNA-Molekülen vermitteln (Lewandoski 2001). Die Integrase-Protein-Familie umfasst mehr als 100 bekannte Mitglieder verschiedener Spezies (zusammengefasst in (Nunes-Duby, Kwon et al. 1998) und in (Esposito and Scocca 1997). Als molekularer Schalter in transgenen Tieren oder in Zellkultur wird im wesentlichen die Cre-Rekombinase des P1-Bakteriophagen und die Hefe-Rekombinase FLP verwendet (Sauer 1998) (Buchholz, Angrand et al. 1998). Durch die Aktivität einer zelltyp-spezifischen oder einer während der Entwicklung reguliert exprimierten Rekombinase kann ein nachgeschaltetes Reportergen in vivo aktiviert werden. Damit können verschiedene molekulare Marker dauerhaft aktiviert werden und zu einer Vielzahl von Analysen im Tier genutzt werden (Lewandoski 2001). Die Cre-Rekombinase wurde auch in Säugerzellen als Reportergen zur Analyse von Signaltranduktionsmechanismen verwendet (Mattheakis, Olivan et al. 1999). Weiterhin sind Cre-Rekombinase-basierte Genregulationssysteme beschrieben worden, mit denen Zielgene induzierbar angeschaltet oder inaktiviert werden können und auf Ligandenkontrolliertem Kernimport von Fusionsproteinen der Cre-Rekombinase mit Ligandenbindenden-Domänen verschiedener Kernrezeptoren beruhen (Kellendonk, Tronche et al. 1996) (Feil, Brocard et al. 1996) (Metzger, Clifford et al. 1995). DNA-Doppelstrang-spezifische Rekombinasen, die Protein-Interaktionsabhängig ein Reportergen dauerhaft aktivieren können, stellen ein ideales molekulares Schalter-System dar, um transiente Interaktionen in dauerhafte Signale umzusetzen. Rekombinasen, sowie die großen Vorteile, die sie durch die Erzeugung von permanenten und gegenüber der klassischen Transkriptionsaktivierung stark amplifizierten Detektionssignalen für die Analyse von Protein-Interaktionen bieten, sind im Zusammenhang mit der Analyse von Protein Interaktionen bisher nicht beschrieben worden.

Im erfindungsgemäßen Verfahren kann die Aktivität einer Rekombinase durch Protein-Interaktions-abhängigen Transport vom Zytoplasma in den Kern oder Transkriptionsfaktor-abhängig als Reportergen exprimiert werden. Die Induktion der Transkription der Rekombinase kann durch Protein-Interaktions-abhängig durch Transkomplementation, z.B. durch die funktionelle Rekonstituierung eines Transkriptionsfaktors oder durch Protein-Interaktions-abhängigen Transport eines funktionellen Transkriptionsaktivators vom Zytoplasma in den Kern erfolgen.

Für die Regulation der Aktivität der Cre-Rekombinase als transkriptionelles Reportergen oder durch kontrollierten Kernimport sowohl für in vitro- als auch für in vivo-Anwendungen zur Aktivierung nachgeschalteter Gene gibt es eine Vielzahl von Studien (Lewandoski 2001). In keiner dieser Studien wird jedoch die Cre-Rekombinase als transkriptionelles Reportergen oder durch kontrollierten Kernimport zur Analyse neuer oder bekannter Protein-Interaktionen genutzt. Ebenso sind keine Untersuchungen über die Transkomplementation einer Rekombinase oder Rekombinaseaktivität im Zusammenhang mit der Analyse von Protein-Interaktionen beschrieben. Die Cre-Rekombinase bindet als Protein-Dimer an jeweils eine doppelsträngige DNA-Erkennungssequenz, die sogenannte loxP-Erkennungssequenz. Der aktive rekombinations-vermittelnde Komplex wird durch ein Homo-Tetramer und zwei loxP-Erkennungssequenzen ausgebildet. Intermolekulare Transkomplementationen mutanter Cre-Proteine, gekennzeichnet durch den Verlust unterschiedlicher Funktionen, wurden beschrieben (Shaikh and Sadowski 2000). Diese Untersuchungen lassen den Schluß zu, daß eine inter-molekulare Transkomplementation verschiedener Cre-Mutanten als Strategie zur Analyse von Protein-Interaktionen möglich ist. Ebenso erscheint eine intramolekulare Transkomplementationsstrategie aufgrund von funktionalen Analysen von chimären Proteinen der FLP- und Cre-Rekombinasen und insbesondere aufgrund der bekannten Kristallstruktur des Proteins möglich (Guo, Gopaul et al. 1997). Das Cre-Protein faltet sich in zwei distinkte globuläre Domänen (AS 20-129 und AS 132-341), die durch einen kurzen Abschnitt verbunden sind.

Anwendungsabhängig können durch die Rekombinase aktivierte, nachgeschaltete Reporter-Systeme folgende sein:
- direkt in vivo und in vitro nachweisbare und quantifizierbare Proteine (Epi- bzw. autofluoreszente Proteine wie z.B. das green fluorescent protein GFP und seine Derivate oder Aequorin).
- indirekt in vivo und in vitro nachweisbare und quantifizierbare Enzyme (Luziferase, beta-Galaktosidase, alkalische Phosphatase, beta-Lactamase, etc.).
- Exponierte Oberflächenproteine, die biochemisch nachweisbar sind bzw. zur Affinitätsisolation von Zellen geeignet sind.
- Resistenz gegen zytotoxische Substanzen oder Minimalmedien vermittelnde Proteine oder Enzyme (Neomyzin/G418, Puromyzin, Blastizidin S, Zeocin, Ampicillin, Kanamycin, Gentamycin, Tetrazyklin, Xanthine-Guanine Phophoribosyl Transferase (XGPT) etc.).
- zytotoxische oder pro-apoptotische Proteine (Diphterie Toxin, aktivierte Caspasen etc.)
- Proteine, die das Wachstum oder die Morphologie der Zelle ändern, in der sie exprimiert werden.

Erfindungsgemäß kann ggf. zusätzlich in Verfahrensschritt a) Protein-Interaktionsabhängig die Aktivität einer Rekombinase durch die Transfektion oder Infektion der Zelle mit einem Expressionsvektor bereitgestellt werden, wobei der Expressionsvektor ein Rekombinase-Gen unter der Kontrolle eines Transkriptionsfaktors umfasst und die andauernde Generierung des aktiven Reporterproteins in der betreffenden Zelle nach Verfahrensschritt b) wird durch die Einzelschritte b1) bis b3) durchgeführt:
b1) Transfektion oder Infektion der Zelle mit einem Konstrukt, das eine von Rekombinationsstellen flankierte Stop-Kassette mit der nachgeschalteten Nukleotidsequenz eines Reporter-Gens unter der Kontrolle eines konstitutiven Promoters umfaßt,
b2) Entfernung der von Rekombinationsstellen flankierten Stop-Kassette des Konstruktes mittels der Rekombinase aus Verfahrensschritt a),
b3) konstitutive Expression des Reporter-Gens.

Transfektionen oder Infektionen der Zelle werden nach Standardmethoden entweder transient oder stabil durchgerührt.

Wird in einer solchen Zelle Protein-Interaktions-abhängig die Expression der Rekombinase induziert, so kann durch diese Rekombinase die von den Rekombinase-Erkennungsstellen flankierte Stop-Kassette aus der Nukleotidsequenz des Reporter-Gens herausgeschnitten werden. Dadurch kommt es zur Aktivierung des nachgeschalteten Reporter-Gens und damit zur permanenten Expression und zur ständigen Akkumulation des funktionalen Reporter-Proteins in der Zelle.

Unter einer Stop-Kassette ist in diesem Zusammenhang eine DNA-Insertion zu verstehen, die in das Reporter-Gen auf die Art und Weise inseriert ist, daß ihre Gegenwart zu einer Inaktivierung des Reporter-Gens führt. Nach Entfernung der von den Rekombinationsstellen flankierten Stop-Kassette durch die Protein-Interaktions-abhängig bereitgestellte Rekombinase-Aktivität steht einer dauerhaften Aktivierung des Reporter-Gens und damit einer ständigen Akkumulation an beispielsweise fluoreszierendem Reporterprotein oder an beispielsweise enzymatischem Reporter-Protein nichts mehr entgegen. Die Akkumulation an Reporter-Protein führt weiterhin zu einer erheblichen Verstärkung des Detektionssignals im Vergleich zur Reporter-Gen-Aktivierung durch klassische Transkriptionsaktivierung. Das Ausmaß der Amplifikation des Signals wird hierbei lediglich von der Expressionshöhe des Reportergens oder auch vom Umsatz der enzymatischen Aktivität des Reporter-Gens begrenzt, nicht aber von der Dauer oder der Stärke der zu detektierenden Protein-Interaktion.

Die Erfindung betrifft auch ein weiteres hier beschriebenes Schaltersystem auf molekularer Ebene, welches auf der Protein-Interaktions-abhängig bereitgestellten Aktivität einer TEV- Protease bzw. auf der Kopplung einer TEV-Protease-Aktivität mit einem durch proteolytische Prozessierung aktivierten Reporter basiert. Die kurzzeitige Aktivität einer TEV-Protease kann zur Generierung eines dauerhaften Detektionssignals als Folge der andauernden Generierung eines aktiven Reporterproteins über die Dauer der Protein-Interaktion hinaus in der Zelle führen.

Die Erfindung betrifft auch Verfahren, die zwei molekularen Schalter-Systeme nutzen, eines basierend auf einer Rekombinase- und eines basierend auf einer TEV-Protease-Aktivität, sowie Verfahren, die nur auf der Aktivität eines der molekularen Schalter-Systeme der TEV-Protease-Aktivität beruhen.

In dem erfindungsgemäßen Verfahren wird die TEV-Protease-Aktivität durch die Transkomplementation einer funktionalen TEV-Protease, bereitgestellt.

Die Transkomplementation der TEV-Protease kann - wie Beispiel 8 zeigt - durch die Teilung der Protease-Sequenz an beliebigen Positionen des Proteins erfolgen, aber insbesondere an den Positionen zwischen Aminosäure 60 und 80 sowie an der Position zwischen Aminosäure 95 und 120 der TEV-Protease. Die beiden Teile der TEV-Protease können hierbei auch überlappen wie Beispiel 8 ebenfalls zeigt. Die Expression der beiden zu komplementierenden TEV-Protease-Fragmente jeweils als Fusionsprotein mit einen potentiell interagierenden weiteren Protein führen zu Fusionsproteinen umfassen Protease-Fragmente, die jedes für sich genommen keine Protease-Aktivität mehr besitzen. Die jeweiligen Protease-Fragmente führen jedoch - sofern die Fusionsproteine aufgrund einer Interaktion der beiden potentiell interagierenden Proteine in räumliche Nähe zueinander geraten - zu einer funktionalen TEV-Protease.

Die Erfindung betrifft daher ein Verfahren zur Detektion und Analyse von Protein-Interaktionen in einer Zelle, umfassend die Schritte
e) Expression eines
   e1) ersten Fusionsproteins, umfassend den ersten Interaktionspartner und einen Teil einer TEV-Protease, und
   e2) eines zweiten Fusionsproteins, umfassend den zweiten Interaktionspartner und einen weiteren Teil der TEV-Protease,
      wobei gegebenenfalls mindestens eines der beiden Fusionsproteine eine weitere Domäne besitzt, die zur Verankerung des Fusionsproteins außerhalb des Zellkerns führt, und
   e3) Expression einer funktionalen Rekombinase,
      welche gegebenenfalls eine weitere Domäne des ersten oder zweiten Fusionsproteins darstellt und von den übrigen Domänen durch eine Erkennungs- und Schnittstelle für die TEV-Protease proteolytisch abspaltbar ist, oder
      Expression einer funktionalen Rekombinase
      auf einem dritten Fusionsprotein, welches neben der funktionalen Rekombinase selbst, die durch eine Erkennungs- und Schnittstelle für die TEV-Protease proteolytisch abspaltbar ist, eine weitere Domäne umfaßt, die zur Verankerung des dritten Fusionsproteins außerhalb des Zellkerns führt,
   in einer Zelle,
f) Rekonstituierung einer funktionalen TEV-Protease durch die Interaktion des ersten und des zweiten Interaktionspartners miteinander,
g) proteolytisches Abspalten der funktionalen Rekombinase von seiner Verankerung außerhalb des Zellkerns durch die rekonstituierte TEV-Protease aus f),
h) Transport der funktionalen Rekombinase in den Zellkern und Aktivierung eines Rekombinase-abhängigen Reporter-Gens, wodurch ein Detektionssignal erzeugt wird.

Die Komponente e3) besitzt hierbei vorzugsweise eine Domäne zu ihrer Verankerung außerhalb des Zellkerns, welche zu einer Verankerung an der Zellmembran führt.

Darüberhinaus kann die zytoplasmatische Struktur außerhalb des Zellkerns, an der mindestens eines der beiden Fusionsproteine über eine dazu geeignete Domäne verankert ist, jedoch auch jedes andere von einer Membran umschlossene Zellorganell, mit Ausnahme des Zellkerns sein. Auch bestimmte proteolytisch abspaltbare Protein-Targeting-Domänen, die ein Targeting des Trägerproteins in das Lumen eines bestimmten Zellorganells bewirken, können zur Fixierung mindestens eines der beiden Fusionsproteine außerhalb des Zellkerns dienen.

In einer weiteren bevorzugten Ausführungsform des erfindungsgemäßen Verfahrens wird der spezifische funktionale Transkriptionsfaktor im Zellkern der Zelle durch die Protein-Interaktions-abhängige Transkomplementation einer TEV-Protease bereitgestellt.

Diese Protein-Interaktions-abhängige Transkomplementation der Protease kann insbesondere durch die folgenden Schritte erreicht werden:
p) Expression eines
   p1) ersten Fusionsproteins, umfassend den ersten Interaktionspartner und einen Teil einer TEV-Protease, und
   p2) eines zweiten Fusionsproteins, umfassend den zweiten Interaktionspartner und einen weiteren Teil der TEV-Protease,
      wobei gegebenenfalls mindestens eines der beiden Fusionsproteine eine weitere Domäne besitzt, die zur Verankerung des Fusionsproteins außerhalb des Zellkerns führt, und
   p3) Expression eines funktionalen Transkriptionsfaktors,
      welcher gegebenenfalls eine weitere Domäne des ersten oder zweiten Fusionsproteins darstellt und von den übrigen Domänen des Fusionsproteins durch eine Erkennungs- und Schnittstelle für eine TEV-Protease proteolytisch abspaltbar ist, oder
      Expression eines funktionalen Transkriptionsfaktors auf einem dritten Fusionsprotein, welches neben dem funktionalen Transkriptionsfaktor selbst, der durch eine Erkennungs- und Schnittstelle für die TEV-Protease proteolytisch abspaltbar ist, eine weitere Domäne umfaßt, die zur Verankerung des dritten Fusionsproteins außerhalb des Zellkerns führt;
      in einer Zelle;
q) Rekonstituierung einer funktionalen TEV-Protease durch die Interaktion des ersten und des zweiten Interaktionspartners miteinander;
r) proteolytisches Abspalten des funktionalen Transkriptionsfaktors von seiner Verankerung außerhalb des Zellkerns durch die rekonstituierte TEV-Protease aus q)
s) Bereitstellung eines funktionalen Transkriptionsfaktors im Zellkern und anschließende Induktion der Expression eines Rekombinase-abhängigen oder eines Rekombinase-unabhängigen, klassischen Reporter-Systems, wodurch ein Detektionssignal erzeugt wird.

In einer bevorzugten Abwandlung des Verfahrens führt der in Schritt r) proteolytisch abgespaltene funktionale Transkriptionsfaktor in Schritt s) im Zellkern
- zur Induktion der Expression eines Rekombinase-unabhängigen, klassischen Reporter-Systems
- und zusätzlich zur Induktion der Expression des Gens einer funktionalen Protease zur weiteren dauerhaften Aktivierung des eingesetzten Reporter-Systems.

Die zusätzliche Transkriptionsaktivierung des Gens einer funktionalen Protease durch die Aktivität des funktionalen Transkriptionsfaktors führt hierbei zu einer andauernden Generierung eines aktiven Reporterproteins ohne die Verwendung einer Rekombinase-Aktivität.

Des Weiteren betrifft die Erfindung ein Verfahren zur Detektion und Analyse von Protein-Interaktionen in einer Zelle, umfassend die Schritte
u) Expression eines
   u1) ersten Fusionsproteins, umfassend den ersten Interaktionspartner und einen Teil einer Tobacco-Etch-Virus-Protease (TEV-Protease) und
   u2) eines zweiten Fusionsproteins, umfassend den zweiten Interaktionspartner und einen weiteren Teil der TEV-Protease, und
   u3) eines durch Proteolyse aktivierbaren oder inaktivierbaren Reporterproteins ausgewählt aus der Gruppe, bestehend aus fluoreszierenden Reporterproteinen, Luciferase, beta-Galaktosidase, alkalische Phosphatase, beta-Lactamase, Resistenz gegen zytotoxische Substanzen vermittelnde Proteine, zytotoxische Proteinen, Proteinen, die Wachstum und Morphologie der Zelle ändern und Rekombinasen,
in der Zelle,
v) Rekonstituierung einer funktionalen TEV-Protease durch die Interaktion des ersten und des zweiten Interaktionspartners miteinander,
w) Aktivierung des durch Proteolyse aktivierbaren oder Inaktivierung des durch Proteolyse inaktivierbaren Reporterproteins durch die rekonstituierte funktionale TEV-Protease aus Schritt v).

Vorzugsweise wird in Schritt u) als Komponente u3) ein durch Proteolyse aktivierbares Reporterprotein exprimiert, dessen Reporteraktivität durch die Insertion einer zusätzlichen Aminosäuresequenz und/oder durch die Insertion von mindestens einer Erkennungs- und Schnittstelle für eine TEV-Protease inaktiviert ist und durch die Aktivität einer TEV-Protease proteolytisch aktivierbar ist.

Alternativ dazu kann jedoch auch in Schritt u) als Komponente u3) ein durch Proteolyse inaktivierbares Reporterprotein exprimiert werden, das mindestens eine Erkennungs- und Schnittstellen für eine TEV-Protease enthält und dessen Reporteraktivität proteolytisch inaktivierbar ist.

Die Erfindung betrifft weiterhin ein Verfahren zur Detektion und Analyse von Protein-Interaktionen in einer Zelle, umfassend die folgenden Schritte:
J) Expression eines
   J1) ersten Fusionsproteins, umfassend den ersten Interaktionspartner und einen Teil einer TEV-Protease, und
   J2) eines zweiten Fusionsproteins, umfassend den zweiten Interaktionspartner und einen weiteren Teil der TEV-Protease, und
   J3) konstitutive Expression eines über eine geeignete Domäne außerhalb des Zellkerns verankerten Reporter-Proteins, welches von dieser Verankerung proteolytisch abspaltbar ist, und zusätzlich eine weitere Domäne umfaßt, welche nach der proteolytischen Abspaltung die Lokalisation des Reporter-Proteins in ein bestimmtes Kompartiment der Zelle bewirkt;
   in der Zelle;
K) Rekonstituierung einer funktionalen TEV-Protease durch die Interaktion des ersten und des zweiten Interaktionspartners miteinander;
L) Proteolytische Abspaltung des Reporter-Proteins mitsamt seiner Domäne, die die Lokalisation des freien Reporter-Proteins in ein bestimmtes Kompartiment der Zelle bewirkt, durch die funktionale TEV-Protease aus Schritt K);
M) Detektion der veränderten Lokalisation des Reporter-Proteins.

Zur Isolierung neuer Proteasen oder Protease-regulierender Proteine oder Moleküle wurden für die Anwendung in Hefe bereits Nachweis-Systeme entwickelt, die auf der proteolytischen Abspaltung von membran-gebundenen Transkriptionsfaktoren beruhen (Kamada, Kusano et al. 1998) (Hirowatari, Hijikata et al. 1995) (Broad 1999). Die Verwendung von exogenen Proteasen ist im Zusammenhang mit der Analyse von Protein-Interaktionen bisher nicht beschrieben.

Eine solche proteolytische Aktivität kann -wie oben im Detail beschrieben- durch Transkomplementation erfolgen. Anwendungsabhängig können die durch die proteolytische Spaltung aktivierten Proteine oder Enzyme folgende sein:
- Eine ausserhalb des Zellkerns gebundene Rekombinase, die erst nach proteolytischer Abspaltung in den Kern gelangt und dort aktiv werden kann.
- Ein ausserhalb des Zellkerns fixierter Transkriptionsfaktor, der erst nach proteolytischer Abspaltung in den Kern gelangt und dort aktiv werden kann.
- Ein ausserhalb des Zellkerns fixierte GFP-Variante, die erst nach proteolytischer Abspaltung in den Kern gelangt. Der Nachweis erfolgt durch die veränderte Morphologie des Fluoreszenz Signals.
- Ein durch proteolytische Spaltung aktivierbares oder inaktivierbares Enzym oder Protein- oder Molekül oder Protein- oder Molekülpaar. Dies können Rekombinasen, Proteasen, GFP-Varianten, Enzyme oder zelluläre Signalproteine sein.
- Eine modifizierte Protease, die ebenfalls erst nach proteolytischer Spaltung aktiv werden kann. Letzteres führt zu einer dauerhaften und maximalen Signalaktivierung durch eine Kaskade aufeinanderfolgender proteolytischer Spaltungen.
- Ein Transkriptionsaktivator, dessen Kernlokalisationssignal durch eine auf demselben oder einem weiteren Protein liegende und eine Proteaseerkennungsstelle enthaltende Proteindomäne so maskiert ist, daß dessen Kernlokalisationssignal erst nach proteolytischer Spaltung erkannt wird und er erst dann in den Kern gelangen kann.

Ein wesentliches Merkmal der vorliegenden Erfindung ist, daß Protein-Interaktionen in der Zelle dort erfaßt werden, wo sie natürlicherweise auch erfolgen, z.B. bei ER-Residenzproteinen im ER oder bei Oberflächenrezeptoren an der Plasmamembran.
Die Art der Verankerung und die daraus resultierende Lokalisation eines proteolytisch abspaltbaren Transkriptionsaktivators, einer Rekombinase oder eines anderen Protease-Sensors wie z.B. ein proteolytisch aktivierbares oder inaktivierbares Enzym bzw. ein proteolytisch aktivierbares oder inaktivierbares fluoreszierendes Protein ermöglicht es, Protein-Interaktionen an ihrem natürlichen Ort innerhalb der Zelle spezifisch zu detektieren und darüberhinaus zu untersuchen, an welchen Orten innerhalb der Zelle die diese Interaktion stattfindet. Das erfindungsgemäße Verfahren umfaßt daher verschiedene Methoden zur gezielten Verankerung bzw. Lokalisation von oben genannten Protease-Sensoren. Die proteolytisch abspaltbaren Proteine, die ihre Aktivität im Zellkern entfalten, wie Transkriptionsaktivatoren bzw.-faktoren oder Rekombinasen müssen dabei so verankert werden, daß sie auf der cytoplasmatischen Seite von Membranen oder Zellkompartimenten lokalisiert sind, so daß sie nach proteolytischer Abspaltung von der Verankerung in den Zellkern gelangen können. Anwendungsabhängig gehören dazu z.B. folgende Verankerungen:
- Für Lokalisationen an der ER-Membran Fusion des proteolytisch abspaltbaren Transkriptionsaktivators oder der Rekombinase oder eines Protease-Sensors (zusammengefaßt als zu verankernde Proteine) an den C-Terminus eines Membranproteins vom Typ I oder Typ III mit ER-Retentionssignal, das im ER residiert, wobei zwischen dem Verankerungsprotein und dem verankerten Protein eine Protease-Schnittstelle ist.
- Für Lokalisationen im Golgi-Apparat Fusion an Golgi-residierende Membranproteine oder alternativ Isoprenylierung des zu verankernden Proteins oder des Protease-Sensors durch Anhängen einer Protease-Schnittstelle gefolgt von einem Geranylgeranylierungssignal, z.B. CVIL, oder einem Farnesylierungssignal, z.B. CIIM an den C-Terminus.
- Für Lokalisationen an der Plasmamembran Fusion des zu verankernden Proteins an den C-Terminus eines Membranproteins vom Typ I oder Typ III bzw. an den C-Terminus des Transmembranbereichs eines solchen Membranproteins, wobei zwischen dem Verankerungsprotein und dem verankerten Protein eine Protease-Schnittstelle ist.
- Für Lokalisationen an der Plasmamembran Fusion des zu verankernden Proteins an den N-Terminus eines Membranproteins vom Typ II bzw. an den N-Terminus des Transmembranbereichs eines solchen Membranproteins, wobei zwischen dem Verankerungsprotein und dem verankerten Protein eine Protease-Schnittstelle ist.

Ferner für Lokalisationen an der Plasmamembran Fusion mit Proteindomänen die Lipidmodifikationen wie Myristoylierung oder Palmitoylierung tragen, wobei zwischen dem Verankerungsprotein und dem verankerten Protein eine Protease-Schnittstelle ist. Für Lokalisationen an Peroxisomen oder Mitochondrien Fusion an ein Membranprotein, das in der Peroxisomen-Membran bzw. der äußeren Membran der Mitochondrien lokalisiert ist, so, daß das verankerte Protein auf der cytoplasmatischen Seite liegt und über eine Protease-Schnittstelle abgespalten werden kann.
Zur Analyse der Interaktion von cytoplasmatischen Proteinen eignen sich Verankerungen des proteolytisch abspaltbaren Transkriptionsaktivators oder der Rekombinase bzw. eines Protease-Sensors am Cytoskelett der Zelle, z.B. durch Fusion an Actin.

Protease-Sensoren, die direkt ein meßbares Signal generieren können unabhängig von ihrerer Lokalisation, wie proteolytisch aktivierbare oder inaktivierbare fluoresziierende Proteine oder Enzyme können zusätzlich zu den oben genannten Methoden durch entsprechende Proteinfusionen auch auf der Membraninnenseite von Organellen verankert werden bzw. durch Organellen-spezifische Signalsequenzen in deren Lumen löslich vorliegen. Solche Signalsequenzen sind z.B. das für Peroxisomen spezifische Signal peroxysomal targeting signal 1 (PTS1, SKL) am C-Terminus von Proteinen oder N-terminale mitochondriale Targeting Sequenzen wie z.B. die ersten 31 Aminosäuren des Prepeptids der Untereinheit VIII der Cytochrom c Oxidase.

Proteolytische Enzyme, welche besonders geeignet für eine intrazelluläre Verwendung sind, sind die Potyvirus NIa Proteasen, wie insbesondere die 27 kDa NIa Protease des Tobacco Etch Virus. Diese Protease wird in eukaryontischen Zellen sehr gut toleriert und zeigt im Zytosol spezifische Aktivität (Faber, Kram et al. 2001) (Uhlmann, Wernic et al. 2000). Sie gehört zur C4-Familie der Cystein Proteasen, die Primärstruktur zeichnet sich durch die charakteristische Verteilung der Aminosäuren der katalytischen Triade, Histidin (Position 46), Aspartat (Position 81) und Cystein (Position 151), aus (Ryan and Flint 1997). Über die dreidimensionale Struktur ist hingegen wenig bekannt, eine Sekundärstrukturvorhersage und der Vergleich mit anderen Proteasen, deren 3D-Struktur bereits aufgelöst wurde, implizieren jedoch eine große Homologie zu den Trypsinähnlichen Serin-Proteasen (Bazan and Fletterick 1988) (Bazan and Fletterick 1989). Deren charakteristisches Strukturmerkmal ist der hohe Anteil von β-Faltblattdomänen in der Sekundärstruktur, die sich zu einer typischen bilobalen Gesamtstruktur falten. Dabei verteilen sich die katalytischen Aminosäuren Histidin und Arginin auf den N-terminalen Lobus und das Serin (bzw. Cystein) liegt auf dem C-terminalen Lobus. Diese Verteilung der drei katalytischen Aminosäuren auf die beiden "Hemisphären" der Protease dient als Grundlage für die Transkomplementations-Strategie. Hierbei sind mehrere Varianten denkbar, bei denen das Protein in zwei Fragmente geteilt wird, auf denen sich dann jeweils eine oder zwei der Aminosäuren der Triade finden. Entscheidend für die funktionelle Transkomplementation ist die unabhängige Faltung der Fragmente. Um diese zu gewährleisten, ist es u.U. notwendig, überlappende Fragmente zu generieren und diese auf Aktivität zu testen. Ziel der Transkomplementation ist es, die Fragmente so zu wählen, dass sie für sich gesehen keine Aktivität besitzen und diese Aktivität nur nur dann wiedererlangen, wenn sie an interagierende Proteine, interagierende Proteindomänen oder andere interagierende Moleküle fusioniert werden.

Aufgrund ihrer großen strukturellen Homologie (Auflistung in (Barrett-AJ, Rawlings-ND et al. 1998)) eignen sich für das Verfahren prinzipiell alle Proteasen. Vorraussetzung ist allerdings, daß ihre Anwesenheit in den entsprechenden Zellen oder Zellkompartimenten toleriert wird. Es wurde zudem die normalerweise in die Blutbahn sekretierte Protease Renin ebenfalls in PC-12 Zellen exprimiert. Mit einem Reportergenkonstrukt, welches mit der spezifischen Erkennungsstelle aus dem Reninsubstrat Angiotensinogen ausgestattet war, konnte die intrazelluläre Aktivität nachgewiesen werden. Renin eignet sich theoretisch sehr gut für eine Transkomplementationsstrategie, da diese Protease große Homologie zur HIV-Protease 1 aufweist, von der wiederum bekannt ist, daß das funktionelle Molekül aus zwei identischen Untereinheiten aufgebaut ist.

Darüberhinaus ist es auch möglich, die Transkomplementation einer Protease an der Zelloberfläche oder extrazellulär durchzuführen. Zu diesem Zweck müssten die Fragmente und u.U. Reporter in einer solchen Weise an Membranproteine, Proteine der Extrazellulärmatrix oder sezernierte Proteine fusioniert werden, daß sie ins Außenmedium der Zelle ragen oder sezerniert werden. Die Aktivität kann in einer solchen Version des Verfahrens durch Zugabe eines Substrates oder Ko-Expression eines entsprechend modifizierten Reporters detektiert werden. Der Umsatz eines spezifischen Substrates (z.B. durch fluoreszenz-gekoppelte Substrat-Peptide oder Enzyme deren Aktivität durch spezifische Proteolyse zerstört wird) erlaubt letztendlich die Transkomplementation völlig zellfrei zu analysieren, indem man rekombinant bzw. in vitro produzierte Fusionsproteine im Reaktionsgefäß untersucht.

Intrazellulär kommt es, direkt oder indirekt, durch die Protein-Interaktions-abhängige Aktivität einer TEV-Protease und ggf. zusätzlich einer Rekombinase oder einer weiteren Protease in den oben beschriebenen erfindungsgemäßen Ausführungsformen zu einer dauerhaften Aktivierung entsprechender Reportergene oder Reporterproteine in der Zelle. Eine Kombination aus beiden Systemen (dem Rekombinase-basierten und dem Proteasebasierten Schalter-System) erlaubt eine hohe Sensitivität und bietet viele Möglichkeiten zur Feinregulation der Detektionsgrenze, bzw. des Signal-Hintergrund-Verhältnisses.

Die molekularen Schalter-Systeme basieren auf einer TEV-Protease-Aktivität und ggf. zusätzlich einer Rekombinase oder einer weiteren Protease und können auch molekulare Rückkoppelungsmechanismen involvieren, die letztlich zu einer quasi-endlosen Aktivierung des Reporters führen.

Eine bevorzugte Ausführungsform des erfindungsgemäßen Verfahrens, die einen solchen molekularen Rückkoppelungsmechanismus zur quasi-endlosen Signalverstärkung umfaßt, beinhaltet die folgenden Einzelschritte:
A) Expression eines
   A1) ersten Fusionsproteins, umfassend den ersten Interaktionspartner und einen Teil einer TEV-Protease, und
   A2) eines zweiten Fusionsproteins, umfassend den zweiten Interaktionspartner und einen weiteren Teil der TEV-Protease, und
   A3) einer Protease, die durch Proteolyse aktivierbar oder durch Proteolyse inaktivierbar ist,
   in der Zelle;
B) Transkomplementation einer funktionalen TEV-Protease durch die Interaktion des ersten und des zweiten Interaktionspartners miteinander;
C) Aktivierung der Proteasen, die proteolytisch aktivierbar sind, durch die transkomplementierte funktionale TEV-Protease aus Schritt B);
D) Aktivierung oder Inaktivierung eines proteolytisch aktivierbaren oder eines proteolytisch inaktivierbaren Reporter-Systems durch die funktionalen Proteasen aus den Schritten B) und C). Dadurch wird ein Detektionssignal erzeugt.

Bei dieser Ausführungsform bewirkt zunächst das Auftreten einer spezifischen Protein-Interaktion eine Transkomplementation der TEV-Protease. Die durch Proteininteraktion transkomplementierte funktionale TEV-Protease kann dann sowohl eine konstitutiv in der Zelle exprimierte Protease, die selbst proteolytisch aktivierbar ist, durch Proteolyse aktivieren als auch das ebenfalls proteolytisch aktivierbare, konstitutiv exprimierte Reporter-Protein durch Proteolyse aktivieren. Die -ähnlich wie in einer Kettenreaktionansteigende Anzahl von durch Proteolyse aktivierten funktionalen Protease-Moleküle können schließlich zu einer dauerhaften, quasi-endlosen Bereitstellung des proteolytisch aktivierbaren, ebenfalls konstitutiv exprimierten Reporter-Proteins führen. Es ist bei dieser Ausführungsform alternativ auch möglich, ein durch Proteolyse inaktivierbares Reporter-Protein konstitutiv in der Zelle zu exprimieren.

Zu den klassischen Reportergenen, die in dieser Ausführungsform in proteolytisch aktivierbarer oder in proteolytisch inaktivierbarer Form in der Zelle exprimiert werden, werden in diesem Zusammenhang folgende Reporter gezählt:
- direkt in vivo und in vitro nachweisbare und quantifizierbare Proteine (Epi- bzw. autofluoreszente Proteine wie z.B. das green fluorescent protein GFP und Verwandte oder Aequorin).
- indirekt in vivo und in vitro nachweisbare und quantifizierbare Enzyme (Luziferase, beta-Galaktosidase, alkalische Phosphatase, beta-Lactamase, u.ä.).
- Exponierte Oberflächenproteine die biochemisch nachweisbar sind bzw. zur Affinitätsisolation von Zellen geeignet sind.
- Resistenz gegen zytotoxische Substanzen oder Minimalmedien vermittelnde Proteine oder Enzyme (Neomyzin, Puromyzin, Blastizidin S, Zeocin, Ampicillin, Kanamycin, Gentamycin, Tetrazyklin, Xanthine-Guanine Phophoribosyl Transferase (XGPT) u.ä.).
- zytotoxische oder pro-apoptotische Proteine (Diphterie Toxin, aktivierte Caspasen u.ä.)
- Proteine die Wachstum oder Morphologie der exprimierende Zelle ändern.

Zur lokalen Detektion der Interaktion losgelöst vom beschriebenen Reportersystem im Kern, können in einer Abwandlung alternative Reporter verwendet werden. Eine durch Proteininteraktion transkomplementierte Protease aktiviert eine proteolytisch aktivierbare Protease und ein durch Proteolyse aktivierbares Reporterprotein wie z.B. GFP. Die konstitutiv exprimierten Komponenten, die proteolytisch aktivierbare Protease und das proteolytisch aktivierbare Reporterprotein, werden sofort nach Synthese wieder gespalten, was zu einer andauernden Aktivierung führt. Der Nachweis kann prinzipiell in jedem Kompartiment und außerhalb der Zelle erfolgen. Die Komponenten werden nach Fusion mit entsprechenden Signalsequenzen in die entsprechenden Kompartimente sortiert und werden dort prozessiert und aktiviert. Der Nachweis erfolgt in situ.

Weitere Abwandlungen des obigen Verfahrens zur Analyse von Protein-Interaktionen mit einer quasi-endlosen Signalverstärkung stellen die folgenden Ausführungen 1.) und 2.) dar und werden im folgenden näher erläutert:
1. In dieser Abwandlung aktiviert eine durch eine spezifische Proteininteraktion transkomplementierte TEV-Protease proteolytisch einen Transkriptionsfaktor, der dann zusätzlich zu einem herkömmlichen Reportergen, wie z.B. beta-Galaktosidase, Luziferase, GFP-Varianten, etc., auch die Expression einer intakten Version der gleichen Protease anschaltet. Nach der Aktivierung der Komponenten kommt es so zu einer dauerhaften Aktivierung des Gesamtreportersystems: der konstitutiv exprimierte proteolytisch aktivierbare Transkriptionsfaktor wird unabhängig von einer weiteren durch Transkomplementation bereitgestellen TEV-Protease-Aktivität sofort nach seiner Synthese wieder gespalten, da die mit dem Reportergen koexprimierte Protease permanent zur Verfügung steht und sich in der Zelle akkumulieren kann.
2. In dieser weiteren Abwandlung wird eine proteolytisch aktivierbare Protease und ein durch Proteolyse aktivierbares Reporterprotein wie z.B. GFP oder seine Varianten, durch eine durch Proteininteraktion transkomplementierte TEV-Protease aktiviert. Die proteolytische Aktivierbarkeit des Reporter-Proteins bzw. der Protease kann beispielsweise durch das Einfügen einer oder mehrerer Erkennungsstellen für die Protease in das entsprechende Protein auf DNA-Ebene erreicht werden. Die konstitutiv exprimierten Komponenten, die proteolytisch aktivierbare Protease und das proteolytisch aktivierbare Reporterprotein, werden sofort nach ihrer Synthese proteolytisch gespalten und damit aktiviert. Auf diese Weise stehen nachfolgend immer ausreichend funktionale Proteasen zur Verfügung, die wiederum ständig die konstitutiv exprimierten proteolytisch aktivierbaren Reporterproteine aktivieren können. Das Detektionssignal verstärkt sich somit selbst und ist somit permanent.
   Der Nachweis kann prinzipiell auch in jedem Kompartiment der Zelle und auch außerhalb der Zelle erfolgen. Die Komponenten werden hierbei nach Fusion mit entsprechenden Signalsequenzen in die entsprechenden Kompartimente sortiert und werden dort prozessiert und aktiviert. Der Nachweis erfolgt hierbei in situ.
3. In dieser Abwandlung des Verfahrens wird ein durch Proteolyse aktivierbares oder ein durch Proteolyse inaktivierbares Reporter-Protein in der Zelle konstitutiv exprimiert, welches dann durch eine Protein-Interaktions-abhängige Transkomplementation einer TEV-Protease-Aktivität proteolytisch gespalten und damit -je nach Ausgestaltung des Systems- entweder aktiviert oder inaktiviert wird. Auch bei dieser Ausführungsform kommt es zu einer gewissen Verstärkung des Detektionssignals, da das konstitutiv exprimierte proteolytisch aktivierbare bzw. inaktivierbare Reporter-Protein sich in der Zelle akkumulieren kann und somit im Überschuß vorhanden ist. Eine weitere Potenzierung der Signalstärke kann durch den Einsatz von beispielsweise Enzymen mit quantifizierbarer Aktivität und mit hoher Umsatzrate als Reporter-Gen erreicht werden.

Die Verfahren können eingesetzt werden für Screening-Verfahren zur Identifizierung eines spezifischen Interaktionspartners eines Köder-Proteins.. Besonders bevorzugt sind hierbei Screening-Verfahren, bei denen eine cDNA-Bibliothek oder eine sogenannte ORF (open reading frame)- Bibliothek eingesetzt wird.

Zu den klassischen Reporter-Systemen zählen im Rahmen dieser Erfindung fluoreszierende Reporter-Proteine, wie GFP und alle seine kommerziell erhältlichen Varianten, Enzyme mit nachweisbarer Aktivität, wie Luciferase, beta-Galaktosidase, etc., oder Resistenz-vermittelnde Gene oder Gene, deren Expression zum Wachstum der Zellen unter bestimmten Mangelbedingungen notwendig ist. Weiterhin können alle proteolytisch aktivierbaren und proteolytisch inaktivierbaren Formen der oben genannten Reporter-Gen-Systeme bei den erfindungsgemäßen Ausführungsformen eingesetzt werden.

Die Zellen, die für die erfindungsgemäßen Verfahren verwendet werden, sind entweder Hefezellen, Bakterienzellen oder Zellen höherer eukaryontischer Organismen (insbesondere neuronale Zellen und Säuger-Zelllinien [z.B. Embryonale Karzinomazellen, Embryonale Stammzellen, P 19-, F9-, PC 12-, HEK293-, HeLa-, Cos-, BHK-, CHO-, NT2-, SHSY5Y-Zellen]).

Ein weiterer Gegenstand der Erfindung ist ein Kit zur Detektion und zur Analyse von Protein-Interaktionen in einer Zelle umfassend Expressionsvektoren, die die Nukleinsäurekonstrukte 1a) und 2a) jeweils unter der Transkriptionskontrolle eines heterologen Promotors umfassen:
1 a) ein erstes Nukleinsäurekonstrukt kodierend für ein erstes Fusionsprotein, umfassend die Nukleinsäuresequenz kodierend für ein erstes TEV-Proteasefragment und eine Klonierungsstelle, die sich zur Einklonierung des Köder-Proteins im Leseraster des ersten TEV-Proteasefragments eignet, und
2a) ein zweites Nukleinsäurekonstrukt kodierend für ein zweites Fusionsprotein, umfassend die Nukleinsäuresequenz kodierend für ein zweites TEV-Proteasefragment und eine Klonierungsstelle, die sich zur Einklonierung des Beute-Proteins im Leseraster des zweiten TEV-Proteasefragments eignet, wobei durch Interaktion des ersten und zweiten Fusionsproteins eine funktionale TEV-Protease rekonstituiert wird.

Der Kit kann gegebenenfalls Expressionsvektoren umfassen, die mindestens eines der folgenden Konstrukte umfassen:
3a) Nukleinsäurekonstrukt kodierend für eine funktionale Rekombinase oder für einen funktionalen Transkriptionsfaktor, der von einer Domäne zur Verankerung an einer zytoplasmatischen Struktur durch eine Erkennungs- und Schnittstelle für eine Protease proteolytisch abspaltbar ist, wobei diese Komponenten entweder als weitere Anteile des ersten oder zweiten Fusionsproteins oder als eigenständiges drittes Fusionsprotein eingesetzt werden können;
4a) ein Nukleinsäurekonstrukt kodierend für eine Rekombinase, die unter der Kontrolle des funktionalen Transkriptionsfaktors aus Nr. 3a. steht;
5a) ein Nukleinsäurekonstrukt, das eine von Erkennungsstellen für Rekombinasen flankierte Stop-Kassette mit der nachgeschalteten Nukleotidsequenz eines Reporter-Gens unter der Kontrolle eines konstitutiven Promoters umfaßt;
6a) ein Nukleinssäurekonstrukt kodierend für ein proteolytisch aktivierbares oder proteolytisch inaktivierbares Reporter-Protein unter der Kontrolle eines Promoters;
7a) ein Nukleinsäurekonstrukt kodierend für eine proteolytisch aktivierbare Protease unter der Kontrolle eines Promoters.

Der Kit kann auch Expressionsvektoren enthalten, die anstelle der oben genannten Nukleinsäurekonstrukte 1a) und 2a) auch modifizierte Varianten dieser beiden Konstrukte umfassen, nämlich ein erstes Nukleinsäurekonstrukt, welches eine funktionale Protease und eine Klonierungsstelle zur Einklonierung des Köder-Proteins im Leseraster der TEV-Protease umfaßt, und ein zweites Nukleinsäurekonstrukt, welches ein funktionales proteolytisch abspaltbares Protein aus der Gruppe der Rekombinasen oder Transkriptionsfaktoren und eine Klonierungsstelle zur Einklonierung des Beute-Proteins im Leseraster des abspaltbaren Proteins umfaßt.

Vorzugsweise werden die Expressionsvektoren derart im Kit bereitgestellt, daß in der Klonierungsstelle des zweiten Nukleinsäurekonstruktes bereits die Einzelkomponenten einer cDNA-Bibliothek einkloniert sind.

Alle erforderlichen Protein-Komponenten der erfindungsgemäßen Verfahren können hierbei sowohl durch transiente als auch durch stabile Transfektion oder Infektion in die Test-Zellen eingebracht werden. Das Einbringen in die Test-Zellen kann durch entsprechende Expressionsvektoren sowohl durch sämtliche dem Fachmann bekannten Techniken der Transformation und Transfektion erfolgen, kann jedoch auch alternativ durch die Infektion mit Retroviren oder anderen viral-basierten Verfahren durchgeführt werden.

Weiterhin können TEV-Proteasen zur Protein-Interaktions-abhängigen Erzeugung eines permanenten und verstärkten Detektionssignals in der Zelle verwendet werden

Die erfindungsgemäßen Verfahren zur Analyse der Assoziation von Interaktionspartnem oder von Komponenten eines Proteinkomplexes sind insbesondere dazu geeignet,
A) unbekannte Proteine aus cDNA- oder ORF- (Open Reading Frame)-Bibliotheken zu isolieren, die stimulations-abhängig mit einem bekannten Partner interagieren oder neuartige Interaktionen bekannter Proteine zu charakterisieren und Interaktionsdomänen und Regulationsmechanismen zu identifizieren.
B) biologische Mechanismen aufzuklären, die das Ausbilden von Proteinkomplexen und deren dynamische Zusammensetzung steuern.
C) Substanzen zu isolieren, die direkt oder indirekt mit spezifischen Proteininteraktionen interferieren, d.h. die Protein-Interaktionen inhibieren oder fordern.
D) stimulus-abhängige Protein-Protein Interaktionen für eine Vielzahl verschiedener Signaltransduktionswege zu charakterisieren. Diese können dann als Nachweisverfahren für die Aktivierung von den entsprechenden zellulären Signalmechanismen oder zur Analyse des physiologischen Zustandes von Zellen z.B. nach der Zugabe von Wirkstoffen oder nach Änderung der Kultivierungsbedingungen genutzt werden.

Für die Identifizierung und Charakterisierung neuartiger Protein-Interaktionen bieten sich folgende Strategien und Techniken an:
1.) Zelllinien, die entsprechende Komponenten des Reportersystems stabil exprimieren, können mit einer komplexen Mischung von Retroviren infiziert werden, wobei die Retroviren so konstruiert sind, daß nach Integration ein bekanntes Bait-Fusionsprotein und ein unbekanntes Prey-Fusionsprotein in einer Zelle koexprimiert werden können. Die Prey-Fusionsproteine können durch molekulare Klonierung Gewebe- oder zelltyp-spezifischer komplexer cDNAs hergestellt werden. Die Koexpression der Bait- und Prey-Fusionsproteine kann durch bicistronische RNAs durch Verwendung von *internal ribosomal entry sites,* den sogennanten IRES-Elementen (wie z.B. des EMC-Virus), erreicht werden (Vagner, Galy et al. 2001) (Pestova, Kolupaeva et al. 2001). Die Verwendung bidirektionaler Promoter ermöglicht ebenfalls die simultane Koexpression zweier Proteine (Baron, Freundlieb et al. 1995). Die Identifizierung unbekannter Interaktionspartner kann durch Amplifikation und Sequenzierung der exprimierten cDNAs aus wachstums-selektierten oder durch *fluorescent-activated-cell-sorting* (FACS) isolierten Zellen bzw. Zellklonen erfolgen.
2.) Zelllinien, die entsprechende Komponenten des Reportersystems stabil exprimieren, können mit einer komplexen Mischung von Retroviren infiziert werden, wobei die Retroviren so konstruiert sind, daß nach Integration jeweils ein unbekanntes Bait-Prey Paar in einer Zelle koexprimiert wird. Die Bait- und Prey-Fusionsproteine können durch molekulare Klonierung und Kombination definiertkomplexer cDNA-Pools hergestellt werden. Die Koexpression und Identifizierung der unbekannten Partner kann wie unter 1.) beschrieben erfolgen.
3.) Zelllinien, die entsprechende Komponenten des Reportersystems stabil exprimieren, können mit einer komplexen Mischung von Retroviren infiziert werden, wobei die Retroviren so konstruiert sind, daß nach Integration jeweils ein unbekanntes Prey-Fusionsprotein in einer Zelle exprimiert wird. Nach transienter Transfektion oder Infektion einer entsprechend großen Zahl von Zellen mit Expressions-Plasmiden, die für ein oder mehrere Bait-Fusionsproteine kodieren, können neuartige Interaktionspartner, wie unter 1.) beschrieben, isoliert werden.
4.) Die unter 3.) beschriebene Strategie kann auch in Zellen durchgeführt werden, die keine oder nur eine Komponente des entsprechenden Reportersystems exprimiert. Bei der transienten Transfektion oder Infektion des Bait-Expressionsplasmids müßen die fehlenden Komponenten mit in die Zelle eingebracht werden.
5.) Verschiedene Zelltypen, die Komponenten des Reportersystems stabil exprimieren, aber nicht notwendigerweise müssen, können in Hochdurchsatz-Transfektionen oder Transformationen von komplexen Bibliotheken charakterisierter Prey-Expressionsplasmide zusammen mit einem oder mehreren bekannten Bait-Expressionsplasmiden analysiert werden. Bei nicht modifizierten Zellen müßen u.U. Komponenten des bevorzugten Reportersystems kotransfiziert werden. Komplexe Plasmidbibliotheken, mit einer stetig wachsenden Anzahl definierter cDNAs mit einem offenen Leseraster (*open reading frame* = ORF) werden von mehreren Anbietern angeboten (Brizuela, Braun et al. 2001). Das Format dieser Bibliotheken ist so gestaltet, daß durch einfache Rekombination die gewünschten N- oder C-terminalen Fusionsproteine in entsprechenden Plasmidvektoren exprimiert werden können (Simpson, Wellenreuther et al. 2000). Für den Nachweis ist es notwendig, daß bei einer gegebenen Transfektionseffizienz eine ausreichend große Zahl von Zellen pro Ansatz transfiziert wurde. Der Nachweis kann bei der Verwendung von Fluoreszenz-Reportern durch automatisierte Bildanalyse erfolgen oder bei Verwendung enzymatischer oder bioluminiszenter Reporter durch etablierte Verfahren nach dem Stand der Technik erfolgen.
6.) Wie unter 5.) beschrieben, können Zellen zur Analyse von Protein-Interaktionen mit einer Kombination entsprechender Expressionsplasmide transfiziert werden, wobei die Transfektion durch das Aufbringen der Plasmid-DNA oder der Plasmid-DNA-Mischung auf eine entsprechende Oberfläche und Behandlung mit bestimmten Transfektionsagentien erreicht wird. Dieses Verfahren wird als 'reverse Transfektion' bezeichnet und ermöglicht eine simultane Hochdurchsatz-Analyse entsprechender ORF-Expressionsbibliotheken (Ziauddin and Sabatini 2001).
7.) Durch die Verwendung der hier dargestellten Nachweisverfahren, die transiente Effekte in ein dauerhaftes Signal umwandeln können, ist es weiterhin möglich, gängige Methoden des Einbringens von Peptiden- oder Proteinfusionen in Zellen zu nutzen, obwohl die Stabilität von exogenen Proteinen bzw. Peptiden in der Zelle sehr kurz sein kann. Gängige Verfahren zum Einbringen von Peptiden oder Fusionsproteinen sind von Prochiantz beschrieben (Prochiantz 2000). Die entsprechenden zellgängigen Fusionsproteine können vorher auf verschiedene Weise modifiziert worden sein, z.B. durch Kopplung niedermolekularer Wirkstoffe, durch Kopplung bestimmter Lipide oder anderer Substanzklassen.

Die erfindungsgemäßen Verfahren werden durch die Zeichnungen näher erläutert:

Zur Verdeutlichung der molekularen Schaltermechanismen sind in den schematischen Zeichnungen inaktive Elemente grau dargestellt, aktivierte Elemente sind schwarz unterlegt.

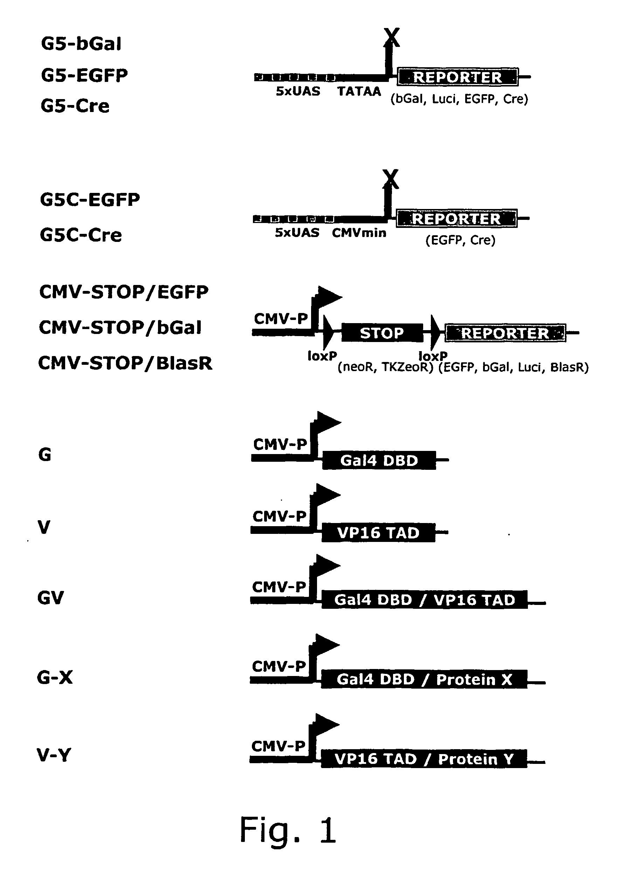

Es zeigt
Fig. 1 zeigt die schematische Darstellung der verwendeten Plasmid-Konstrukte für das Cre-Rekombinase abhängige Zwei-Hybrid System in Säugerzellen, wobei im einzelnen
   - die als G5-Reporter bezeichneten Konstrukte G5-bGal, G5-EGFP und G5-Cre die beta-Galaktosidase, das Enhanced Green Fluorescent Protein bzw. die Cre-Rekombinase unter der Kontrolle eines Minimal-Promoters, der E1B-TATA Box, und fünf aufeinander folgenden Gal4-abhängigen Enhancer-Elementen aus der Hefe (Upstream Activating Sequence, UAS) exprimieren.
   - die als G5C-Reporter bezeichneten Konstrukte G5C-EGFP und G5C-Cre das Enhanced Green Fluorescent Protein bzw. die Cre-Rekombinase unter der Kontrolle des humanen CMV-Minimal-Promoters (CMVmin) und fünf aufeinander folgenden Gal4-abhängigen Enhancer-Elementen aus der Hefe (Upstream Activating Sequence, UAS) exprimieren.
   - die als CMV-STOP/Reporter bezeichneten Konstrukte CMV-STOP/EGFP, CMV-STOP/bGal und CMV-STOP/BlasR das Enhanced Green Fluorescent Protein, die beta-Galaktosidase, bzw. das gegen BlasticidinS-Resistenz vermittelnde Enzym unter der Kontrolle eines humanen CMV-Promoters exprimieren können, wenn die durch loxP-Sequenzelemente flankierte STOP-Kassette nach Cre-Rekombinase Aktivität entfernt wurde.
   - die Komponenten des verwendeten Transkriptionsfaktors für das Zwei-Hybrid System in Säugerzellen dargestellt sind.
   - Mit G bezeichnet ist das Konstrukt zur Expression der DNA-bindenden Domäne des Gal4 Transkriptionsfaktors aus Hefe (Gal4 DBD) unter der Kontrolle des humanen CMV-Promoters.
   - Mit V bezeichnet ist das Konstrukt zur Expression der Transkriptions Aktivierungsdomäne des Herpes Simplex Virus-Protein VP16 (VP16 TAD) unter der Kontrolle des humanen CMV-Promoters.
   - Mit GV bezeichnet ist das Konstrukt zur Expression der Fusion der DNA-bindenden Domäne des Gal4 Transkriptionsfaktors aus Hefe (Gal4 DBD) und der Transkriptions-Aktivierungsdomäne des Herpes Simplex Virus-Protein VP16 (VP16 TAD) unter der Kontrolle des humanen CMV-Promoters.
   - Mit GX bezeichnet ist das Konstrukt zur Expression der DNA-bindenden Domäne des Gal4 Transkriptionsfaktors aus Hefe (Gal4 DBD) fusioniert mit Protein X unter der Kontrolle des humanen CMV-Promoters.
   - Mit VY bezeichnet ist das Konstrukt zur Expression der Transkriptions Aktivierungsdomäne des Herpes Simplex Virus-Protein VP16 (VP16 TAD) fusioniert mit Protein Y unter der Kontrolle des humanen CMV-Promoters.

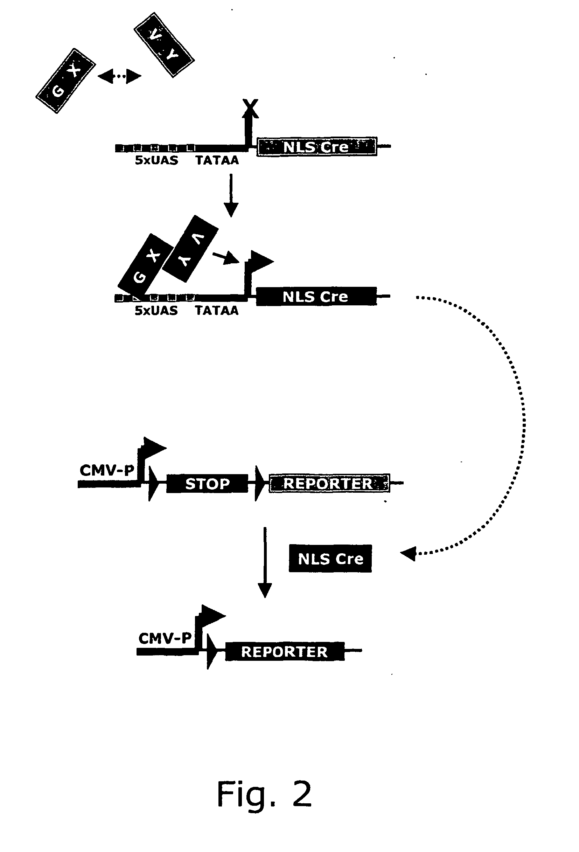

Fig. 2 zeigt ein Ablaufschema des Cre-Rekombinase abhängigen Zwei-Hybrid Systems zur Detektion konstitutiver Protein-Protein Interaktionen. Im Falle einer spezifischen Interaktion von GX und VY, die durch die Proteindomänen X und Y vermittelt wird, kommte es zur funktionellen Rekonstituierung eines Transkriptionsfaktors, der die Gal4-abhängige Expression der Cre-Rekombinase induziert. Die Aktivität des kern-lokalisierten Cre-Proteins führt zur Entfernung des transkriptionellen Inaktivierungselements (STOP) und zur dauerhaften Aktivierung nachgeschalteter Reportergene.

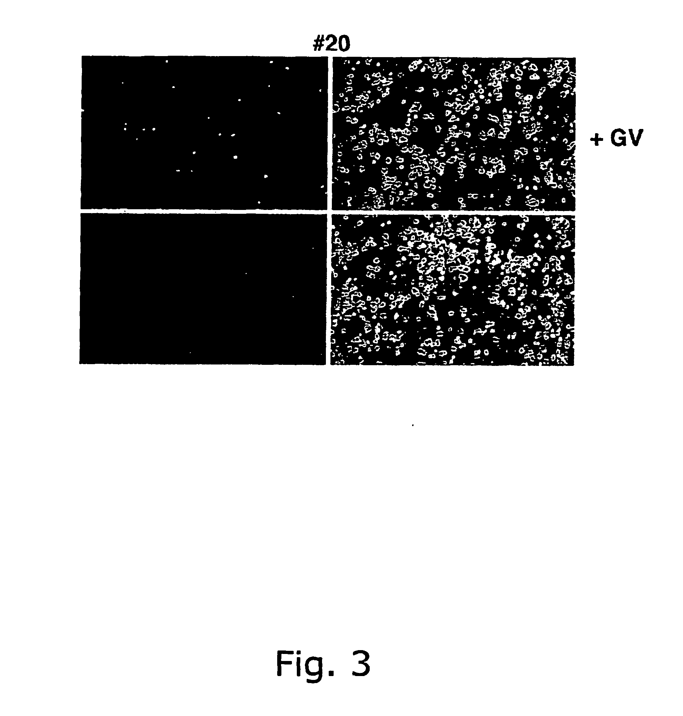

Fig. 3 zeigt GFP-Fluoreszenz- und Phasenkontrast-Aufnahmen der PC12 Zelllinie #20 nach Transfektion mit CMV-STOP/EGFP und dem Transkriptionsfaktor GV. Die Zelllinie #20 hat das Konstrukt G5C-Cre stabil integriert und zeigt nach Ko-Transfektion mit dem Cre-abhängigen Reporterkonstrukt CMV-STOP/EGFP keine EGFP Expression (unten links). Nach Ko-Transfektion von CMV-STOP/EGFP mit dem Transkriptionsfaktor GV kann eine GFP-Fluoreszenz nachgewiesen werden (oben links). Die Phasenkontrast-Aufnahmen (rechts) zeigen eine vergleichbare Anzahl von Zellen.

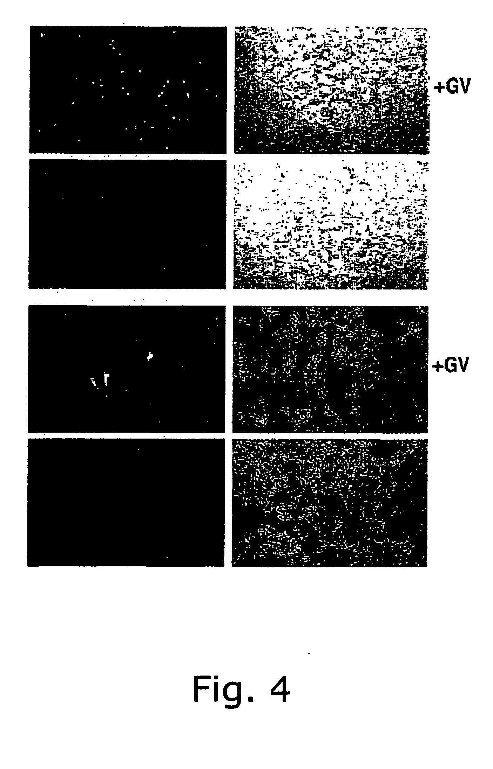

Fig. 4 zeigt GFP-Fluoreszenz- und Phasenkontrast-Aufnahmen der stabilen PC12 Zelllinie #20 nach Transfektion mit CMV-STOP/EGFP und GV drei Tage nach neuronaler Differenzierung durch NGF. Die Induktion neuronaler Differenzierung und die damit verbundene Verhinderung weiterer Zellteilungen nehmen keinen Einfluß auf die Aktivierung der Cre-Rekombinase vermittelten GFP-Fluoreszenz nach Ko-Transfektion von CMV-STOP/EGFP mit dem Transkriptionsfaktor GV in der PC12-Zelllinie #20 (oben links). Die Darstellung der GFP-positiven Zellen unter höherer Vergrößerung zeigt die neuronale Morphologie (unten links).

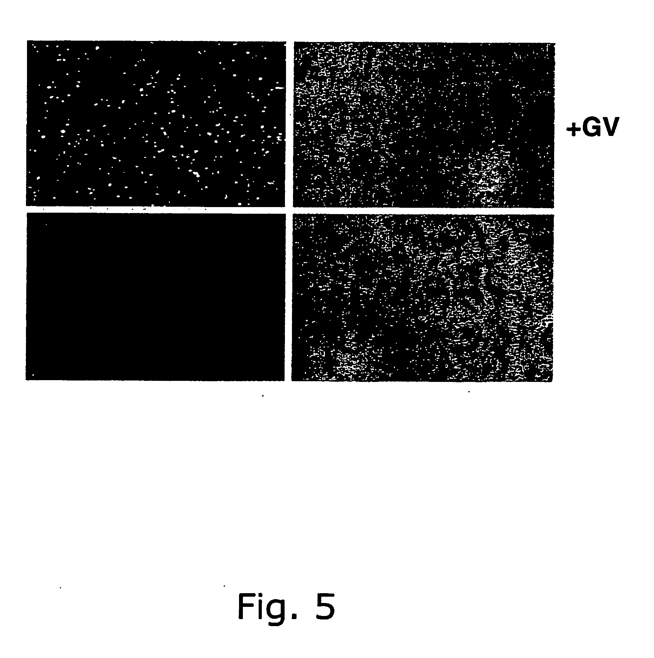

Fig. 5 zeigt GFP-Fluoreszenz- und Phasenkontrast-Aufnahmen der stabilen PC 12 Zelllinie #20.4 nach Transfektion mit GV. Die Zelllinie #20.4 hat die Konstrukte G5C-Cre, CMV-STOP/EGFP und CMV-TkZeoBlasR stabil integriert und zeigt nur nach Transfektion des Transkriptionsfaktors GV GFP-Fluoreszenz (oben links). Unter Kontrollbedingungen ist keine GFP-Fluoreszenz zu detektieren (unten links). Die Phasenkontrast-Aufnahmen zeigen eine vergleichbare Anzahl von Zellen (rechts).

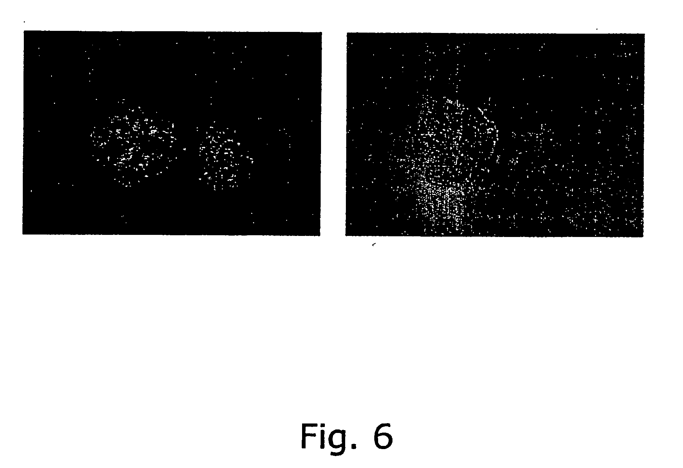

Fig. 6 zeigt die GFP-Fluoreszenz- und Phasenkontrast-Aufnahmen der stabilen PC12 Zelllinie #20.4 nach Transfektion mit GV und vierwöchiger BlasticidinS Selektion. Alle Zellen der BlasticidinS resistenten Zellklone zeigen eine vergleichbare GFP-Fluoreszenz, alle resistenten Zellklone sind GFP-positiv.

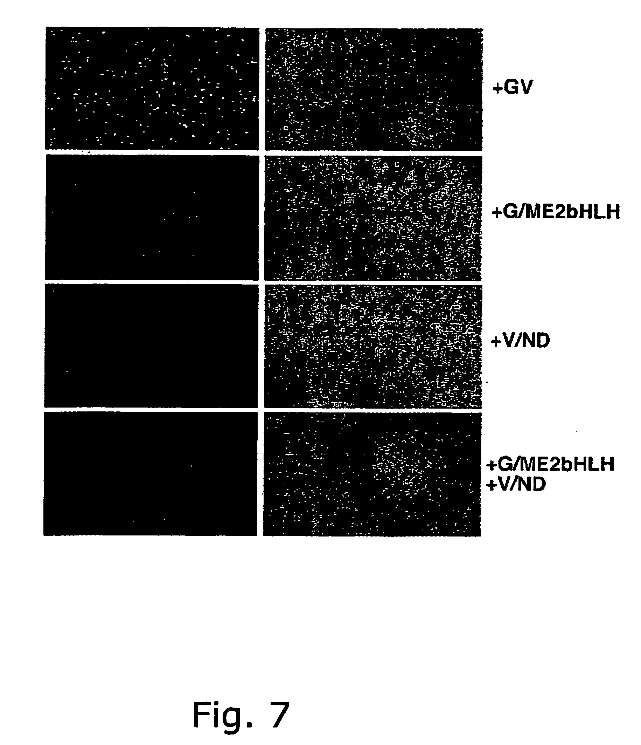

Fig. 7 zeigt die GFP-Fluoreszenz- und Phasenkontrast-Aufnahmen der stabilen PC 12 Zelllinie #20.4 zwei Tage nach Transfektion mit GV, G-ME2bHLH, V-ND und G-ME2bHLH mit V-ND. Die Zelllinie #20.4 zeigt nur nach Transfektion des Transkriptionsfaktors GV GFP-Fluoreszenz (oben links). Nach Ko-Transfektion der Zwei-Hybrid Interaktionspartner V-ND und G-ME2bHLH ist keine GFP-Fluoreszenz zu detektieren (unten links). Unter Kontrollbedingungen, Transfektion von V-ND oder G-ME2bHLH, ist ebenfalls keine GFP-Fluoreszenz nachzuweisen (Mitte links). Die Phasenkontrast-Aufnahmen zeigen eine vergleichbare Anzahl von Zellen (rechts).

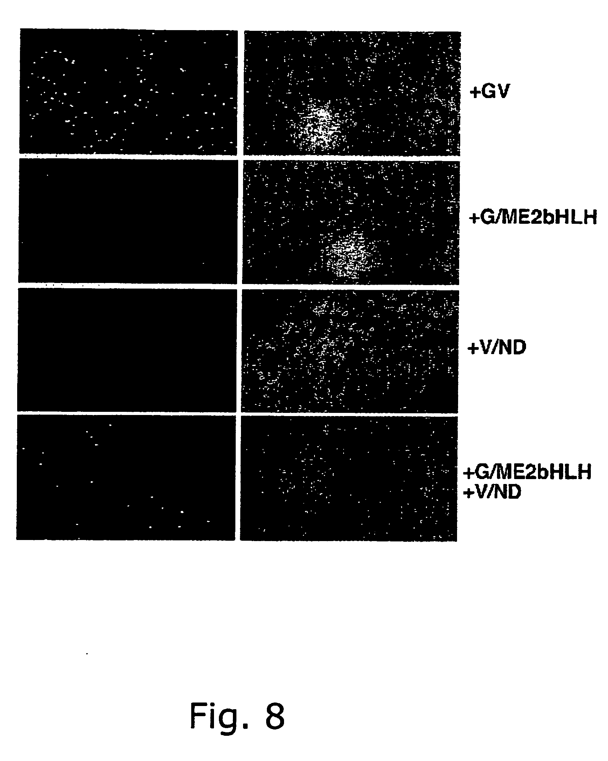

Fig. 8 zeigt die GFP-Fluoreszenz- und Phasenkontrast-Aufnahmen der stabilen PC12 Zelllinie #20.4 zwei Tage nach Transfektion mit GV, G-ME2bHLH, V-ND und G-ME2bHLH mit V-ND unter Zugabe von TSA. Die Zelllinie #20.4 zeigt nach Transfektion des Transkriptionsfaktors GV GFP-Fluoreszenz (oben links). Nach Ko-Transfektion der Zwei-Hybrid Interaktionspartner V-ND und G-ME2bHLH ist unter den TSA-Kultivierungsbedingungen ebenfalls GFP-Fluoreszenz zu detektieren (unten links). Unter Kontrollbedingungen, Transfektion von V-ND oder G-ME2bHLH, ist keine GFP-Fluoreszenz nachzuweisen (Mitte links). Die Phasenkontrast-Aufnahmen zeigen eine vergleichbare Anzahl von Zellen (rechts).

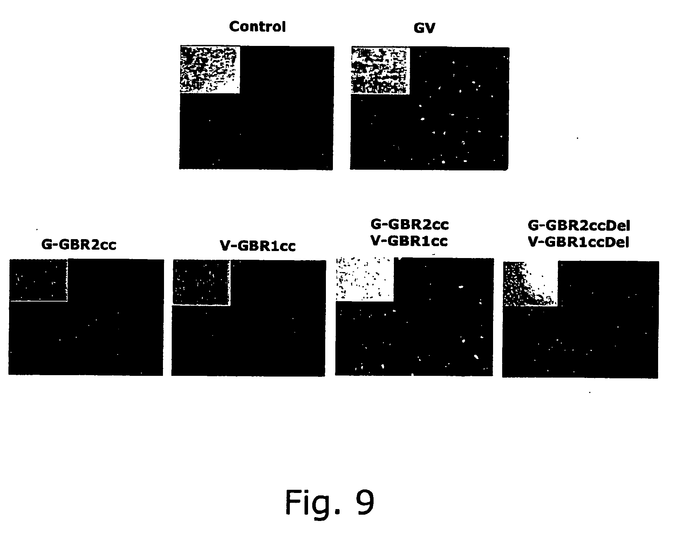

Fig. 9 zeigt die GFP-Fluoreszenz- und Phasenkontrast-Aufnahmen (kleines eingelassenes Bild) der stabilen PC 12 Zelllinie #20.4 nach Transfektion mit GV, G-GBR2cc, V-GBR1cc, G-GBR2cc mit V-GBR1cc und G-GBR2ccDel mit V-GBRIccDel. Die Zelllinie #20.4 zeigt nach Transfektion des Transkriptionsfaktors GV GFP-Fluoreszenz (oben rechts). Nach Ko-Transfektion der Zwei-Hybrid Interaktionspartner G-GBR2cc, V-GBR1cc ist ebenfalls GFP-Fluoreszenz zu detektieren (unten links). Unter Kontrollbedingungen, Transfektion eines Leervektors (Kontrolle, oben links) und der einzeln transfeziertzen Interaktionspartner (G-GBR2cc oder V-GBR1cc, unten links) ist keine GFP-Fluoreszenz nachzuweisen (Mitte links). Nach Ko-Transfektion von Plasmiden die für coiled-coiled Deletionsmutanten der intrazellulären Domänen von GBR1 und GBR2 kodieren ist ebenfalls keine GFP-Fluoreszenz nachzuweisen (G-GBR2ccDel und V-GBR1ccDel, unten halbrechts). Die Phasenkontrast-Aufnahmen zeigen eine vergleichbare Anzahl von Zellen (kleine eingepasste Abbildung).

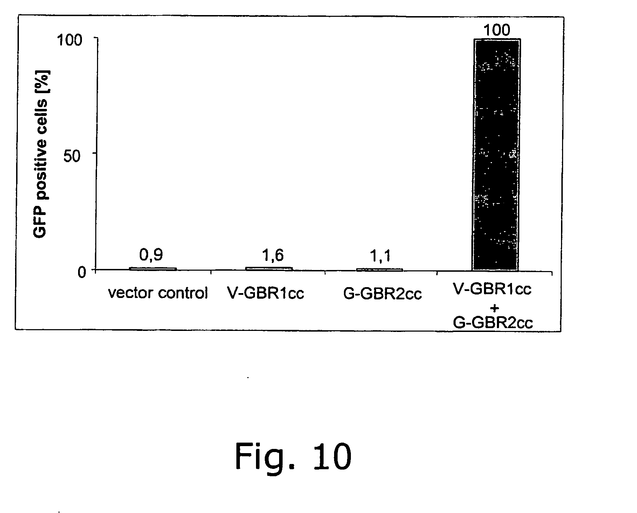

Fig. 10 zeigt die quantitative Auswertung der FACS-Analyse der stabilen PC12 Zelllinie #20.4 zwei Tage nach Transfektion mit GV, V-GBR1cc, G-GBR2cc und V-GBR1cc zusammen mit G-GBR2cc, dargestellt als die relative Zahl der GFP-positiven Zellen. Zwei Tage nach Ko-Transfektion der Interaktionspartner G-GBR2cc und V-GBR1cc können im Vergleich zu den Kontrollen, Leervektor (vector control) und der Einzel-Transfektionen von G-GBR2cc und V-GBR1cc, etwa 100 mal mehr GFP-positive Zellen detektiert werden.

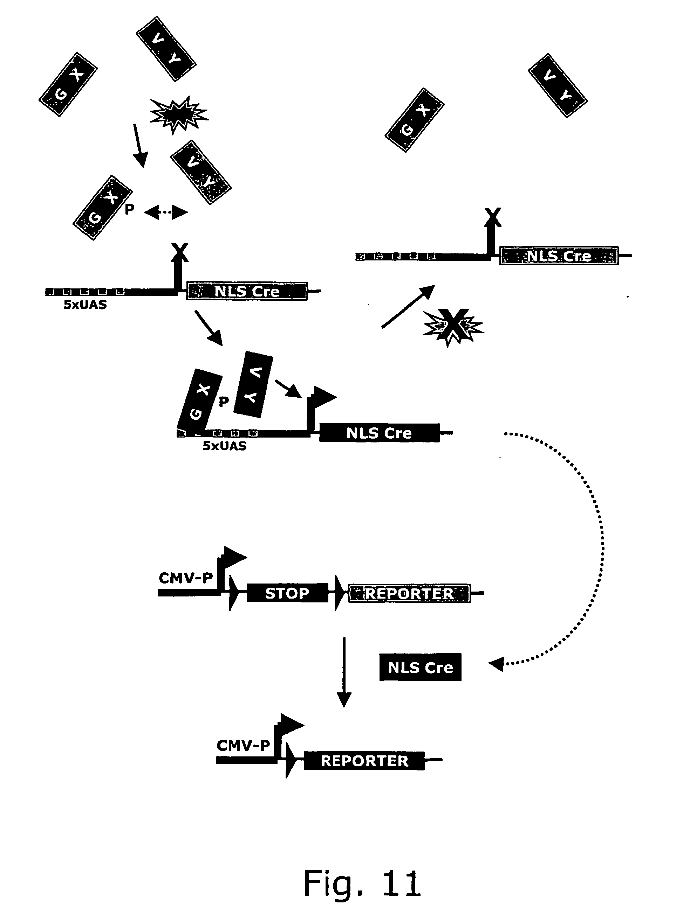

Fig. 11 zeigt ein Ablaufschema des Cre-Rekombinase abhängigen Zwei-Hybrid Systems zur Detektion induzierter und transienter Protein-Protein Interaktionen. Im Falle einer stimulus-abhängigen Interaktion von GX und VY, die durch die Proteindomänen X und Y vermittelt wird, kommte es zur funktionellen Rekonstituierung eines Transkriptionsfaktors, der die Gal4-abhängige Expression der Cre-Rekombinase induziert. Der Stimulus ist durch das Explosionssymbol dargestellt. Die stimulations-abhängige Modifikation des Proteinabschnittes X ist durch ein P gekennzeichnet. Nach Aufhebung der zellulären Veränderung (dargestellt durch das X über dem Explosionssymbol) kommt es zur Aufhebung der Modifikation und zu einer Aufhebung der Protein-Interaktion. Die Aktivität des kem-lokalisierten Cre Proteins führt zur Entfernung des transkriptionellen Inaktivierungselements (STOP) und zur dauerhaften Aktivierung nachgeschalteter Reportergene. Die Aktivität des Cre Proteins kann vorübergehend sein und muss nur einmal einen bestimmten Schwellenwert erreichen.

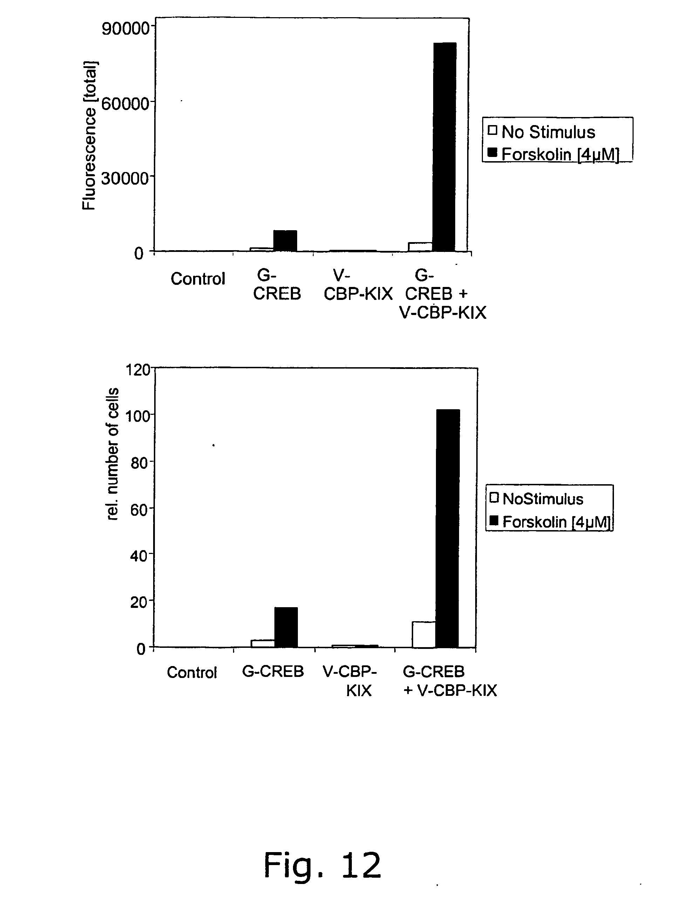

Fig. 12 zeigt die Resultate der FACS-Analyse drei Tage nach Transfektion der stabilen PC12 Zelllinie #20.4 mit einer Vektorkontrolle, G-CREB, V-CBP/KIX und G-CREB mit V-CBP/KIX, mit und ohne Forskolin-Stimulation. Dargestellt ist die kummulierte GFP-Fluoreszenz (oben) und die relative Zahl der GFP-positiven Zellen (unten) ohne Forskolin-Stimulation (umrandete Säulen) und nach vorübergehender Forskolin-Stimulation (schwarze Säulen). Nach Ko-Transfektion der Interaktionspartner, G-CREB mit V-CBP/KIX, kommt es nach Forskolin-Stimulation zu einer etwa 12fach erhöhten Zahl der detektierten GFP-positiven Zellen, die Zunahme der gesamten Fluoreszenz der GFP-positiven Zellen liegt zwanzigfach über der unstimulierten Kontrolle. Die Transfektion mit V-CBP-KIX zeigt nahezu keinen Hintergrund, während die Transfektion mit dem G-CREB Konstrukt sowohl in dem unstimulierten als auch in dem Forskolin-stimulierten Ansatz GFP Hintergrund aufweist. Der ebenfalls starke Anstieg der GFP-Fluoreszenz nach Forskolin Stimulation reflektiert vermutlich die Interaktion mit endogen vorhandenen CBP-ähnlichen transkriptionellen Ko-Faktoren.

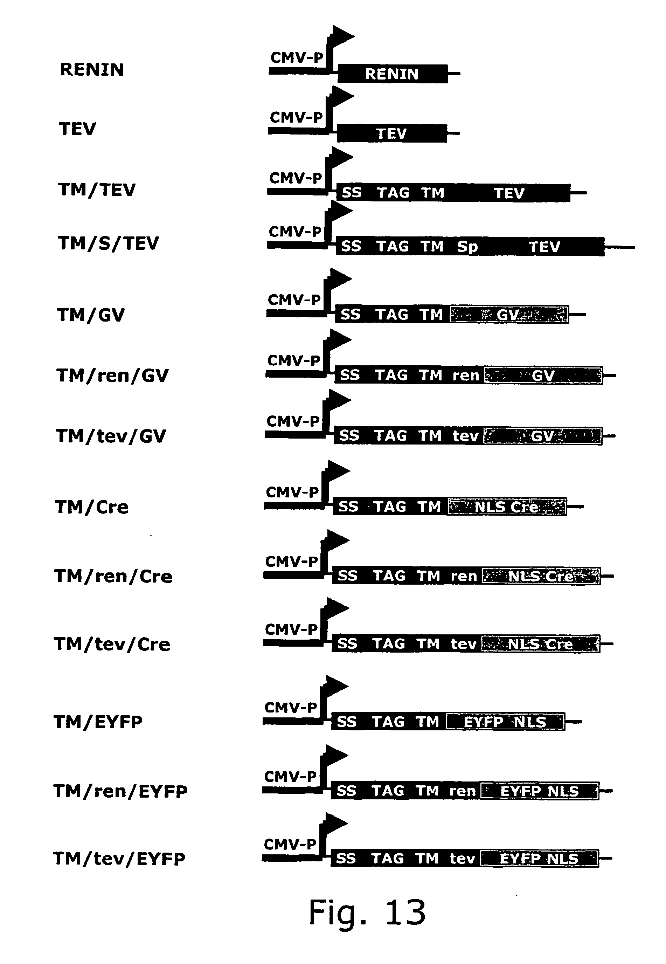

Fig. 13 zeigt die schematische Darstellung der verwendeten Plasmid-Konstrukte für das Cre-Rekombinase gekoppelte Zwei-Schalter System in Säugerzellen. Durch SS wird die Signalsequenz bezeichnet, TAG steht für die extrazelluläre Domäne die spezifische Epitope einschließt, TM steht für die PDGF-alpha-Rezeptor Transmembrandomäne. Im einzelnen sind die Konstrukte näher beschrieben:
   - das als Renin bezeichnete Konstrukt exprimiert die humane Renin-Protease unter der Kontrolle des humanen CMV-Promoters.
   - das als TEV bezeichnete Konstrukt exprimiert die NIa-Protease des Tobacco Etch Virus (TEV-Protease) unter der Kontrolle des humanen CMV-Promoters.
   - das als TM/TEV bezeichnete Konstrukt exprimiert eine Transmembran-gebundene Form der TEV-Protease des Tobacco Etch Virus unter der Kontrolle des humanen CMV-Promoters.
   - das als TM/S/TEV bezeichnete Konstrukt exprimiert eine Transmembran-gebundene Form der TEV-Protease des Tobacco Etch Virus unter der Kontrolle des humanen CMV-Promoters, wobei die TEV-Protease durch einen Peptidabschnitt (S steht für Spacer) von der Transmembrandomäne getrennt ist.
   - das als TM/GV bezeichnete Konstrukt exprimiert eine Transmembran-gebundene inaktive Form des Transkriptionsaktivators GV unter der Kontrolle des humnanen CMV-Promoters.
   - das als TM/ren/GV bezeichnete Konstrukt exprimiert eine Transmembran-gebundene inaktive Form des Transkriptionsaktivators GV unter der Kontrolle des humanen CMV-Promoters, wobei der Transkriptionsaktivators GV durch einen Peptidabschnitt ren von der Transmembrandomäne getrennt ist. ren stellt eine für die humane Renin-Protease spezifische Erkennungs- und Spaltsequenz dar.
   - das als TM/tev/GV bezeichnete Konstrukt exprimiert eine Transmembran-gebundene inaktive Form des Transkriptionsaktivators GV unter der Kontrolle des humanen CMV-Promoters, wobei der Transkriptionsaktivators GV durch einen Peptidabschnitt tev von der Transmembrandomäne getrennt ist. tev stellt eine für die TEV-Protease spezifische Erkennungs- und Spaltsequenz dar.
   - das als TM/Cre bezeichnete Konstrukt exprimiert eine Transmembran-gebundene inaktive Form der Cre-Rekombinase unter der Kontrolle des humanen CMV-Promoters.
   - das als TM/ren/Cre bezeichnete Konstrukt exprimiert eine Transmembran-gebundene inaktive Form der Cre-Rekombinase unter der Kontrolle des humanen CMV-Promoters, wobei die Cre-Rekombinase durch einen Peptidabschnitt ren von der Transmembrandomäne getrennt ist. ren stellt eine für die humane Renin-Protease spezifische Erkennungs- und Spaltsequenz dar.
   - das als TM/tev/Cre bezeichnete Konstrukt exprimiert eine Transmembran-gebundene inaktive Form der Cre-Rekombinase unter der Kontrolle des humanen CMV-Promoters, wobei die Cre-Rekombinase durch einen Peptidabschnitt tev von der Transmembrandomäne getrennt ist. tev stellt eine Für die TEV-Protease spezifische Erkennungs- und Spaltsequenz dar.
   - das als TM/EYFP bezeichnete Konstrukt exprimiert eine Transmembran-gebundene Form des Enhanced Yellow Fluorescent Protein (EYFP) mit C-terminalen Kern-Lokalisations-Signalen (NLS) unter der Kontrolle des humanen CMV-Promoters.
   - das als TM/ren/EYFP bezeichnete Konstrukt exprimiert eine Transmembran-gebundene Form des Enhanced Yellow Fluorescent Protein (EYFP) mit C-terminalen Kern-Lokalisations-Signalen (NLS) unter der Kontrolle des humanen CMV-Promoters, wobei das EYFP-NLS Protein durch einen Peptidabschnitt ren von der Transmembrandomäne getrennt ist. ren stellt eine für die humane Renin-Protease spezifische Erkennungs- und Spaltsequenz dar.
   - das als TM/tev/EYFP bezeichnete Konstrukt exprimiert eine Transmembran-gebundene Form des Enhanced Yellow Fluorescent Protein (EYFP) mit C-terminalen Kern-Lokalisations-Signalen (NLS) unter der Kontrolle des humanen CMV-Promoters, wobei das EYFP-NLS Protein durch einen Peptidabschnitt tev von der Transmembrandomäne getrennt ist. ren stellt eine für die humane Renin-Protease spezifische Erkennungs- und Spaltsequenz dar.

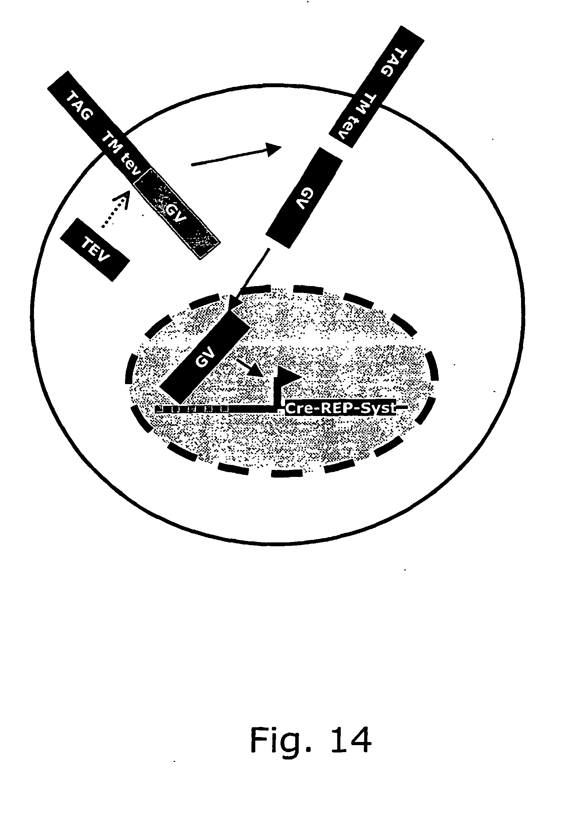

Fig. 14 zeigt ein Ablaufschema des TEV Protease-abhängigen molekularen Schalters zur Aktivierung eines membran-gebundenen Transkriptionsfaktors und der nachfolgenden Aktivierung eines Reporters oder Reportersystems. Bei Ko-expression der TEV Protease mit einem transmenmbran-gebundenen Transkriptionsfaktor (GV), der N-terminal eine TEV-Protease spezifische Erkennungs- und Schnittstelle (tev) hat, kommt es zur Spaltung und anschließenden Translokalisation des Transkriptionsfaktors GV in den Zellkern. GV abhängige Reportergene oder Reportersysteme werden aktiviert.

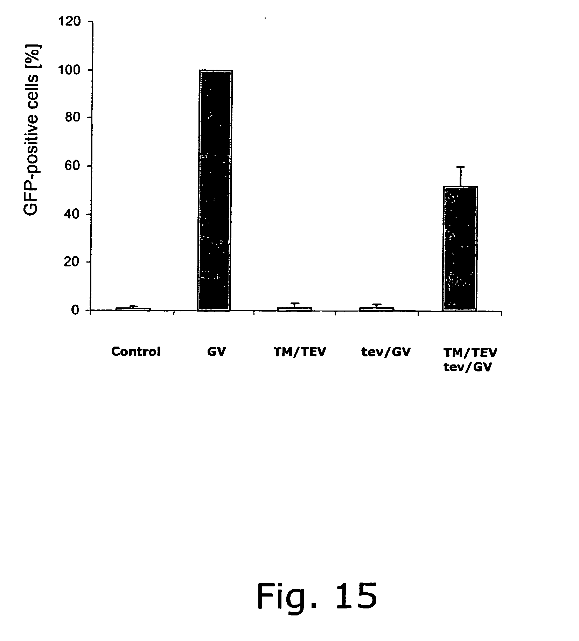

Fig. 15 zeigt die quantitative Auswertung der FACS-Analyse zwei Tage nach Transfektion der stabilen PC12 Zelllinie #20.4 mit einem Kontrollvektor (Control), GV, TM/Tev, tevGV und TM/Tev zusammen mit tev/GV. Gezeigt ist die relative Anzahl GFP-positiver Zellen im Vergleich zur Transfektion mit dem löslichen Transkriptionsfaktor GV. Die Zahl der detektierten Zellen nach Einzel-Transfektion mit dem Leervektor, mit der transmembran-gebundenen TEV oder dem transmembran-gebundenen GV liegt unter 1%. Nach Ko-Transfektion der transmembran-gebundenen TEV mit dem transmembran-gebundenen Transkriptionsfaktor GV können im Vergleich zur Transfektion unter Vergleichsbedingungen mit dem löslichen GV durchschnittlich die Hälfte an GFP-positiven Zellen detektiert werden.

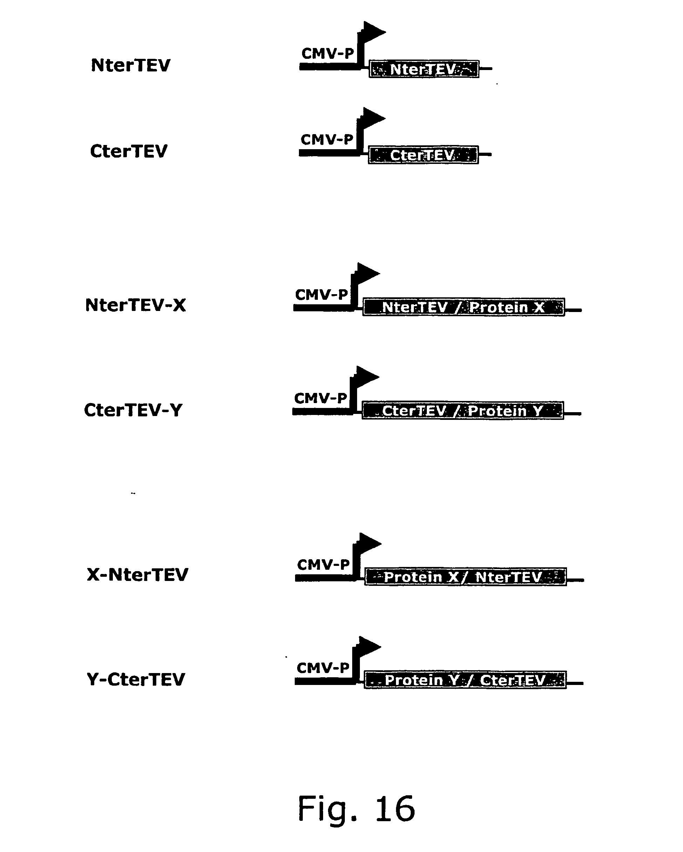

Fig. 16 zeigt die schematische Darstellung der verwendeten Plasmid-Konstrukte für die Protein-Interaktions gekoppelte Trans-Komplementation von Fragmenten der TEV-Protease in Säugerzellen, wobei im einzelnen
   - das als NterTEV bezeichnete Konstrukt ein N-terminales Fragment der TEV-Protease des Tobacco Etch Virus unter der Kontrolle des humanen CMV-Promoters exprimiert.
   - das als CterTEV bezeichnete Konstrukt ein N-terminales Fragment der TEV-Protease des Tobacco Etch Virus unter der Kontrolle des humanen CMV-Promoters exprimiert.
   - das als NterTEV-X bezeichnete Konstrukt ein N-terminales Fragment der TEV-Protease des Tobacco Etch Virus als Fusionsprotein mit dem N-terminus eines Protein X unter der Kontrolle des humanen CMV-Promoters exprimiert.
   - das als CterTEV-Y bezeichnete Konstrukt ein C-terminales Fragment der TEV-Protease des Tobacco Etch Virus als Fusionsprotein mit dem N-terminus eines Protein Y unter der Kontrolle des humanen CMV-Promoters exprimiert.
   - das als X-NterTEV bezeichnete Konstrukt ein N-terminales Fragment der TEV-Protease des Tobacco Etch Virus als Fusionsprotein mit dem C-terminus eines Protein X unter der Kontrolle des humanen CMV-Promoters exprimiert.
   - das als Y-CterTEV bezeichnete Konstrukt ein C-terminales Fragment der TEV-Protease des Tobacco Etch Virus als Fusionsprotein mit dem C-terminus eines Protein Y unter der Kontrolle des humanen CMV-Promoters exprimiert.

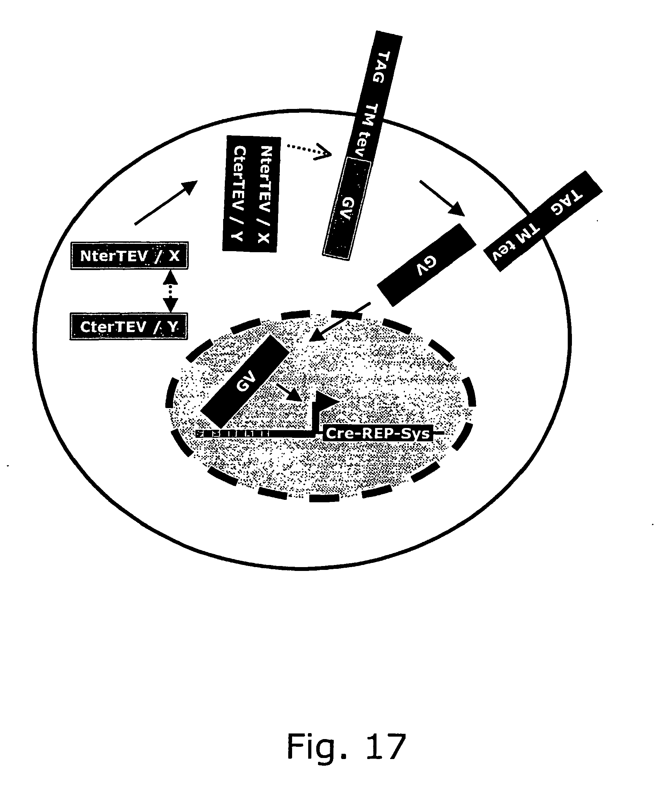

Fig. 17 zeigt ein Ablaufschema des Protease-abhängigen molekularen Schalters zur Aktivierung eines membran-gebundenen Transkriptionsfaktors nach Protein-Interaktions gekoppelter funktioneller Rekonstituierung von TEV-Protease Fragmenten. Nach Ko-Expression von Plasmiden, die zum einen für ein N-terminales TEV Fragment - Protein X-Fusionsprotein und für ein C-terminales TEV Fragment-Protein Y- Fusionsprotein kodieren, kommt es im Falle der spezifischen Interaktion der Proteine oder Proteinabschnitte X und Y, zur funktionellen Rekonstituierung der proteolytischen Aktivität. Daraufhin wird ein stabil exprimierter oder transient ko-transfizierter membran-gebundener und eine TEV-spezifischen Erkennungs- und Spaltsequenz (tev) enthaltender Transkriptionsfaktor (GV) von der Membran gelöst. Es kommt zu einer Translokalisation von GV in den Kern und zur Aktivierung eines Reportergens oder Reportersystems.

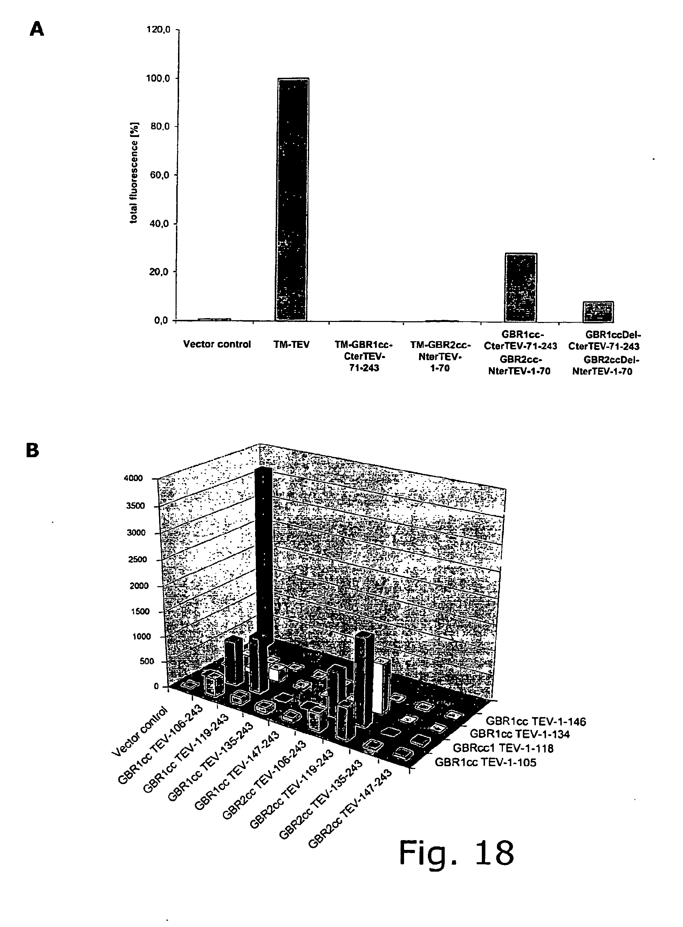

Fig. 18 Teil A zeigt die quantitative Auswertung der FACS-Analyse zwei Tage nach Transfektion der stabilen PC 12 Zelllinie #20.4 mit einem Expressionsvektor, der kodiert für den membran-gebundenen Transkriptionsfaktor GV mit einer N-terminalen TEV-spezifischen Erkennungs- und Spaltsequenz (TM-tev-GV). Gezeigt ist die kummulierte Fluoreszenz relativ zu der Aktivität der membrangebundenen TEV-Protease in %. Ko-transfiziert mit TMtevGV wurden Expressionsvektoren, die kodieren für:
   - Kontrolle: membran-gebundene TEV (TM-TEV),
   - eine membran-gebundene GBR1cc-Domäne fusioniert an ein C-terminales Fragment der TEV-Protease, von Aminosäure 71-243 (GBR1cc-CterTEV-71-243)
   - eine membran-gebundene GBR2cc-Domäne fusioniert an ein N-terminales Fragment der TEV-Protease, von Aminosäure 1-70 (GBR2cc-CterTEV-1-70) und
   - eine membran-gebundene GBR1cc-Domäne fusioniert an ein C-terminales Fragment der TEV-Protease, von Aminosäure 71-243 (GBR1cc-CterTEV-71-243) zusammen mit einer membran-gebundenem GBR2cc-Domäne fusioniert an ein N-terminales Fragment der TEV-Protease, von Aminosäure 1-70 (GBR2cc-CterTEV-1-70).

   Nach Ko-Transfektion der TEV-Fragmente als Fusionsproteine der Interaktionsdomänen GBR2cc und GBR1cc wird etwa 30 % der Aktiviät der membran ständigen TEV-Protease erreicht.
   In B sind Kombinationen weiterer TEV-Fragmente, fusioniert an die coiled-coil Interaktionsdomänen von GBR1 und GBR2, gezeigt. Die Konstrukte wurde in PC12-Zellen kotransfiziert mit dem membranverankerten TM-tev-GV, einem durch GV transkriptionell aktivierbaren, Cre-Rekombinase kodierenden, Plasmid, und einem Reporterplasmid kodierend für Firefly Luziferase unter Kontrolle eines starken Promotors. Die Transkription der Luziferase war allerdings durch eine zwischen Gen und Promotor liegende, von pLox flankierte, Stop-Kassette unterbrochen. Aktivierung des Systems erfolgte durch die spezifische Transkomplementation der TEV Protease, was am a Ende der Kaskade zur Produktion von Luziferase führte. im Balkendiagramm ist diese Aktivität in RLU (relative Lunineszenz Units) dargestellt und mit Signal der intakten Protease verglichen.

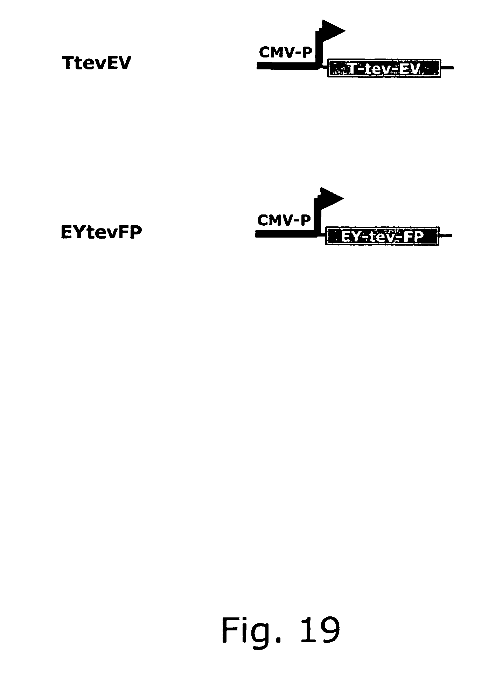

Fig. 19 zeigt die schematische Darstellung der verwendeten Plasmid-Konstrukte für das Protease gekoppelte Endlos-Schalter System, wobei im einzelnen
   - das als TtevEV bezeichnete Konstrukt eine inaktive TEV-Protease unter der Kontrolle des humanen CMV-Promoters darstellt. Durch Insertion einer Peptidsequenz, die mindestens eine TEV-spezifische Erkennungs- und Spaltsequenz enthält, ist diese modifizierte TEV-Protease inaktiv. Nach spezifischer proteolytischer Spaltung durch eine aktive TEV-Protease wird die modifizierte TEV-Protease TtevEV aktiviert.
   - das als EYtevFP bezeichnete Konstrukt ein inaktives, nicht-fluoreszierendes Enhanced Green Fluorescent Protein (EYFP) unter der Kontrolle des humanen CMV-Promoters darstellt. Durch Insertion einer Peptidsequenz, die mindestens eine TEV-spezifische Erkennungs- und Spaltsequenz enthält, ist dieses modifizierte EYFP inaktiv. Nach spezifischer proteolytischer Spaltung durch eine aktive TEV-Protease wird das modifizierte EYtevFP aktiviert.

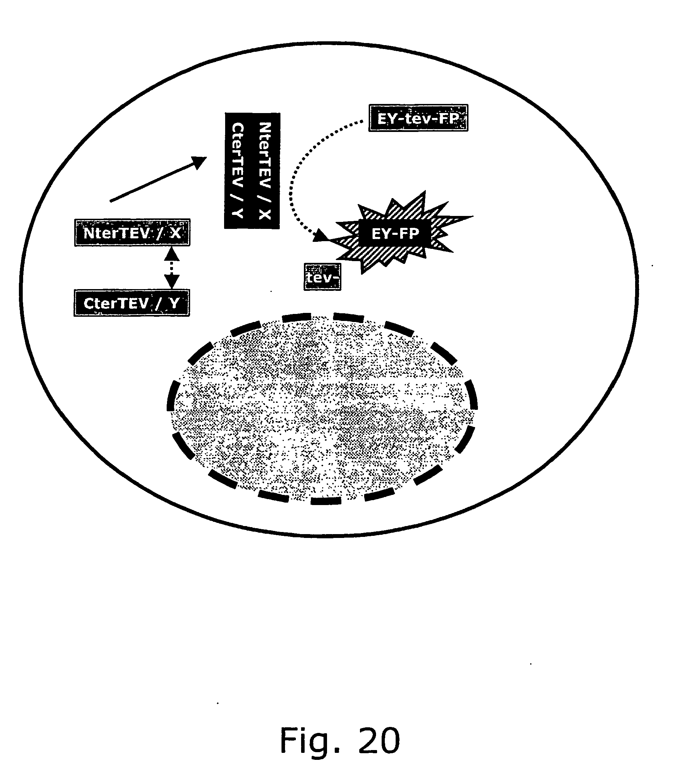

Fig. 20 zeigt ein Ablaufschema des Protease-abhängigen molekularen Schalters zur Aktivierung einer proteolytisch aktivierbaren nicht-fluoreszenten EYFP-Variante nach Protein-Interaktions gekoppelter funktioneller Rekonstituierung von TEV-Protease Fragmenten. Nach Ko-Expression von Plasmiden, die zum einen für ein N-terminales TEV Fragment - Protein X-Fusionsprotein und für ein C-terminales TEV Fragment-Protein Y- Fusionsprotein kodieren, kommt es im Falle der spezifischen Interaktion der Proteine oder Proteinabschnitte X und Y, zur funktionellen Rekonstituierung der proteolytischen Aktivität. Daraufhin wird ein stabil exprimiertes oder transient ko-transfiziertes inaktives, proteolytisch-aktivierbares Reporterprotein, wie z.B. ein entsprechend modifiziertes EYFP (EY-tev-FP) spezifisch proteolytisch gespalten und aktiviert.

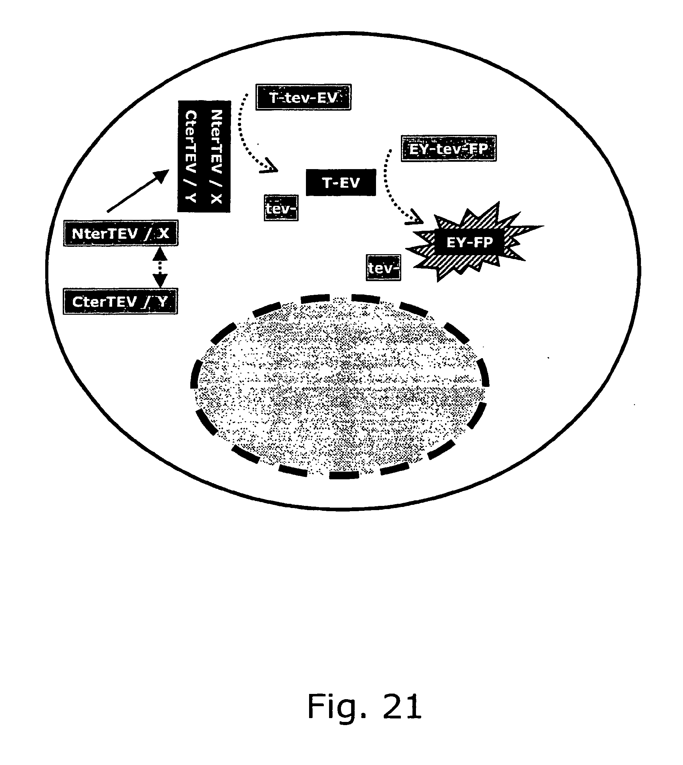

Fig. 21 zeigt ein Ablaufschema des Protease-abhängigen molekularen Endlos-Schalters zur Aktivierung einer proteolytisch aktivierbaren, inaktiven TEV-Protease und einer proteolytisch aktivierbaren, nicht-fluoreszenten GFP-Variante nach Protein-Interaktions gekoppelter funktioneller Rekonstituierung von TEV-Protease Fragmenten. Nach Ko-Expression von Plasmiden, die zum einen für ein N-terminales TEV Fragment - Protein X-Fusionsprotein und für ein C-terminales TEV Fragment-Protein Y- Fusionsprotein kodieren, kommt es im Falle der spezifischen Interaktion der Proteine oder Proteinabschnitte X und Y, zur funktionellen Rekonstituierung der proteolytischen Aktivität. Daraufhin wird eine stabil exprimiertee oder transient ko-transfiziertee inaktive, proteolytischaktivierbare TEV-Protease (TtevEV) spezifisch proteolytisch gespalten und dauerhaft aktiviert. Inder Folge wird ein stabil exprimiertes oder transient ko-transfiziertes inaktives, proteolytisch-aktivierbares Reporterprotein, wie z.B. ein entsprechend modifiziertes EYFP (EY-tev-FP) spezifisch proteolytisch gespalten und aktiviert. Die dauerhafte Aktivierung der konstitutiv exprimierten proteolytisch-aktivierbaren Funktionselemente führt zu einer molekularen EndlosSchleife.

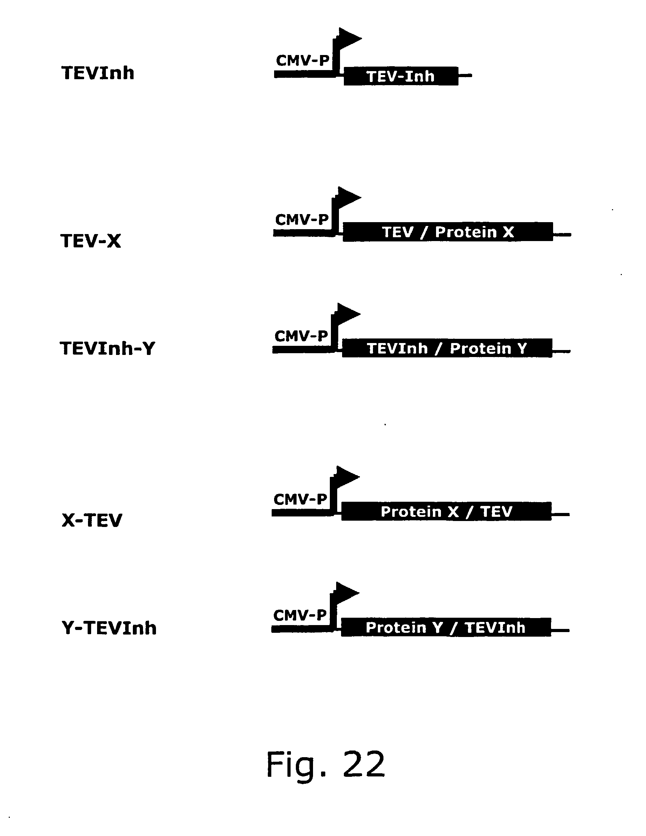

Fig. 22 zeigt die schematische Darstellung der verwendeten Plasmid-Konstrukte für das Protease gekoppelte Reverse-Schalter System, wobei im einzelnen
   - das als TEVInh bezeichnete Konstrukt einen Protein- oder Peptidinhibitor der TEV-Protease des Tobacco Etch Virus unter der Kontrolle des humanen CMV-Promoters exprimiert.
   - das als TEV-X bezeichnete Konstrukt ein Fusionsprotein der intakten TEV-Protease des Tobacco Etch Virus und ein Protein X unter der Kontrolle des humanen CMV-Promoters exprimiert. Das Protein X ist mit dem C-terminus der TEV-Protease fusioniert.
   - das als TEVInh-Y bezeichnete Konstrukt ein Fusionsprotein eines TEV-Inhibitors und ein Protein Y unter der Kontrolle des humanen CMV-Promoters exprimiert. Das Protein Y ist mit dem C-terminus des TEV-Inhibitors fusioniert.
   - das als X-TEV bezeichnete Konstrukt ein Fusionsprotein der intakten TEV-Protease des Tobacco Etch Virus und ein Protein X unter der Kontrolle des humanen CMV-Promoters exprimiert. Das Protein X ist mit dem N-terminus der TEV-Protease fusioniert.
   - das als Y-TEVInh bezeichnete Konstrukt ein Fusionsprotein eines TEV-Inhibitors und ein Protein Y unter der Kontrolle des humanen CMV-Promoters exprimiert. Das Protein Y ist mit dem N-terminus des TEV-Inhibitors fusioniert.

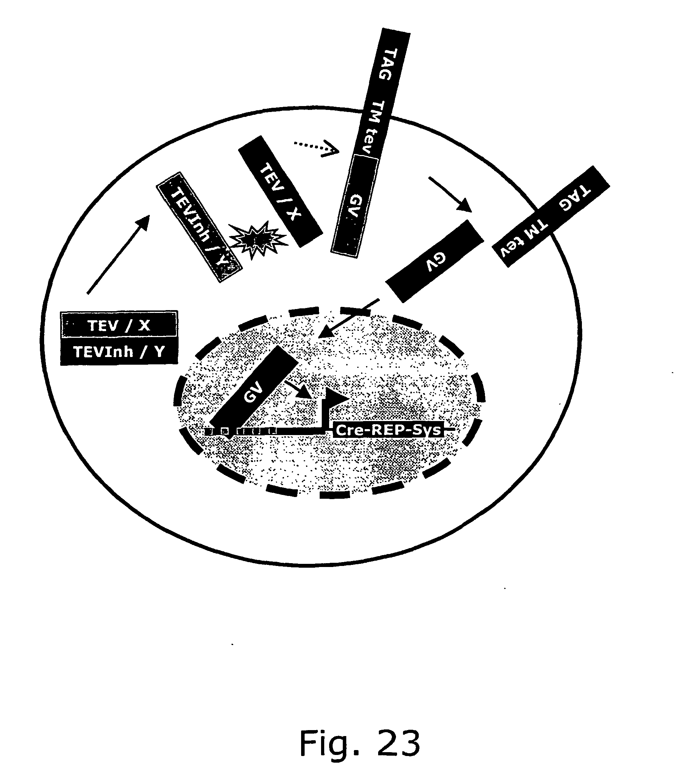

Fig. 23 zeigt ein Ablaufschema des reversen Schalter Systems nach induzierter Dissoziation der bekannten Interaktion von ProteinX fusioniert mit einem TEV-Inhibitor und ProteinY fusioniert mit der intakten TEV-Protease, gekoppelt an das Zwei-Schalter System. Durch die Protein X und Protein Y vermittelte Interaktion des TEV-Inhibitors mit der intakten TEV-Protease kommt es zu einer Inaktivierung der TEV-Protease. Nach induzierter Aufhebung der Interaktion, dargestellt durch das Explosionssymbol, kommt es zur Aktivierung der TEV-Protease. Als nachgeschaltetes Reportersystem ist hier das Zwei-Schalter System (siehe Fig. 14) dargestellt.

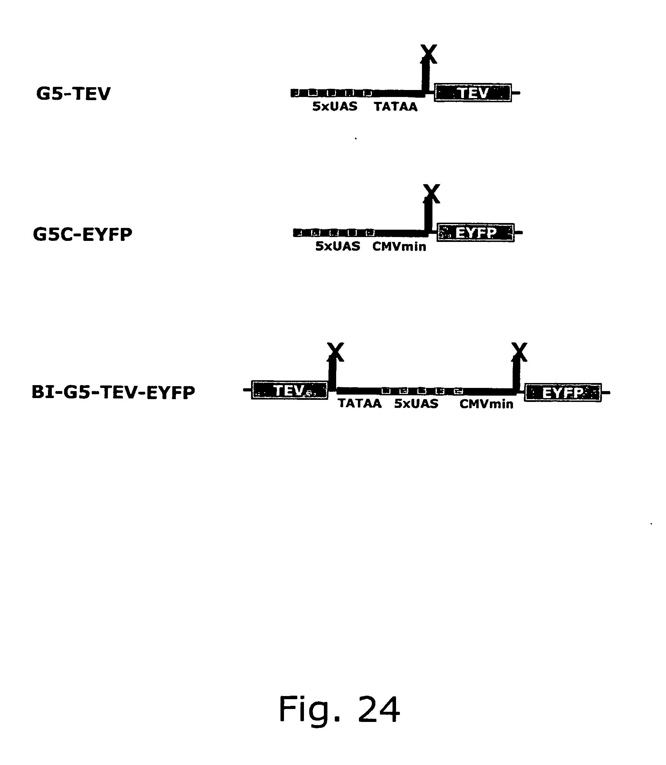

Fig. 24 zeigt die schematische Darstellung der verwendeten Plasmid-Konstrukte für das durch Proteaseexpression rückgekoppelte System zur Endlos-Aktivierung, wobei im einzelnen
   □ das als G5-TEV bezeichnete Konstrukte die TEV-Protease unter der Kontrolle eines Minimal-Promoters, der E1B-TATA Box, und fünf aufeinander folgenden Gal4-abhängigen Enhancer-Elementen aus der Hefe (Upstream Activating Sequence, UAS) exprimiert.
   □ das als GSC-TEV bezeichnete Konstrukt die TEV-Protease unter der Kontrolle des humanen CMV-Minimal-Promoters und fünf aufeinander folgenden Gal4-abhängigen Enhancer-Elementen aus der Hefe (Upstream Activating Sequence, UAS) exprimiert.

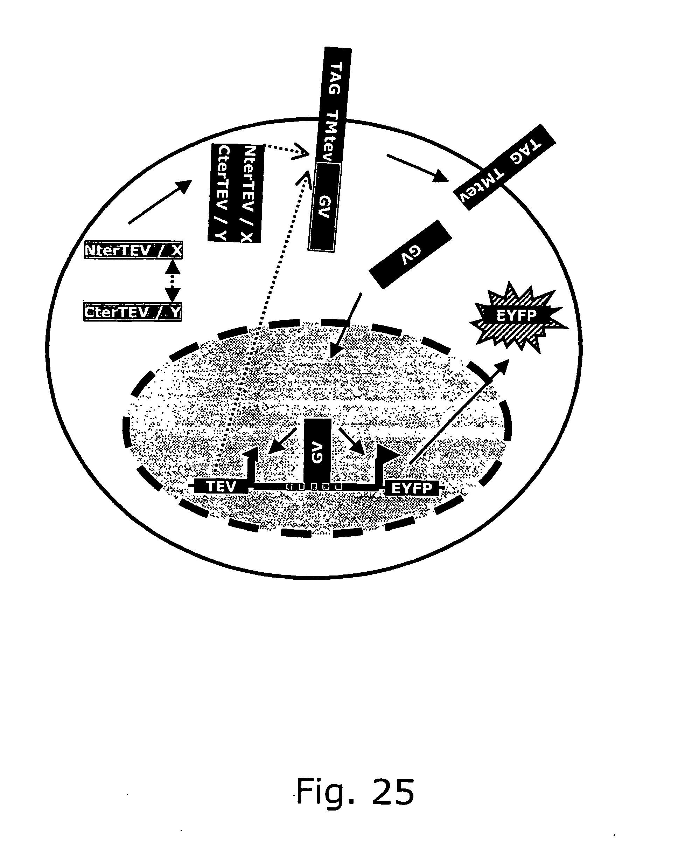

Fig. 25 zeigt ein Ablaufschema des durch Protein-Interaktions regulierte Proteaseexpression rückgekoppelte System zur Endlos-Aktivierung. Nach Ko-Expression von Plasmiden, die zum einen für ein N-terminales TEV Fragment - Protein X-Fusionsprotein und für ein C-terminales TEV Fragment-Protein Y-Fusionsprotein kodieren, kommt es im Falle der spezifischen Interaktion der Proteine oder Proteinabschnitte X und Y, zur funktionellen Rekonstituierung der proteolytischen Aktivität. Daraufhin wird ein stabil exprimierter oder transient ko-transfizierter membran-gebundener und eine TEV-spezifischen Erkennungs- und Spaltsequenz (tev) enthaltender Transkriptionsfaktor (GV) von der Membran gelöst. Es kommt zu einer Translokalisation von GV in den Kern und zur Aktivierung von zwei ko-regulierten oder unabhängigen Reportergenen, von denen das eine die intakte TEV-Protease ist. Die GV-reguliert exprimierte TEV-Protease führt zu einer weiteren Spaltung des konstitutiv exprimierten TM-tev-GVs und führt zu einer dauerhaften Aktivierung des Gesamtreportersystems.

Die einzelnen Ausführungsformen des erfindungsgemäßen Verfahrens werden durch die folgenden Beispiele näher beschrieben.

Alle molekularen Klonierungen und Transfektionen wurden mit Standardprotokollen nach Sambrook et al durchgeführt (Sambrook-J and Russell-DW 2001).

### Beispiel 1: Herstellung der Plasmidvektoren des Cre-Rekombinase basierten Reportersystems

Die funktionalen Elemente der Plasmidvektoren zur Durchführung des Cre-Rekombinase basierten Reportersystems und der Anwendung im Zwei-Hybrid System in Säugerzellen sind in Fig.1 schematisch dargestellt.

Konstruktion der Reporterplasmide: Das Plasmid G5-CAT trägt 5' des Chloramphenicol-Transferase (CAT) Reporter Gens die TATAA-Box des humanen E1B-Gens und fünf aufeinanderfolgende Enhancerelemente (*Upsteam Activating Sequence,* UAS) mit der optimalen Erkennungssequenz für den Gal4-Transkriptionsfaktor aus *saccharomyces cerevisiae*. Das G5-CAT Plasmid-DNA diente als Ausgangsvektor zur Herstellung der verwendeten G5-Reporter und wurde mit Kombinationen von Restriktionsenzymen so geschnitten, daß das CAT-Gen 5' und 3' aus dem Vektorgerüst entfernt werden konnte und die entsprechend vorbereiteten Reportergen-DNA-Fragmente eingesetzt werden konnten. Die Cre-Rekombinase des Bacteriophagen P1 (Cre) wurde dazu mit spezifischen Oligonukleotiden durch die Polymerase Ketten Reaktion (PCR) amplifiziert und 5' mit einem durch die Kozak-Sequenz flankierten Start-Codon modifiziert (Kozak 1989) (Kozak 1987). Zur Klonierung der Plasmidvektoren G5C-EGFP bzw. G5C-Cre dienten die Plasmide tetO-EGFP und tetO-Cre und G5-Cat als Gerüst. Die E1B-TATAA Box und der CAT-Reporter wurde entfernt und durch den humanen CytoMegalieVirus (CMV) minimal Promotor und das entsprechende Reportergen ersetzt.

Kodierende Bereiche der DNA-bindenden Domäne (DBD) des Hefe Transkriptionsfaktors Gal4 und der Transaktivierungsdomäne (TAD) des Herpes Simplex Proteins VP16 wurde mit PCR aus entsprechenden Hefe-Zwei-Hybrid Vektoren amplifiziert und in die eukaryontischen Expressionsvektoren pCMV kloniert. Die Oligonukleotide waren so gestaltet, daß 5' ein Restriktionsschnittstelle und ein Kozak-Sequenz flankiertes ATG eingeführt wurde und 3' das letzte Codon im Leserahmen mit einer weiteren eingeführten Restriktionsschnittstelle ohne Stop-Codon war. Im Vektor pCMV befinden sich 3' der multiplen Klonierungssequenzen (MCS) Stop-Codons der drei möglichen Leseraster, so daß die Vektoren pCMV-Gal4 DBD (G) und pCMV-VP16 TAD (V) (siehe Fig. 1) als Ausgangsvektoren für C-terminale Fusionen mit weiteren Proteinen oder Proteinabschnitten genutzt werden konnten. Der Vektor GV, der für ein Fusionsprotein der Gal4 DBD und der VP16 TAD kodiert, wurde ausgehend von dem Plasmidvektor pCMV-Gal4 und dem für die V 16 TAD kodierenden PCR-Produkt unter Berücksichtigung eines durchgehenden Leserahmens hergestellt (siehe Fig. 1).

Die Cre-aktivierbaren CMV-STOP/REPORTER Konstrukte wurden durch sequentielle Klonierung in pCMV hergestellt. Zunächst wurden mit PCR die STOP-Kassetten generiert, wobei jeweils zwei gleichgerichtete loxP-sites (Erkennungs- und Rekombinationselemente der Cre-Rekombinase) 5' und 3' eines Neomycin-Resistenz und eines Zeocin-Resistenz vermittelndes Elementes (neoR und TKZeoR, siehe Fig. 1) eingebracht wurden. Anschließend wurde das entsprechende Cre-nachgeschaltete Reportergen 3' der STOP-Kassette einkloniert. EGFP und bGal wurde 3' der neoR STOP-Kassette eingeführt und ein gegen BlasticidinS Resistenz vermittelndes Element 3' der TKZeoR STOP-Kassette. Die Funktionalität der STOP-REPORTER Kassetten wurde durch transiente Transfektionen in Cos7-Zellen analysiert. Dazu wurde diese zusammen mit einem Kontrollvektor oder mit einem CMV-Cre Plasmidvektor kotransfiziert, nur nach Kotransfektion mit CMV-Cre konnte die Aktivität der nachgeschalteten Reporter, EGFP, bGal und BlasR, beobachtet werden.

### Beispiel 2: Funktionsanalyse der Komponenten des Cre-Rekombinase basierten Reportersystems durch transiente Transfektion in PC12 und Cos7 Zellen

Die Reporterplasmide oder Kombinationen von Reporterplasmiden wurden durch transiente Transfektionen in die PC12 und Cos7 Zelllinien getestet. Dazu wurden zwischen 10⁵ und 10⁶ PC12- oder Cos7-Zellen unter Verwendung eines GenePulser II mit Capacity Extender-Modul (BioRad, München) elektroporiert. Die Plasmid-DNA wurde mit dem Qiafilter-Verfahren (Qiagen, Hilden) aufgereinigt. Für jede Elektroporation wurden insgesamt immer 5 µg Plasmid-DNA eingesetzt, je nach Ansatz wurde mit Plasmid-DNA eines Leervektors ausgefüllt. Die Elektroporation wurde in speziellen Kuvetten (PeqLab) in dem entsprechenden Zell-Wachstumsmedium und mit den folgenden Parametern durchgeführt: Cos7 Zellen, 10⁵ Zellen in 300µl pro Ansatz, Pulse mit 250 mV bei 500 µF; PC12 Zellen, 10⁶ Zellen in 300µl pro Ansatz, Pulse mit 220 mV bei 960 µF. Nach der Elektroporation wurden die Zellen in 6 cm oder 24 Loch Zellkultur Schalen überführt und kultiviert. Die Analyse erfolgte üblicherweise 12-72 h nach der Transfektion, je nach verwendetem Reporter durch Fluoreszenzmikroskopie, FAC-Sorting (EGFP) oder durch colorimetrische Detektion mit X-Gal in fixierten Zellen (bGal). Die durchschnittliche Transfektionseffizienz bei Cos7 Zellen betrug etwa 40%, bei PC12 Zellen etwa 30%. Die DNA-Menge der Reporter-Plasmide G5-bGal, G5-EGFP, G5C-EGFP, CMV-STOP/EGFP und CMV-STOP/bGal betrug immer 1 µg pro Ansatz, die Mengen der Cre-Rekombinase Reporter, G5-Cre und G5C-Cre wurden variiert. Die Funktionalität der Reporter wurde durch Kotransfektion mit dem Expressionsplasmid des vollständigen Transkriptionsaktivators GV (1 µg pro Ansatz) getestet. Die Ergebnisse waren zwischen PC12 und Cos7 Zellen vergleichbar, mit etwas stärkeren Hintergrund aber insgesamt stärker Signalintensität in Cos7 im Vergleich zu PC12 Zellen.

Das hier verwendete Cre-basierte Reportersystem beruht auf der Gal4-abhängigen transkriptionellen Aktivierung eines Cre-Reporterplasmids. Daraufhin kann das exprimierte Cre-Protein das Ausschneiden einer transkriptionellen STOP-Kassette, die von gleichgerichteten Cre-Erkennungs- und Rekombinationssequenzen (loxP-sites) flankiert ist, katalysieren. Das aktivierte Reportergen steht jetzt unter der Kontrolle des konstitutiven und in den meisten Zelllinien sehr starken humanen CMV-Promotors, was zu einer enormen Verstärkung des Signals führt.

Nach Kotransfektion von GV mit dem G5-bGal Reporter konnte bereits nach 12 h eine X-Gal-Färbung in einer Vielzahl von Zellen nachgewiesen werden. Nach Kotransfektion von GV mit dem G5-EGFP Reporter konnte nur in Cos7-Zellen eine GFP-Fluoreszenz nach 72 h in einer geringen Anzahl von Zellen nachgewiesen werden. Die Transfektionen der G5-bGal- bzw. G5-EGFP-Reporter zeigte keine Hintergrundaktivität. Nach Kotransfektion des G5C-EGFP Reporterplasmids mit GV konnte bereits nach 48 h eine deutlich erhöhte Zahl an GFP positiven Zellen im Vergleich zur Kontroll-Transfektion ohne GV detektiert werden. Einige GFP-positive Zellen konnten allerdings auch in der Kontroll-Transfektion detektiert werden. Die Transfektion von GV zusammen mit CMV-STOP/EGFP oder CMV-STOP/bGal zeigte keinen Hintergrund. Die Kotransfektion von je 1 µg G5-Cre und CMV-STOP/EGFP zeigte eine sehr hohe Zahl an GFP-positiven Zellen nach 48 h. Bereits nach 12 h waren die ersten GFP-Signale zu detektieren. Das beste Verhältnis von GVinduziertem Signal zum Hintergrund ergab sich durch Tranfektion von 50 ng G5-Cre mit je 1µg CMV-STOP/EGFP oder CMV-STOP/bGal und GV. Durch die erhöhte basale Promotor-Aktivität des G5C-Cre Konstruktes war es nicht möglich den Hintergrund durch geringere eingesetzte Mengen so zu reduzieren wie für G5-Cre.

Die Auswertung der transienten Transfektionen zur Analyse der Komponenten des Cre-basierten Reportersystems zeigte folgendes:
1) Das Reportersystem zeichnet sich durch eine sehr hohe Sensitivität aus. 2) Aufgrund der hohen Sensitivität ist es nicht möglich in transienten Transfektionen den Hintergrund der Komponenten des Systems vollständig zu beseitigen. 3) Durch die Verwendung der Cre-Rekombinase ist es möglich EGFP als Cre-nachgeschalteten Reporter mit einer bGalvergleichbaren Sensitivität und Kinetik einzusetzten. 4) Auch bei Verwendung eines relativ starken basalen minimal Promotors (CMVmin) ist EGFP als Reporter weniger sensitiv als bGal.

### Beispiel 3: Anwendung des Cre-Rekombinase basierten Reportersystems nach transienter Transfektion in PC12 und Cos7 Zellen als Zwei-Hybrid System zur Analyse konstitutiver Protein-Interaktionen

Die Anwendung des Cre-Rekombinase basierten Reportersystem im Zwei-Hybrid System in Säugerzellen wurde durch die Analyse bekannter Interaktionspartner getestet (siehe Fig. 2, Ablaufschema des Cre-Rekombinase abhängigen Zwei-Hybrid Systems). Die meisten basischen Helix-Loop-Helix (bHLH) Proteine bilden heterodimere Komplexe aus. Die Interaktion wird über zwei amphipatische Helices vermittelt, die ein charakteristisches Vier-Helix-Bündel ausbilden (Ma, Rould et al. 1994) (Baxevanis and Vinson 1993). Die Interaktion zwischen den bHLH-Proteinen ME2 und Nex bzw. NeuroD diente als ein Testsystem. Dazu wurde die bHLH-Domäne von ME2 mit PCR amplifiziert und als Fusionsprotein mit der Gal4-DBD exprimiert (G-ME2bHLH). Volllänge-NEX bzw. - NeuroD wurden als Fusionsproteine mit der VP16 TAD exprimiert (V-ND bzw. V-Nex).

Ein weiteres bekanntes Motiv, das spezifische Protein-Protein Interaktionen vermittelt, ist das Leuzin-Zipper motif in coiled-coil (cc) Domänen (Lupas 1996). Als weiteres Testsystem wurden Teile der intrazellulären Abschnitte von GBR1 und GBR2 genutzt, die jeweils eine cc-Domäne enthalten und darüber Heterodimere bilden (Kuner, Kohr et al. 1999). GBR1cc (L859 - K960) und GBR2cc (1744 - G849) wurde mit spezifischen Oligonukleotiden mittels PCR amplifiziert und kloniert. GBR1cc wurde an den C-terminus von VP16 fusioniert (V-GBR1cc), GBR2cc an den C-terminus von Gal4 (G-GBR2cc). Als Negativkontrolle wurden Deletionsmutanten der genannten Proteindomänen verwendet GBR1 (L859 - K960 ΔS887-L921) und GBR2 (I744 - G849ΔS785-Q816) (V-GBR1ccDel und G-GBR2ccDel), für diese Mutanten war zuvor durch Immunpräzipitation und mittels Hefe-Zwei-Hybrid Technik gezeigt worden, daß sie nicht interagieren.

Sowohl für die Interaktionspartner G-ME2bHLH und V-NEX bzw. V-ND als auch für G-GBR2cc und V-GBR1cc konnte mit dem Cre-Reportersystem und GFP-Fluoreszenz als Maß in PC12 und Cos7-Zellen eine Interaktion nachgewiesen werden. Die Kontrollen, Einzeltransfektionen und die coiled-coil Deletionskonstrukte (V-GBR1ccDel und G-GBR2ccDel), zeigten ein kein bzw. wesentlich schwächere Signale. Die relative Stärke der Interaktionen ergab sich aus den Experimenten als folgende: GBR2cc/V-GBR1cc >> G-ME2bHLH/V-ND >> G-ME2bHLH/V-NEX. Die Ergebnisse konnten mit bGal als Zwei-Hybrid Reporter bestätigt werden.

### Beispiel 4: Herstellung von stabilen PC12- und Cos7 Zelllinien mit den Komponenten des Cre-Reportersstems

Die Ergebnisse aus den Versuchen des Cre-Rekombinase basierten Zwei-Hybrid Systems nach transienter Transfektion in Säugerzellen zeigten auf, daß 1) die Sensitivität des Systems auch bei Verwendung eines Cre-nachgeschalteten EGFP vergleichbar war mit einem beta-Galaktosidase Reporter und daß 2) aufgrund der erhöhten Sensitivität und des Schaltermechanismus der Cre-Aktivität der Hintergrund nicht vollständig zu reduzieren war. Um den Hintergrund des Systems kontrollieren zu können, wurde zunächst eine Komponente des Tetrazyklin abhängigen Genregulationssystem stabil in PC12- und Cos7-Zellen eingebracht, mit der eine Feinregulation der Expression der Interaktionspartner möglich ist (Gossen, Freundlieb et al. 1995). Dazu wurde ein DNA-Fragment, das für den tet-abhängigen TransAktivator (tTA) unter der Kontrolle des CMV-Promotors kodiert, mit einem gegen das Aminoglykosid G418 Resistenz vermittelnden linearisierten DNA-Element, neoR, kotransfiziert. Drei Tage nach Transfektion wurde mit G418-Selektion (400 µg/ml) begonnen und nach 3-4 Wochen konnten Zellklone identifiziert und isoliert werden. Diese wurden durch Kotransfektion mit einem tTA-abhängigen Reporter (tetO-EGFP) unabhängig nach funktioneller tTA-Expression analysiert. Jeweils ein PC12- bzw. Cos7-Zellklon mit Tetrazyklin-abhängiger Regulation des GFP-Reporters wurde für die weiteren Schritte verwendet.

Im nächsten Schritt wurden die G5-Cre oder G5C-Cre DNA-Fragmente mit einem gegen HygromcinB Resitenz-vermittelnden Fragment kotransfiziert. Vier Wochen nach HygromycinB-Selektion (60 µg/ml) wurden Zellklone isoliert und nach GV-abhängiger Cre-Aktivität analysiert. Dazu wurden die Cos7- bzw. PC12-Zellklone jeweils mit CMV-STOP/EGFP transfiziert und mit CMV-STOP/EGFP und GV kotransfiziert und nach GV/Cre abhängiger Induktion der GFP-Fluoreszenz analysiert. Alle Cos7- und die meisten der PC12-Zellkone, die durch GV eine Erhöhung der Cre-Aktivität aufweisen konnten, zeigten ebenfalls eine gewisse Cre-Hintergrund Aktivität. PC12-Zellklon #20 zeigte absolut keine konstitutive Cre-Expression, d.h. nach Transfektion mit CMV-STOP/EGFP konnte keine GFP-positive Zelle detektiert werden (siehe Fig. 3, unten links). Nach Kotransfektion von CMV-STOP/EGFP und GV wurden dahingegen eine Vielzahl GFP-positiver Zellen nachgewiesen (siehe Fig. 3, oben links).

PC12-Zellen sind eine etablierte Kulturzellline eines Pheochromozytoms einer Ratte und stammen damit von sympato-adrenergem Gewebe des Nebennierenmarks ab (Greene and Tischler 1976). Diese Zellen sind mit *nerve growth factor* (NGF) stimulierbar, und differenzieren NGF-abhängig zu einem Neuron-verwandten Zelltyp, der postmitotisch ist und neuronale Fortsätzte ausbildet. Um das Cre-basierte Reportersytem unter diesen postmitotischen Bedingungen zu testen, wurden GV-transfizierte PC12-Zellen der Linie #20 unmittelbar nach Transfektion mit NGF-stimuliert (2,5 S NGF, 5 ng/ml, Promega) und drei Tage nach GV-induzierter GFP-Fluoreszenz analysiert (siehe Fig. 4). Die Effizienz des Cre-Reportersystems (niedrigere Vergrößerung Fig. 4 oben) wurde nicht durch die NGF induzierter Differenzierung beeinflußt (niedrigere Vergrößerung Fig. 4 oben). Die neuronale Morphologie der Zellen wurde durch das Cre-Reportersystem nicht beeinträchtigt (niedrigere Vergrößerung Fig. 4 oben). Zusammengenommen zeigen diese Resultate, daß das Cre-Reportersystem hintergrundfrei in stabilen PC12-Zellklonen funktioniert, auch unter postmitotischen Bedingungen.

Um alle notwendigen Komponenten des Cre-basierten Reportersystems stabil zu exprimieren wurden PC12-Zellen der Linie #20 mit den linearisierten Plasmidkonstrukten CMV-STOP/EGFP (STOP = neoR) und CMV-STOP/BlasR (STOP = TKZeoR) kotransfiziert und mit Zeocin (500 µg/ml, Invitrogen) selektioniert. GFP-negative und BlasticidinS-sensitive Zellklone wurden durch Transfektion mit GV analysiert. GFP-positive Zellen des Zellklons #20.4 konnten zwei Tage nach GV Transfektion nachgewisen werden (siehe Fig. 5). Drei bis vier Wochen nach GV Transfektion und BlasticidinS-Selektion (2 µg/ml, Calbiochem) konnten resistente Zellklone identifiziert werden, wobei alle Zellen dieser Klone GFP-positiv waren (siehe Fig. 6). Zusammengenommen zeigen diese Versuche, daß der Zellklon 20.4 alle Komponenten des Cre-basierten Reportersystems stabil integriert hat und funktional exprimiert.

### Beispiel 5: Anwendung des Zwei-Hybrid Systems in der PC12 Zelllinie 20.4 zur Analyse konstitutiver Protein-Interaktionen

Wie in Beispiel 4 beschrieben, exprimiert die PC12 Zelllinie 20.4 alle Komponenten des Cre-basierten Reportersystems vollständig funktional. Um zu testen, ob die Zelllinie 20.4 für die Anwendung im Zwei-Hybrid System (siehe Fig. 2) genutzt werden kann, wurden die in Beispiel 3 beschriebenen bekannten Interaktiospartner analysiert. Zwei Tage nach Kotransfektion der Interaktionspartner G-ME2bHLH und V-ND konnten keine GFP-positiven Zellen detektiert werden (siehe Fig. 7, unten links). Nach Einzeltransfektionen von G-ME2bHLH bzw. V-ND konnten ebenfalls keine GFP-positiven Zellen nachgewiesen werden (siehe Fig. 7, mittlere Abbildungen links). Die Kontrolle nach Transfektion mit GV zeigte die erwartete hohe Zahl an GFP-positiven Zellen (siehe Fig. 7, oben links). Dieses Ergebnis, zusammen mit der Beobachtung der vollständigen Abwesenheit konstitutiven Hintergrunds in Bezug auf uninduzierte Cre Expression, ließen vermuten, daß zumindest der Gal4-abhängige Cre-Reporter in einen Bereich des Heterochromatins integriert war und somit nur durch sehr starken Transkaktivatoren wie GV zugänglich war. Deshalb wurden im folgenden Versuch die gleiche Zwei-Hybrid Analyse nach vorübergehender Zugabe von Trichostain A (3 µM, für 12 h, Sigma) durchgeführt (Fig. 8). TSA wirkt als Inhibitor von Deacetylasen, die Hemmung der Deacetylierung führt zu einem insgesamt weniger dicht verpackten, transkriptionell inaktiven Heterochromatin-Abschnitten. Nach vorübergehender Zugabe von TSA ins Kultivierungsmedium der PC12 20.4 Zellen konnten nunmehr zwei Tage nach Transfektion der Interaktionspartner G-ME2bHLH und V-ND eine Vielzahl GFP-positiver Zellen detektiert werden (siehe Fig. 8, unten links). Nach Einzeltransfektionen von G-ME2bHLH bzw. V-ND konnten keine GFP-positiven Zellen nachgewiesen werden (siehe Fig. 8, mittlere Abbildungen links). Die Kontrolle nach Transfektion mit GV zeigte die erwartet hohe Zahl an GFP-positiven Zellen (siehe Fig. 8, oben links). Die relativ hohe Konzentration von 3 µM TSA für die Dauer von 12h führte allerdings auch zu einer geringeren Zahl an überlebenden Zellen (vgl. Fig. 7 und Fig. 8, rechte Spalte). Die TSA-Konzentration mit dem besten Verhältnis aus Zellüberleben und nahezu Hintergrund freier Detektion der Interaktion von G-ME2bHLH und V-ND war 300 µM für 12h. Für die etwas stärkere Interaktion von GBR2cc und V-GBR1cc (siehe Beispiel 3) wurden die Versuche mit TSA-Konzentration von 200 µM TSA für 12 h durchgeführt (siehe Fig. 9). Nur nach Kotransfektion der Interaktionspartner GBR2cc und V-GBR1cc konnte eine Vielzahl an GFP-positiven Zellen detektiert werden, nach Einzeltransfektionen und nach Kotransfektion der cc-Deletionsmutanten, GBR2ccDel und V-GBR1ccDel, konnten mikroskopisch keine GFP-positiven Zellen nachgewiesen werden (siehe Fig. 9, untere Leiste). Die Analyse durch Fluorescent Activated Cell Sorting (FACS) konnte diese Ergebnisse bestätigen (siehe Fig. 10).

Zusammengenommen zeigen diese Ergebnisse, daß eine Zwei-Hybrid Analyse in den PC 12 Zellen der Linie 20.4 durchführbar ist und daß die Sensitivität bzw. der Hintergrund durch die Zugabe von TSA gesteuert werden kann.

### Beispiel 6: Anwendung des Cre-Rekombinase basierten Reportersystems als Zwei-Hybrid System zur Analyse induzierter und transienter Interaktionen

Dieses Beispiel beschreibt die Nutzung des Cre-Rekombinase basierten Reportersystems im Zwei-Hybrid Ansatz zum Nachweis einer stimulus-induzierten und transienten Interaktion *in vivo.* Wie in Fig. 11 schematisch dargestellt, reicht die vorübergehende Aktivierung des Cre-Reporters um über die Funktion der kernlokalisierten Cre-Rekombinase eine dauerhafte Aktivierung nachgeschalteter Reporter zu gewährleisten. Ein gut charakterisiertes Beispiel für eine induzierte Protein-Protein Interaktion ist die phosphorylierungs-abhängige Bindung des Transkriptionsaktivators CREB an den Transkriptions Ko-Aktivator CBP (CREB *Binding Protein*) (Chrivia, Kwok et al. 1993). Zum Beispiel führt Protein Kinase A (PKA) vermittelte Phosphorylierung von CREB an Ser133, in der sogenannten *kinase-inducible-domain* (KID), zur spezifischen Bindung an die sogenannte KIX-Domäne von CBP. Die Stimulation der PKA kann durch Zugabe der Adenylat-Zyklase stimulierenden Substanz Forskolin erreicht werden, was zu einer transienten Erhöhung des intrazellulären cAMP-Spiegels und damit zur PKA Aktivierung führt. Nach Aufhebung der PKA-Stimulation durch entfernen des Forskolins aus dem Kultur-Medium von Zellen kommt es über zell-endogen aktive Phosphatasen zur schnellen Dephosphorylierung von CREB-Ser133. Zur Analyse im Zwei-Hybrid System in den PC 12 20.4 Zellen, wurde CREB C-terminal an die Gal4 DBD fusioniert (G-CREB) und die CBP-KIX Domäne an den C-terminus der VP16 TAD (V-CBP-KIX).
Drei Tage nach Transfektion von PC12 20.4 Zellen mit G-CREB und V-CBP-KIX und den entsprechenden Kontrollen, wurde der Nachweis der Interaktionen durch FACS analysiert. Ohne Forskolin Stimulation führten weder die Einzel- noch Kotransfektion der Konstrukte zu einer signifikanten Aktivierung des Cre/EGFP Reportersystems (siehe Fig. 12, obere und untere Graphik, umrandete Balken). Nach vorübergehender Forskolin-Stimulation unmittelbar nach Transfektion (4 µM für 12 h) und FACS-Analyse nach 3 Tagen konnte ein deutlicher Anstieg der Gesamtzahl GFP-positiver Zellen und der kumulierten Gesamtfluoreszenz nach Kotransfektion von G-CREB und V-CBP-KIX beobachtet werden (siehe Fig. 12, schwarzer Balken, rechts). Ein weitaus geringerer aber ebenfalls signifikanter Anstieg der Anzahl GFP-positiver Zellen und Gesamtfluoreszenz konnte Forskolin abhängig für die Einzeltransfektion mit G-CREB gemessen werden. Dies läßt sich durch die ebenfalls stimulierte Interaktion von G-CREB mit dem endogen vorhandenen CBP oder verwandter transkriptioneller Ko-Faktoren erklären. Die stärkere relative Zunahme der Gesamt-Fluoreszenz mit und ohne Stimulation im Vergleich zur etwas geringeren relativen Zunahme an GFP-positiven Zellen deutet auf eine kinetische Komponente in dem System hin.
Zusammengenommen zeigen diese Ergebnisse, daß auch Zwei-Hybrid Analysen in den PC 12 20.4 Zellen durchgeführt werden können mit Interaktionen die stimulus-abhängig und transient sind. Wie transient diese Interaktion ist wurde von Chawla und Bading gezeigt. Ihre Analysen zur CREB Phosphorylierung nach kurzzeitigen Kalzium Signalen ergab, dass S 133 Phosphorylierung zur schnellen Aktivierung von CREB führt, das Protein jedoch schon nach wenigen Minuten durch Dephosphorylierung wieder inaktive ist (Chawla und Bading, 2001) (Dephosphorylierung von S133 führ zur Dissoziation von CREB und CBP-KIX).

### Beispiel 7: Proteolytische Aktivierung eines membranverankerten Transkriptionsaktivators nach transienter Kotransfektion und die damit verbundene Aktivierung des Reportersystems in PC12 20.4.

Membranverankerung des Gal4/VP16 Fusionsproteins in PC12 20.4 und die Aktivierung des Reportersystems nach proteolytische Abspaltung.
In diesem Beispiel wird die prinzipielle Machbarkeit des Protease Schalters an der Membran in PC12 20.4 Zellen beschrieben. Ziel war es einen weiteren intrazellulären Mechanismus zu etablieren der proteolytische Ereignisse an der Peripherie in permanente Signale transduziert. Dazu wurde das zur Rekombinase-Aktivierung notwendige Gal4/VP16 Fusionsprotein an der Membran verankert (TM-GV) und sollte dann durch eine spezifische proteolytische Spaltung das Reportersystem im Zellkern aktivieren. Eine stabile Lokalisation von Gal4/VP16 an der Zellmembran wurde durch Fusion an die Transmembrandomäne des PDGF (Platelet Derived Growth Factor)- Rezeptors erreicht, Insertion und korrekte Orientierung des Konstrukts in der Membran wurde durch eine N-terminale Signalsequenz sichergestellt. Die Effektivität der Aktivierung wurde über die Expression des Reporters EGFP analysiert. Dazu wurden die transfizierten Zellen nach 48h trypsiniert und in einem FAC-Analyzer (FACSCalibur von BD Bioscience) der Anteil an positiven Zellen quantitativ bestimmt. Für dieses Experiment wurden auf einer 24-Loch Platte je 1,5x10⁵ PC12 20.4 Zellen ausplattiert und am folgenden Tag mit jeweils 0,5 µg der entsprechenden Plasmid-DNA transfiziert (Lipofectamine2000; Invitrogen). Transiente Expression des chimären Membranproteins in PC12 20.4 Zellen führte zu keiner signifikanten Aktivierung des Reportersystems (Fig. 15), was zeigt, daß der GV-Transkriptionsaktivator stabil an der Membran verankert ist. Zur proteolytischen Abspaltung des GV-Transaktivators wurde in der DNA Sequenz zwischen PDGF-Transmembransegment und Gal4/VP16 die kodierenden Basen der 7 Aminosäuren (ENLYFQG) langen Erkennungssequenz der Tobacco Etch Virus (TEV) Nla Protease eingefügt (TEV-Protease). Die Einführung dieser oder alternativer Proteaseschnittstellen hatte keine unspezifische Freisetzung des Gal4/VP16 Fusionsproteins zur Folge. Koexpression der TEV-Protease führte jedoch zur effizienten Spaltung des TM/tev/GV Konstrukts und anschließend zu einer deutlichen Aktivierung des Cre/EGFP Reportersystems. In einem weiteren Schritt sollte gezeigt werden, daß die TEV-Protease auch nach Membranverankerung aktiv ist. Letzteres ist Grundvoraussetzung für die Interaktionsanalyse von Membranproteine. Zu diesem Zweck wurde die TEV Protease in Analogie zum Gal4/VP16 Reporter N-Terminal mit der Transmembrandomäne des PDGF-Rezeptors fusioniert und in PC12 20.4 Zellen mit dem TM/tevS/GV Konstrukt koexprimiert. Das Ergebnis zeigte keinen signifikanten Unterschied in der Aktivierung des Cre/EGFP Reportersystems von löslicher und membrangebundener Protease. Dieses Beispiel unterstreicht die prinzipielle Eignung der Methode zur Detektion von Protein-Interaktionen außerhalb des Zellkerns, insbesondere an der Zellmembran. Voraussetzung dafür ist die funktionelle Kopplung einer Interaktion an die proteolytische Spaltung, dies kann durch Transkomplementation einer Protease oder bei einer niederen Konzentration der beteiligten Partner auch durch Herstellung einer geeigneten Proximität zwischen Protease und Schnittstelle erfolgen (Fig. 15).

In Analogie zum Gal4/VP16 Transkriptionsaktivator-Konstrukt wurde der Cre-Reporter direkt an der Zellmembran verankert, wobei dessen proteolytische Freisetzung dann direkt zur Aktivierung von EGFP im Zellkern führt. Bei schwachen Interaktionen von sehr raren Proteinen besteht die Möglichkeit, daß die beschriebene Doppelstrategie nicht sensitiv genug ist eine Interaktion an der Membran in ein Signal zu transduzieren. Eine weitaus höhere Sensitivität wird erreicht wenn statt des bisher verwendeten Gal4/VP16 Aktivators, direkt die Cre-Rekombinase mittels einer PDGF Tansmembrandomäne und verbunden durch eine Protease Erkennungstelle an die Membran gekoppelt wird. Überexpression des TM-Cre-Konstruktes führte zunächst zu einer erhöhten Hintergrundaktivität und mußte durch schwächere Expression kompensiert werden. Dazu wurde das TM-Cre Konstrukt stabil in PC 12 Zellen transfiziert und auf hintergrundfreie Klone selektioniert. Diese Zellinien, kotransfiziert mit TEV-Protease und STOP-EGFP-Reporterplasmid zeigten anschließend eine rasche und deutliche Grünfärbung.

### Beispiel 8: Transkomplementation der TEV-Protease

Eine Möglichkeit für die Umsetzung einer Protein-Interaktion in die proteolytische Abspaltung eines membrangebundenen Transkriptionaktivators bietet die Transkomplementation der TEV Protease. Die Nla Protease des Tobacco Etch Virus gehört zu der Familie der C4 Cystein Peptidasen, die strukturell den Trypsin ähnlichen Serin Protease homolog sind. Sie weisen demnach ebenfalls eine bilobale β-Barrel Struktur auf, in der drei Aminosäuren charakteristisch angeordnet sind. Aufmodelierung der Sequenz der TEV-Protease auf eine bekannte 3D-Struktur einer verwandten Protease (Dengue virus NS3 Protease PDB 1bef) mit Hilfe der Swissmodel Software legte nahe, dass diese Aminosäuren, die sogenannten Triade, auf die beiden Loben der Struktur verteilt sind und sich gegenüberstehen. Zwar sind die beiden Loben physikalisch miteinander verbunden, scheinen sich aber voneinander unabhängig zu falten. Ansatzpunkt für die Transkomplemetation war das Ziel die Aminosäuren der Triade so zu trennen, dass sie auf unterschiedlichen Fragmenten liegen, die für sich gesehen zwar eine Tertiärstruktur ausbilden könnten, jedoch keine Aktivität zeigen. Mittels spezifischer PCR Oligonukleotide wurde die DNA für die N und C-terminale Fragmente der TEV-Protease amplifiziert wobei 5' eine NheI und eine 3' KpnI Restriktionsschnittstelle eingeführt wurde und in einem Plasmid über NheI und KpnI an die 3'-termini der über die PDGF-Transmembrandomäne verankerten "coiled coil" Domänen von GBR1 und GBR2 (siehe Beispiel 3 und 5) kloniert. Diese Konstrukte waren so konzipiert, daß die Interaktion der membranverankerten "coiled-coil" Regionen, N- und C-terminales Fragment der TEV Protease zu einer aktiven Form wieder zusammenfügen und dies über die Abspaltung des ebenfalls membranverankerten Gal4/VP16 Transaktivators in PC 12 20.4 Zellen detektiert werden kann. Um sicherzustellen, daß keine der beiden generierten TEV- "Untereinheiten" proteolytische Aktivität besitzt wurden sie unabhängig transfiziert und getestet. Ebenso wurde durch Einklonierung der jeweiligen GBR Deletionsmutanten (siehe Beispiel 5) überprüft, dass die C und N-terminalen Loben keine Aktivität nach Kotransfektion erlangen. Im Experiment zeigte sich, das sich mehrere Bereiche in der TEV Protease für die Transkomplementation eignen, insbesondere die Region zwischen Aminosäure 60 und 80 sowie insbesondere der Bereich zwischen 95 und 120. Die Teilung der Protease an diesen Stellen führte zu zwei inaktiven Fragmenten, die Koexpression dieser Varianten fusioniert an Interaktionsdomänen führte in vielen Fällen zur Rekonstitution der proteolytischen Aktivität, einige Beispiele werden im Folgenden genauer beschrieben. Die beiden TEV Fragmente Gly1-Thr70 und Thr71-Gly243 erlangten bei Fusion an die membranverankerten, interagierenden "coiled coil" Domänen von GBR1 und GBR2 über 30% der Aktivität der intakten Protease (Fig.18). Weder Einzelexpression noch Koexpression der Fragmente fusioniert an die nichtinteragierenden Mutanten der GBR-Domänen führte zu einer vergleichbar starken Aktivierung des Reportersystems (Fig.18a). Eine ähnlich gute Aktivierung zeigte eine Kombination aus den teilweise überlappenden Fragmenten Gly1-Thr70 und His61-Gly243. Teile eines Proteins, die durch Transkomplementation zu einem funktionalen Gesamt-Protein führen sollen, können sich daher auch teilweise überlappen.
Im Bereich der Position 100 (der vorausgesagten Verbindungsdomäne von N-terminale und C-terminalem Lobus) wurden mehrere Fragmente gefunden, welche nach Interaktion Aktivität erlangen. Insbesondere die Kombination der Fragmente Glyl-T118 und K119-Gly243, zeichnete sich durch sehr hohe Aktivität nach Transkomplementation aus (Fig. 18b)

### Literatur:

Ashman, K., M. F. Moran, et al. (2001). "Cell signalling - the proteomics of it all." Sci STKE 2001(103): E33.
Baron, U., S. Freundlieb, et äl. (1995). "Co-regulation of two gene activities by tetracycline via a bidirectional promoter." Nucleic Acids Res 23(17): 3605-6.
Barrett-AJ, Rawlings-ND, et al. (1998). Handbook of Proteolytic Enzymes, Academic Press.
Baxevanis, A. D. and C. R. Vinson (1993). "Interactions of coiled coils in transcription factors: where is the specificity?" Curr Opin Genet Dev 3(2): 278-85.
Bazan, J. F. and R. J. Fletterick (1988). "Viral cysteine proteases are homologous to the trypsin-like family of serine proteases: structural and functional implications." Proc Natl Acad Sci U S A 85(21): 7872-6.
Bazan, J. F. and R. J. Fletterick (1989). "Comparative analysis of viral cysteine protease structural models." FEBS Lett 249(1): 5-7.
Brizuela, L., P. Braun, et al. (2001). "FLEXGene repository: from sequenced genomes to gene repositories for high-throughput functional biology and proteomics." Mol Biochem Parasitol 118(2): 155-65.
Broad, e. a. (1999). Protease Based Gene Switching System. PCT WO 99/11801 A2, Zeneca Ltd, GB.
Buchholz, F., P. O. Angrand, et al. (1998). "Improved properties of FLP recombinase evolved by cycling mutagenesis." Nat Biotechnol 16(7): 657-62.
Chawla, S. and Bading, H. (2001). "CREB/CBP and SRE-interacting transcriptional regulators are fast on-off switches: duration of calcium transients specifies the magnitude of transcriptional responses." J. Neurochem 2001 Nov;79(4):849-58
Chrivia, J. C., R. P. Kwok, et al. (1993). "Phosphorylated CREB binds specifically to the nuclear protein CBP." Nature 365(6449): 855-9.
Ehrhard, K. N., J. J. Jacoby, et al. (2000). "Use of G-protein fusions to monitor integral membrane protein-protein interactions in yeast." Nat Biotechnol 18(10): 1075-9.
Esposito, D. and J. J. Scocca (1997). "The integrase family of tyrosine recombinases: evolution of a conserved active site domain." Nucleic Acids Res 25(18): 3605-14.
Eyckerman, S., A. Verhee, et al. (2001). "Design and application of a cytokine-receptorbased interaction trap." Nat Cell Biol 3(12): 1114-9.
Faber, K. N., A. M. Kram, et al. (2001). "A novel method to determine the topology of peroxisomal membrane proteins in vivo using the tobacco etch virus protease." J Biol Chem 276(39): 36501-7.
Fearon, E. R., T. Finkel, et al. (1992). "Karyoplasmic interaction selection strategy: a general strategy to detect protein-protein interactions in mammalian cells." Proc Natl Acad Sci U S A 89(17): 7958-62.
Feil, R., J. Brocard, et al. (1996). "Ligand-activated site-specific recombination in mice." Proc Natl Acad Sci U S A 93(20): 10887-90.
Fields, S. and O. Song (1989). "A novel genetic system to detect protein-protein interactions." Nature 340(6230): 245-6.
Gavin, A. C., M. Bosche, et al. (2002). "Functional organization of the yeast proteome by systematic analysis of protein complexes." Nature 415(6868): 141-7.
Gossen, M., S. Freundlieb, et al. (1995). "Transcriptional activation by tetracyclines in mammalian cells." Science 268(5218): 1766-9.
Greene, L. A. and A. S. Tischler (1976). "Establishment of a noradrenergic clonal line of rat adrenal pheochromocytoma cells which respond to nerve growth factor." Proc Natl Acad Sci U S A 73(7): 2424-8.
Guo, F., D. N. Gopaul, et al. (1997). "Structure of Cre recombinase complexed with DNA in a site-specific recombination synapse." Nature 389(6646): 40-6.
Haj, F. G., P. J. Verveer, et al. (2002). "Imaging Sites of Receptor Dephosphorylation by PTP1B on the Surface of the Endoplasmic Reticulum." Science 295(5560): 1708-11.
Hazzalin, C. A. and L. C. Mahadevan (2002). "MAPK-regulated transcription: a continuously variable gene switch?" Nat Rev Mol Cell Biol 3(1): 30-40.
Hirowatari, Y., M. Hijikata, et al. (1995). "A novel method for analysis of viral proteinase activity encoded by hepatitis C virus in cultured cells." Anal Biochem 225(1): 113-20.
Hubsman, M., G. Yudkovsky, et al. (2001). "A novel approach for the identification of protein-protein interaction with integral membrane proteins." Nucleic Acids Res 29(4): E18.
Hunter, T. (2000). "Signaling--2000 and beyond." Cell 100(1): 113-27.
Husi, H., M. A. Ward, et al. (2000). "Proteomic analysis ofNMDA receptor-adhesion protein signaling complexes." Nat Neurosci 3(7): 661-9.
Huttner, W. B. and A. Schmidt (2000). "Lipids, lipid modification and lipid-protein interaction in membrane budding and fission--insights from the roles of endophilin A1 and synaptophysin in synaptic vesicle endocytosis." Curr Opin Neurobiol 10(5): 543-51.
Johnsson, N. and A. Varshavsky (1994). "Split ubiquitin as a sensor of protein interactions in vivo." Proc Natl Acad Sci U S A 91(22): 10340-4.
Kamada, S., H. Kusano, et al. (1998). "A cloning method for caspase substrates that uses the yeast two-hybrid system: cloning of the antiapoptotic gene gelsolin." Proc Natl Acad Sci U S A 95(15): 8532-7.
Karimova, G., J. Pidoux, et al. (1998). "A bacterial two-hybrid system based on a reconstituted signal transduction pathway." Proc Natl Acad Sci U S A 95(10): 5752-6.
Kellendonk, C., F. Tronche, et al. (1996). "Regulation of Cre recombinase activity by the synthetic steroid RU 486." Nucleic Acids Res 24(8): 1404-11.
Kozak, M. (1987). "An analysis of 5'-noncoding sequences from 699 vertebrate messenger RNAs." Nucleic Acids Res 15(20): 8125-48.
Kozak, M. (1989). "The scanning model for translation: an update." J Cell Biol 108(2): 229-41.
Kuner, R., G. Kohr, et al. (1999). "Role of heteromer formation in GABAB receptor function." Science 283(5398): 74-7.
Lewandoski, M. (2001). "Conditional control of gene expression in the mouse." Nat Rev Genet 2(10): 743-55.
Luban, J. and S. P. Goff(1995). "The yeast two-hybrid system for studying protein-protein interactions." Curr Opin Biotechnol 6(1): 59-64.
Lupas, A. (1996). "Coiled coils: new structures and new functions." Trends Biochem Sci 21(10): 375-82.
Ma, P. C., M. A. Rould, et al. (1994). "Crystal structure of MyoD bHLH domain-DNA complex: perspectives on DNA recognition and implications for transcriptional activation." Cell 77(3): 451-9.
Maroun, M. and A. Aronheim (1999). "A novel in vivo assay for the analysis of protein-protein interaction." Nucleic Acids Res 27(13): e4.
Marshall, C. J. (1995). "Specificity of receptor tyrosine kinase signaling: transient versus sustained extracellular signal-regulated kinase activation." Cell 80(2): 179-85.
Mattheakis, L. C., S. E. Olivan, et al. (1999). "Expression of cre recombinase as a reporter of signal transduction in mammalian cells." Chem Biol 6(11): 835-44.
Metzger, D., J. Clifford, et al. (1995). "Conditional site-specific recombination in mammalian cells using a ligand-dependent chimeric Cre recombinase." Proc Natl Acad Sci U S A 92(15): 6991-5.
Michnick, S. W. and A. Galarneau (2001). A Protein FragmentComplementation Assay (PCA) for the Detection of ProteinProtein Interactions Based on the E.coli TEM-1-Beta-Lactamase. PCT WO 01/94617 A2.
Michnick, S. W. and I. Remy (2001). Protein Fragment Complementation Assays for the Detection of Biological or Drug Interactions. United States Patent US 6,294,330. USA, Odyssey Pharmaceuticals Inc.
Migaud, M., P. Charlesworth, et al. (1998). "Enhanced long-term potentiation and impaired learning in mice with mutant postsynaptic density-95 protein." Nature 396(6710): 433-9.
Mohler, W. A. and H. M. Blau (1996). "Gene expression and cell fusion analyzed by lacZ complementation in mammalian cells." Proc Natl Acad Sci U S A 93(22): 12423-7.
Nunes-Duby, S. E., H. J. Kwon, et al. (1998). "Similarities and differences among 105 members of the Int family of site-specific recombinases." Nucleic Acids Res 26(2): 391-406.
Pawson, T. and J. D. Scott (1997). "Signaling through scaffold, anchoring, and adaptor proteins." Science 278(5346): 2075-80.
Pelletier, J. N., F. X. Campbell-Valois, et al. (1998). "Oligomerization domain-directed reassembly of active dihydrofolate reductase from rationally designed fragments." Proc Natl Acad Sci U S A 95(21): 12141-6.
Pestova, T. V., V. G. Kolupaeva, et al. (2001). "Molecular mechanisms oftranslation initiation in eukaryotes." Proc Natl Acad Sci U S A 98(13): 7029-36.
Prochiantz, A. (2000). "Messenger proteins: homeoproteins, TAT and others." Curr Opin Cell Biol 12(4): 400-6.
Rigaut, G., A. Shevchenko, et al. (1999). "A generic protein purification method for protein complex characterization and proteome exploration." Nat Biotechnol 17(10): 1030-2.
Rojo-Niersbach, E., D. Morley, et al. (2000). "A new method for the selection of protein interactions in mammalian cells." Biochem J 348(Pt 3): 585-90.
Rossi, F., C. A. Charlton, et al. (1997). "Monitoring protein-protein interactions in intact eukaryotic cells by beta-galactosidase complementation." Proc Natl Acad Sci U S A 94(16): 8405-10.
Ryan, M. D. and M. Flint (1997). "Virus-encoded proteinases of the picornavirus supergroup." J Gen Virol 78(Pt 4): 699-723.
Sambrook-J and Russell-DW (2001). Cold Spring Harbor, NY, CSHL Press.
Sauer, B. (1990). Site Specific Recombination of DNA in Eucaryotic Cells. United States Patent 4,959,317. USA, Du Pont.
Sauer, B. (1998). "Inducible gene targeting in mice using the Cre/lox system." Methods 14(4): 3 81-92.
Shaikh, A. C. and P. D. Sadowski (2000). "Trans complementation of variant Cre proteins for defects in cleavage and synapsis." J Biol Chem 275(39): 30186-95.
Shioda, T., S. Andriole, et al. (2000). "A green fluorescent protein-reporter mammalian two-hybrid system with extrachromosomal maintenance of a prey expression plasmid: application to interaction screening." Proc Natl Acad Sci U S A 97(10): 5220-4.
Siegel, R. M., F. K. Chan, et al. (2000). "Measurement of molecular interactions in living cells by fluorescence resonance energy transfer between variants of the green fluorescent protein." Sci STKE 2000(38): L1.
Simons, K. and E. Ikonen (1997). "Functional rafts in cell membranes." Nature 387(6633): 569-72.
Simpson, J. C., R. Wellenreuther, et al. (2000). "Systematic subcellular localization of novel proteins identified by large-scale cDNA sequencing." EMBO Rep 1(3): 287-92.
Uhlmann, F., D. Wernic, et al. (2000). "Cleavage of cohesin by the CD clan protease separin triggers anaphase in yeast." Cell 103(3): 375-86.
Ullmann, A., D. Perrin, et al. (1965). "[Identification, by in vitro complementation and purification, of a peptide fraction of Escherichia coli beta-galactosidase]." J Mol Biol 12(3): 918-23.
Vagner, S., B. Galy, et al. (2001). "Irresistible IRES. Attracting the translation machinery to internal ribosome entry sites." EMBO Rep 2(10): 893-8.
Xu, Y., D. W. Piston, et al. (1999). "A bioluminescence resonance energy transfer (BRET) system: application to interacting circadian clock proteins." Proc Natl Acad Sci U S A 96(1): 151-6.
Yasukawa, H., A. Sasaki, et al. (2000). "Negative regulation of cytokine signaling pathways." Annu Rev Immunol 18: 143-64.
Ziauddin, J. and D. M. Sabatini (2001). "Microarrays of cells expressing defined cDNAs." Nature 411(6833): 107-10.

## Patentansprüche

1. Verfahren zur Detektion und Analyse von Protein-Interaktionen in einer Zelle, umfassend die Schritte
u) Expression eines
u1) ersten Fusionsproteins, umfassend den ersten Interaktionspartner und einen Teil einer Tobacco-Etch-Virus-Protease (TEV-Protease) und
u2) eines zweiten Fusionsproteins, umfassend den zweiten Interaktionspartner und einen weiteren Teil der TEV-Protease, und
u3) eines durch Proteolyse aktivierbaren oder inaktivierbaren Reporterproteins ausgewählt aus der Gruppe, bestehend aus fluoreszierenden Reporterproteinen, Luciferase, beta-Galaktosidase, alkalische Phosphatase, beta-Lactamase, Resistenz gegen zytotoxische Substanzen vermittelnde Proteine, zytotoxische Proteinen, Proteinen, die Wachstum und Morphologie der Zelle ändern und Rekombinasen,
in der Zelle,
v) Rekonstituierung einer funktionalen TEV-Protease durch die Interaktion des ersten und des zweiten Interaktionspartners miteinander,
w) Aktivierung des durch Proteolyse aktivierbaren oder Inaktivierung des durch Proteolyse inaktivierbaren Reporterproteins durch die rekonstituierte funktionale TEV-Protease aus Schritt v).

2. Verfahren nach Anspruch 1, **dadurch gekennzeichnet, daß** in Schritt u) als Komponente u3) ein durch Proteolyse aktivierbares Reporterprotein exprimiert wird, dessen Reporteraktivität durch die Insertion einer zusätzlichen Aminosäuresequenz, die ein- oder beidseitig von mindestens einer Erkennungsstelle und/oder Schnittstelle für eine TEV-Protease flankiert ist, inaktiviert ist.

3. Verfahren nach Anspruch 1, **dadurch gekennzeichnet, daß** in Schritt u) als Komponente u3) ein durch Proteolyse inaktivierbares Reporterprotein exprimiert wird, das mindestens eine Erkennungs- und Schnittstellen für eine TEV-Protease enthält und dessen Reporteraktivität proteolytisch inaktivierbar ist.

4. Verfahren zur Detektion und Analyse von Protein-Interaktionen in einer Zelle, umfassend die Schritte
e) Expression eines
e1) ersten Fusionsproteins, umfassend den ersten Interaktionspartner und einen Teil einer TEV-Protease, und
e2) eines zweiten Fusionsproteins, umfassend den zweiten Interaktionspartner und einen weiteren Teil der TEV-Protease,
wobei gegebenenfalls mindestens eines der beiden Fusionsproteine eine weitere Domäne besitzt, die zur Verankerung des Fusionsproteins außerhalb des Zellkerns führt, und
e3) Expression einer funktionalen Rekombinase,
welche gegebenenfalls eine weitere Domäne des ersten oder zweiten Fusionsproteins darstellt und von den übrigen Domänen durch eine Erkennungs- und Schnittstelle für die TEV-Protease proteolytisch abspaltbar ist, oder
Expression einer funktionalen Rekombinase
auf einem dritten Fusionsprotein, welches neben der funktionalen Rekombinase selbst, die durch eine Erkennungs- und Schnittstelle für die TEV-Protease proteolytisch abspaltbar ist, eine weitere Domäne umfaßt, die zur Verankerung des dritten Fusionsproteins außerhalb des Zellkerns führt,
in einer Zelle,
f) Rekonstituierung einer funktionalen TEV-Protease durch die Interaktion des ersten und des zweiten Interaktionspartners miteinander,
g) proteolytisches Abspalten der funktionalen Rekombinase von seiner Verankerung außerhalb des Zellkerns durch die rekonstituierte TEV-Protease aus f),
h) Transport der funktionalen Rekombinase in den Zellkern und Aktivierung eines Rekombinase-abhängigen Reporter-Gens, wodurch ein Detektionssignal erzeugt wird.

5. Verfahren nach Anspruch 4, **dadurch gekennzeichnet, daß** in Schritt e) als Komponente e3) eine funktionale Rekombinase exprimiert wird, welche mindestens eine weitere Domäne besitzt, die zur Verankerung der Rekombinase an der Zellmembran führt, und die in Schritt g) proteolytisch von ihrer Verankerung an der Zellmembran abgespalten wird.

6. Verfahren zur Detektion und Analyse von Protein-Interaktionen in einer Zelle, umfassend die Schritte
p) Expression eines
p1) ersten Fusionsproteins, umfassend den ersten Interaktionspartner und einen Teil einer TEV-Protease, und
p2) eines zweiten Fusionsproteins, umfassend den zweiten Interaktionspartner und einen weiteren Teil der TEV-Protease,
wobei gegebenenfalls mindestens eines der beiden Fusionsproteine eine weitere Domäne besitzt, die zur Verankerung des Fusionsproteins außerhalb des Zellkerns führt, und
p3) Expression eines funktionalen Transkriptionsfaktors,
welcher gegebenenfalls eine weitere Domäne des ersten oder zweiten Fusionsproteins darstellt und von den übrigen Domänen des Fusionsproteins durch eine Erkennungs- und Schnittstelle für eine TEV-Protease proteolytisch abspaltbar ist, oder
Expression eines funktionalen Transkriptionsfaktors auf einem dritten Fusionsprotein, welches neben dem funktionalen Transkriptionsfaktor selbst, der durch eine Erkennungs- und Schnittstelle für die TEV-Protease proteolytisch abspaltbar ist, eine weitere Domäne umfaßt, die zur Verankerung des dritten Fusionsproteins außerhalb des Zellkerns führt;
in einer Zelle;
q) Rekonstituierung einer funktionalen TEV-Protease durch die Interaktion des ersten und des zweiten Interaktionspartners miteinander;
r) proteolytisches Abspalten des funktionalen Transkriptionsfaktors von seiner Verankerung außerhalb des Zellkerns durch die rekonstituierte TEV-Protease aus q)
s) Bereitstellung eines funktionalen Transkriptionsfaktors im Zellkern und anschließende Induktion der Expression eines Rekombinase-abhängigen oder eines Rekombinase-unabhängigen, klassischen Reporter-Systems, wodurch ein Detektionssignal erzeugt wird.

7. Verfahren nach Anspruch 6, **dadurch gekennzeichnet, daß** der in Schritt r) proteolytisch abgespaltene funktionale Transkriptionsfaktor in Schritt s) im Zellkern zur Induktion der Expression eines Rekombinase-unabhängigen, klassischen Reporter-Systems führt und zusätzlich zur Induktion der Expression einer funktionalen TEV-Protease zur weiteren dauerhaften Aktivierung des eingesetzten Reporter-Systems führt.

8. Verfahren zur Detektion und Analyse von Protein-Interaktionen in einer Zelle, umfassend die Schritte
A) Expression eines
A1) ersten Fusionsproteins, umfassend den ersten Interaktionspartner und einen Teil einer TEV-Protease, und
A2) eines zweiten Fusionsproteins, umfassend den zweiten Interaktionspartner und einen weiteren Teil der TEV-Protease, und
A3) einer Protease, die durch Proteolyse aktivierbar ist,
in der Zelle,
B) Transkomplementation einer funktionalen TEV-Protease durch die Interaktion des ersten und des zweiten Interaktionspartners miteinander,
C) Aktivierung der Proteasen, die proteolytisch aktivierbar sind, durch die transkomplementierte funktionale TEV-Protease aus Schritt B),
D) Aktivierung oder Inaktivierung eines proteolytisch aktivierbaren oder eines proteolytisch inaktivierbaren Reporter-Systems durch die funktionalen Proteasen aus den Schritten B) und C), wodurch ein Detektionssignal erzeugt wird.

9. Verfahren zur Detektion und Analyse von Protein-Interaktionen in einer Zelle, umfassend die Schritte
J) Expression eines
J1) ersten Fusionsproteins, umfassend den ersten Interaktionspartner und einen Teil einer TEV-Protease, und
J2) eines zweiten Fusionsproteins, umfassend den zweiten Interaktionspartner und einen weiteren Teil der TEV-Protease, und
J3) konstitutive Expression eines über eine geeignete Domäne außerhalb des Zellkerns verankerten Reporter-Proteins, welches von dieser Verankerung proteolytisch abspaltbar ist, und zusätzlich eine weitere Domäne umfaßt, welche nach der proteolytischen Abspaltung die Lokalisation des Reporter-Proteins in ein bestimmtes Kompartiment der Zelle bewirkt,
in der Zelle,
K) Rekonstituierung einer funktionalen TEV-Protease durch die Interaktion des ersten und des zweiten Interaktionspartners miteinander,
L) Proteolytisches Abspalten des Reporter-Proteins mitsamt seiner Domäne, die die Lokalisation des freien Reporter-Proteins in ein bestimmtes Kompartiment der Zelle bewirkt, durch die funktionale TEV-Protease aus Schritt K),
M) Detektieren der veränderten Lokalisation des Reporter-Proteins.

10. Verfahren nach Anspruch 9, wobei das Reporter-Protein, welches proteolytisch abspaltbar ist, eine Rekombinase ist.

11. Verfahren nach Anspruch 9, wobei das Reporter-Protein, welches proteolytisch abspaltbar ist, ein Transkriptionsfaktor ist.

12. Verfahren nach einem der Ansprüche 1 bis 11 **dadurch gekennzeichnet, dass** die TEV-Protease die 27 kDa NIa Protease des Tobacco Etch Virus ist.

13. Screening-Verfahren zur Identifizierung eines spezifischen Interaktionspartners eines definierten Proteins unter Durchführung des Verfahrens nach einem der Ansprüche 1 bis 12.

14. Kit zur Detektion und zur Analyse von Protein-Interaktionen in einer Zelle umfassend Expressionsvektoren, die die Nukleinsäurekonstrukte 1a) und 2a) jeweils unter der Transkriptionskontrolle eines heterologen Promotors umfassen:
1a) ein erstes Nukleinsäurekonstrukt kodierend für ein erstes Fusionsprotein, umfassend die Nukleinsäuresequenz kodierend für ein erstes TEV-Proteasefragment und eine Klonierungsstelle, die sich zur Einklonierung des Köder-Proteins im Leseraster des ersten TEV-Proteasefragments eignet, und
2a) ein zweites Nukleinsäurekonstrukt kodierend für ein zweites Fusionsprotein, umfassend die Nukleinsäuresequenz kodierend für ein zweites TEV-Proteasefragment und eine Klonierungsstelle, die sich zur Einklonierung des Beute-Proteins im Leseraster des zweiten TEV-Proteasefragments eignet, wobei durch Interaktion des ersten und zweiten Fusionsproteins eine funktionale TEV-Protease rekonstituiert wird.

## Claims

1. A method of detecting and analyzing protein interactions a cell, which comprises the steps:
u) expressing in the cell
u1) a first fusion protein comprising the first interaction partner and a part of the protease of tobacco etch virus (TEV protease), and
u2) a second fusion protein comprising the second interaction partner and another part of the TEV protease, and
u3) a reporter protein, which can be activated or inactivated by proteolysis, wherein the reporter protein is selected from the group consisting of fluorescent proteins, luciferases, beta-galactosidase, alkaline phosphatases, beta-lactamase, proteins conferring resistance to cytotoxic substances, cytotoxic proteins, proteins altering the growth or morphology of the cell, and recombinases,
v) reconstituting the functional TEV protease via interaction of the first and second interaction partners,
w) activation of the proteolysis-activatable or inactivation of the proteolysis-inactivatable reporter protein by the reconstituted functional TEV protease of step v).

2. The method of claim 1, wherein as component u3) of step u) a proteolysis-activatable reporter protein is expressed, whose reporter activity is inactivated by the insertion of an additional amino acid sequence which is flanked on one or both sides by at least one recognition and/or cleavage site of a TEV protease.

3. The method of claim 1, wherein as component u3) of step u) a proteolysis-inactivatable reporter protein is expressed, which contains at least one recognition and cleavage site of TEV protease, and whose reporter activity is proteolytically inactivatable.

4. A method of detecting and analyzing protein interactions a cell, which comprises the steps:
e) expressing in the cell
e1) a first fusion protein comprising the first interaction partner and a part of a TEV protease, and
e2) a second fusion protein comprising the second interaction partner and another part of the TEV protease,
with, where appropriate, at least one of the two fusion proteins possessing a further domain which causes the fusion protein to be anchored outside the nucleus, and
e3) expression of a functional recombinase,
which, where appropriate, is a further domain of the first or second fusion protein and can be proteolytically removed from the other domains via a recognition and cleavage site for the TEV protease, or
expression of a functional recombinase on a third fusion protein which, in addition to the functional recombinase itself which is proteolytically removable via a recognition and cleavage site for the TEV protease, comprises a further domain causing the third fusion protein to be anchored outside the nucleus,
f) reconstitution of a functional TEV protease due to the first and second interaction partners interacting with one another,
g) proteolytic removal of the functional recombinase from its anchoring position outside the nucleus by the reconstituted TEV protease of f),
h) transport of the functional recombinase into the nucleus and activation of a recombinase-dependent reporter gene, and thereby generating a detectable signal.

5. The method of claim 4, wherein as component e3) of step e) a functional recombinase is expressed, which possesses at least a further domain, causing the anchoring of the recombinase at the plasma membrane, and which is proteolytically removed from its anchoring position at the plasma membrane within step g).

6. A method of detecting and analyzing protein interactions a cell, which comprises the steps:
p) expressing in the cell
p1) a first fusion protein comprising the first interaction partner and part of a TEV protease, and
p2) a second fusion protein comprising the second interaction partner and another part of the TEV protease,
with, where appropriate, at least one of the two fusion proteins possessing a further domain which causes the fusion protein to be anchored outside the nucleus, and
p3) expression of a functional transcription factor,
which, where appropriate, is a further domain of the first or second fusion protein and which is proteolytically removable from the other domains of the fusion protein via a recognition and cleavage site for a TEV protease, or expression of a functional transcription factor on a third fusion protein which, in addition to the functional transcription factor itself which is proteolytically removable via a recognition and cleavage site for the TEV protease, comprises a further domain which causes the third fusion protein to be anchored outside the nucleus;
q) reconstitution of a functional TEV protease due to the first and second interaction partners interacting with one another;
r) proteolytically removing the functional transcription factor from its anchoring position outside the nucleus by the reconstituted TEV protease of q),
s) providing a functional transcription factor in the nucleus and subsequently inducing expression of a recombinase-dependent or a recombinase-independent classical reporter system, and thereby generating a detectable signal.

7. The method of claim 6, wherein the proteolytically removed functional transcription factor of step r) causes in step s) the induction of the expression of a recombinase-independent classical reporter system in the nucleus and additionally causes the induction of the expression of a functional TEV protease for further permanent activation of the used reporter system.

8. A method of detecting and analyzing protein interactions a cell, which comprises the steps:
A) expressing in the cell
A1) a first fusion protein comprising the first interaction partner and part of a TEV protease, and
A2) a second fusion protein comprising the second interaction partner and another part of the TEV protease, and
A3) a protease which can be activated by proteolysis,
B) transcomplementation of a functional TEV protease by the first and second interaction partners interacting with one anther,
C) activation of the proteolytically activatable proteases by the transcomplemented functional TEV protease of step B),
D) activation or inactivation of a proteolytically activatable or a proteolytically inactivatable reporter system by the functional proteases of steps B) and C), and thereby generating a detectable signal.

9. A method of detecting and analyzing protein interactions a cell, which comprises the steps:
J) expressing in the cell
J1) a first fusion protein comprising the first interaction partner and part of a TEV protease, and
J2) a second fusion protein comprising the second interaction partner and another part of the TEV protease, and
J3) constitutive expression of a reporter protein which is anchored via a suitable domain outside the nucleus and which can be proteolytically removed from the anchoring position, and additionally comprises a further domain which, after proteolytic removal, effects the localization of the reporter protein into a particular compartment of the cell,
K) reconstitution of a functional TEV protease due to said first and second interaction partners interacting with one another,
L) proteolytically removing the reporter protein together with its domain which causes localization of the free reporter protein into a particular compartment of the cell, by the TEV functional protease of step K),
M) detecting the altered location of the reporter protein.

10. The method of claim 9, wherein the proteolytically removable reporter protein is a recombinase.

11. The method of claim 9, wherein the proteolytically removable reporter protein is a transcription factor.

12. The method of any of claims 1 to11, wherein the TEV protease is the 27 kDa NIa protease of tobacco etch virus.

13. A screening method of identifying a specific interaction partner of a defined protein by carrying out the method as claimed in any of claims 1 to 12.

14. A kit for detecting and analyzing protein interactions in a cell, which comprises the expression vectors comprising the nucleic acid constructs 1a) and 2a), in each case under the transcriptional control of a heterologous promoter:
1a) a first nucleic acid construct coding for a first fusion protein comprising the nucleic acid sequence coding for a first TEV protease fragment and a cloning site suitable for cloning the bait protein in the reading frame of the first TEV protease fragment, and
2a) a second nucleic acid construct coding for a second fusion protein comprising the nucleic acid sequence coding for a second TEV protease fragment and a cloning site suitable for cloning the prey protein in the reading frame of the second TEV protease fragment,
whereas reconstitution of a functional TEV protease is caused due to said first and second interaction partners interacting with one another.

## Revendications

1. Procédé de détection et d'analyse des interactions protéiques dans une cellule comprenant les étapes :
u) d'expression de :
u1) une première protéine de fusion comprenant le premier partenaire d'interaction et une partie d'une Tobacco-Etch-Virus (TEV) protéase, et
u2) une seconde protéine de fusion comprenant le second partenaire d'interaction et une partie différente de la TEV-protéase, et
u3) un rapporteur qui peut être activé ou inactivé par protéolyse choisi parmi le groupe comprenant des protéines rapporteurs fluorescentes, la luciférase, la bêta-galactosidase, la phosphatase alcaline, la bêta-lactamase, une protéine développant une résistance contre les substances cytotoxiques, les protéines cytotoxiques, les protéines qui modifient la croissance et la morphologie de la cellules et les recombinases,
dans la cellule,
v) de reconstitution d'une TEV-protéase fonctionnelle par interaction l'un avec l'autre des premier et second partenaires d'interaction,
w) l'activation du rapporteur activable par protéolyse ou l'inactivation du rapporteur inactivable par protéolyse par la TEV-protéase fonctionnelle reconstituée de l'étape v).

2. Procédé selon la revendication 1, **caractérisé en ce que** le composant u3) exprimé dans l'étape u) est une protéine rapporteur activable par protéolyse dont l'activité de rapporteur a été inactivée par insertion d'une séquence supplémentaire d'acide aminé qui est flanquée d'un côté ou sur les côtés d'au moins d'un site de reconnaissance et/ou un site de clivage pour une TEV protéase.

3. Procédé selon la revendication 1, **caractérisé en ce que** le composant u3) exprimé dans l'étape u) est une protéine rapporteur inactivable par protéolyse qui comprend au moins d'un site de reconnaissance et de clivage pour une TEV-protéase et dont l'activité de rapporteur est inactivable par protéolyse.

4. Procédé de détection et d'analyse des interactions protéiques dans une cellule, qui comprend les étapes :
e) d'expression de
e1) une première protéine de fusion comprenant le premier partenaire d'interaction et une partie d'une TEV-protéase, et
e2) une seconde protéine de fusion comprenant le second partenaire d'interaction et une partie différente de la TEV-protéase, et
dans laquelle, le cas échéant, au moins une des deux protéines de fusion possède un autre domaine qui conduit à l'ancrage de ladite protéine de fusion en dehors du noyau cellulaire, et
e3) d'expression d'une recombinase fonctionnelle,
qui, le cas échéant, constitue un autre domaine de la première ou seconde protéine de fusion et qui peut être éliminée par protéolyse des autres domaines par l'intermédiaire d'un site de reconnaissance et de clivage pour la TEV-protéase,
ou d'expression d'une recombinase fonctionnelle
sur une troisième protéine de fusion qui, en plus de la recombinase fonctionnelle elle-même qui est éliminable par protéolyse par l'intermédiaire d'un site de reconnaissance et de clivage pour la TEV-protéase, comprend un autre domaine qui conduit à l'ancrage de la troisième protéine de fusion en dehors du noyau,
dans une cellule,
f) de reconstitution d'une TEV-protéase fonctionnelle par interaction des premier et second partenaires d'interaction l'un avec l'autre,
g) d'élimination protéolytique de la recombinase fonctionnelle de sa position d'ancrage en dehors du noyau par la TEV-protéase reconstituée de f),
h) de transport de la recombinase fonctionnelle dans le noyau et d'activation d'un gène rapporteur recombinase-dépendant, générant ainsi un signal de détection.

5. Procédé selon la revendication 4, **caractérisé en ce que** le composant e3) exprimé dans l'étape e) est une recombinase fonctionnelle qui possède au moins un autre domaine, qui conduit à l'ancrage de ladite recombinase sur la membrane cellulaire et qui est éliminé par proteolyse dans l'étape g) de sa position d'ancrage sur la membrane cellulaire.

6. Procédé de détection et d'analyse des interactions protéiques dans une cellule, qui comprend les étapes :
p) d'expression de :
p1) une première protéine de fusion comprenant le premier partenaire d'interaction et une partie d'une Tobacco-Etch-Virus (TEV) protéase, et
p2) une seconde protéine de fusion comprenant le second partenaire d'interaction et une partie différente de la TEV-protéase, et
dans laquelle, le cas échéant, au moins un des deux protéines de fusion possède un autre domaine qui conduit à l'ancrage de ladite protéine de fusion en dehors du noyau cellulaire, et
p3) expression d'un facteur de transcription fonctionnel,
qui, le cas échéant, constitue un autre domaine de la première ou seconde protéine de fusion et qui peut être éliminée par protéolyse des autres domaines par l'intermédiaire d'un site de reconnaissance et de clivage pour la TEV protéase, ou
expression d'un facteur de transcription fonctionnel sur une troisième protéine de fusion qui, en plus du facteur de transcription fonctionnel lui-même qui est éliminable par protéolyse par l'intermédiaire d'un site de reconnaissance et de clivage pour la TEV-protéase, comprend un autre domaine qui conduit à l'ancrage de la troisième protéine de fusion en dehors du noyau,
dans une cellule,
q) de reconstitution d'une TEV-protéase fonctionnelle par interaction des premier et second partenaires d'interaction l'un avec l'autre,
r) d'élimination protéolytique du facteur de transcription fonctionnel de sa position d'ancrage en dehors du noyau par la TEV protéase reconstituée de q),
s) de fournir un facteur de transcription fonctionnel au noyau et d'induire ultérieurement l'expression d'un système classique recombinase-dépendant ou recombinase-indépendant de rapporteur, générant ainsi un signal de détection.

7. Procédé selon la revendication 6, **caractérisé en ce que** le facteur de transcription fonctionnel éliminé par protéolyse dans l'étape r) conduit dans l'étape s) à l'induction dans le noyau cellulaire de l'expression d'un système rapporteur classique recombinase-indépendant et conduit en outre à l'induction de l'expression d'une TEV-protéase fonctionnelle en vue de l'activation durable ultérieure du système rapporteur employé.

8. Procédé de détection et d'analyse des interactions protéiques dans une cellule, qui comprend les étapes :
A) d'expression de :
A1) une première protéine de fusion comprenant le premier partenaire d'interaction et une partie d'une Tobacco-Etch-Virus (TEV) protéase, et
A2) une seconde protéine de fusion comprenant le second partenaire d'interaction et une partie différente de la TEV-protéase, et
A3) une protéase qui peut être activée par protéolyse
dans la cellule,
B) de transcomplémentation d'une TEV-protéase fonctionnelle par interaction des premier et second partenaires d'interaction l'un avec l'autre,
C) de l'activation des protéases qui sont activables par protéolyse, par la TEV-protéase fonctionnelle transcomplementée de l'étape B),
D) de l'activation ou l'inactivation d'un système rapporteur activable par protéolyse ou inactivable par protéolyse par les protéases fonctionnelles des étapes B) et C), générant ainsi un signal de détection.

9. Procédé de détection et d'analyse des interactions protéiques dans une cellule, qui comprend les étapes :
J) d'expression de :
J1) une première protéine de fusion comprenant le premier partenaire d'interaction et une partie d'une Tobacco-Etch-Virus (TEV) protéase, et
J2) une seconde protéine de fusion comprenant le second partenaire d'interaction et une partie différente de la TEV-protéase, et
J3) l'expression constitutive d'une protéine de rapporteur qui est ancrée par l'intermédiaire d'un domaine approprié en dehors du noyau et qui peut être éliminée par protéolyse de ladite position d'ancrage, et comprend en outre un autre domaine qui, après élimination par protéolyse, effectue la localisation de ladite protéine de rapporteur dans un compartiment particulier de la cellule,
dans la cellule,
K) reconstitution d'une protéase fonctionnelle par interaction des premier et second partenaires d'interaction l'un avec l'autre,
L) d'élimination par protéolyse de la protéine rapporteur ainsi que son domaine qui provoque la localisation de la protéine rapporteur libre dans un compartiment particulier de la cellule, par la protéase fonctionnelle de l'étape K),
M) de détection de la localisation modifiée de la protéine rapporteur.

10. Procédé selon la revendication 9, dans lequel la protéine rapporteur, qui est éliminable par protéolyse, est une recombinase.

11. Procédé selon la revendication 9, dans lequel la protéine rapporteur, qui est éliminable par protéolyse, est un facteur de transcription.

12. Procédé selon l'une des revendications 1 à 11, **caractérisé en ce que** la TEV-protéase est une protéase NIa de 27 kDa de Tobacco Etch Virus.

13. Procédé de criblage en vue de l'identification d'une interaction spécifique d'une protéine particulière, par la mise en oeuvre d'un procédé selon l'une quelconque des revendications 1 à 12.

14. Kit de détection et d'analyse des interactions protéiques dans une cellule, qui comprend les vecteurs d'expression comprenant des constructions d'acide nucléique 1a) et 2a), dans chaque cas sous la commande transcriptionnelle d'un promoteur hétérologue comprenant:
1a) une première construction d'acide nucléique codant pour une première protéine de fusion comprenant la séquence d'acide nucléique codant pour un premier fragment de TEV-protéase et un site de clonage appropriés pour copier la protéine d'appât dans le cadre de lecture du premier fragment de protéase, et
2a) une deuxième construction d'acide nucléique codant pour une deuxième protéine de fusion comprenant la séquence d'acide nucléique codant pour un second fragment de TEV-protéase et un site de clonage appropriés pour copier la protéine de proie dans le cadre de lecture du second fragment de protéase,
dans lequel, par l'interaction des première et seconde protéines de fusion, une TEV-protéase fonctionnelle est reconstituée.
